# EUROPEAN PATENT APPLICATION

(11) **EP 1 593 681 A1**
(43) Date of publication of application: **09.11.2005**
(21) Application number: 04708469.4
(22) Date of filing: 05.02.2004
(51) Int. Cl.: C07D 471/04, C07D 471/06, A61K 31/55, A61K 31/4353, A61P 9/00, A61P 9/10, A61P 13/12, A61P 29/00, A61P 31/18, A61P 37/02, A61P 37/04, A61P 37/06, A61P 37/08, A61P 43/00, C07D 487/04

(54) **TRICYCLIC COMPOUND, PROCESS FOR PRODUCING THE SAME, AND USE**

(30) Priority: 07.02.2003 JP 2003031112
(71) Applicant: Takeda Pharmaceutical Company Limited, Osaka 541-0045 (JP)
(72) Inventor: SHIRAISHI, M. c/o Takeda Pharmaceutical Com. Ltd., Osaka-shi, Osaka 532-8686 (JP); SETO, M. c/o Takeda Pharmaceutical Comp. Ltd., Osaka-shi, Osaka 532-8686 (JP); AIKAWA, K. c/o Takeda Pharmaceutical Comp. Ltd., Osaka-shi, Osaka 532-8686 (JP); KANZAKI, N. c/o Takeda Pharmaceutical Comp. Ltd., Osaka-shi, Osaka 532-8686 (JP); BABA, Masanori, Kagoshima-shi, Kagoshi 8910103 (JP)
(74) Representative: Lewin, John Harvey
(86) International application number: PCT/JP2004/001197
(87) International publication number: WO 2004/069834

(57) **Abstract**

A compound of the formula: 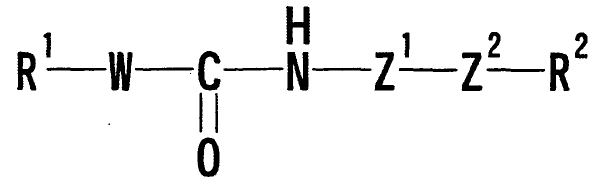 wherein R¹ is a 5- or 6-membered ring;
Z¹ is a 5- or 6-membered aromatic ring;
Z² is a group of -Z^{2a}-W²-Z^{2b}-, wherein Z^{2a} and Z^{2b} are each O, S(O)_{q} (wherein q is 0, 1 or 2), an imino group, or a bond; and W² is an alkylene chain;
W is a group represented by 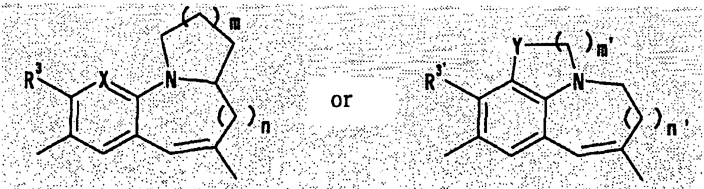 wherein R³ and R^{3'} are each a hydrogen atom, a lower alkyl group, or a lower alkoxy group; X is CH or N; n and n' are each an integer of 0 or 1 to 4; m and m' are each 1 or 2; Y is O, S(O)ₚ (wherein p is 0, 1 or 2), CH₂ or NR⁴ (wherein R⁴ is a hydrogen atom, a lower alkyl group, or a lower acyl group); and
R² is (1) an amino group, in which the nitrogen atom may be converted to a quaternary ammonium or an oxide, or (2) a nitrogen-containing heterocyclic group which may contain a sulfur atom or an oxygen atom as the ring-constituting atom, in which the nitrogen atommay be converted to a quaternary ammonium or an oxide; or a salt thereof.

The compound exhibits excellent CCR antagonist activity against CCR5, and is useful as a prophylactic and/or therapeutic agent for HIV infection in human peripheral blood mononuclear cells, especially for AIDS.

## Description

### Technical Field

The present invention relates to a new cyclic compound having CCR antagonist activity, especially CCR5 antagonist activity, and to use thereof.

### Background Art

Recently, HIV (human immunodeficiency virus) protease inhibitors have been developed for the treatment of AIDS (acquired immune deficiency syndrome). With combined use of the protease with either of two HIV reverse transcriptase inhibitors which have been commonly used, treatment of AIDS has made remarkable progress. However, the treatment is still not efficient enough for the eradication of AIDS, and development of a new anti-AIDS medicine based on a different mechanism of action is desired.

As a receptor upon invasion of HIV into a target cell, CD4 has already been known. Recently, new receptors, namely, CCR5 as a second receptor of macrophage directed HIV, and CXCR4 as a second receptor of T cell directed HIV, which is a G-protein coupled chemokine receptor having a seven-transmembrane protein structure, have been found, and these chemokine receptors are considered to play an essential role for infection and transmission of HIV. As a matter of fact, it has been reported that a man having resistance to HIV infection even after repeated exposures to the virus had a mutation in which CCR5 gene was deleted homozygously. Thus, the CCR5 antagonists have been expected to become a new anti-HIV medicine, and examples of synthesis of new anilide derivatives having CCR5 antagonist activity have been reported in the below-mentioned patent applications such as Patent Document 1, Patent Document 2 and Patent Document 3, while there has been no report of a CCR5 antagonist which has been commercialized as a therapeutic medicine for AIDS. Further, a compound having CCR5 antagonist activity is described to be useful as a prophylactic and/or therapeutic medicine for AIDS in the below-mentioned Patent Document 4, but said compound has a different structure from the compound of the present invention.

Patent Document 1: WO99/32100

Patent Document 2: Japanese Patent Application No. 10-234388

Patent Document 3: Japanese Patent Application No. 10-363404

Patent Document 4: JP-A No. 2001-026586

### Disclosure of the Invention

The present invention is to provide a novel tricyclic compound that is useful for preventing and/or treating HIV infectious disease, especially AIDS, based on its CCR antagonist activity, especially CCR5 antagonist activity.

The present inventors have intensively studied on a compound having CCR5 antagonist activity, and found a compound of the formula (I) below or a salt thereof, (hereinafter sometimes referred to as Compound (I)), has an excellent clinically-favorable pharmacological property including CCR antagonist activity, especially CCR5 antagonist activity, and completed the present invention.

Thus, the present invention provides:
[1] A compound of the formula: 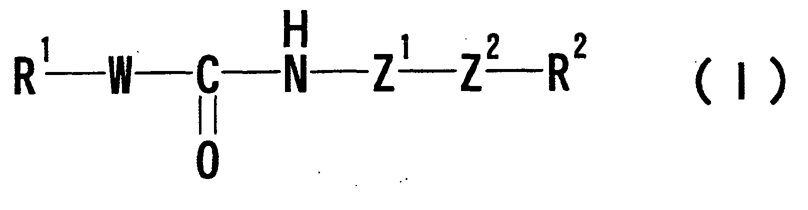 wherein R¹ is a 5- or 6-membered ring which may be substituted;
   Z¹ is a 5- or 6-membered aromatic ring which may be further substituted;
   Z² is a group of -Z^{2a}-W¹-Z^{2b}-, wherein Z^{2a} and Z^{2b} are each O, S(O)_{q} (wherein q is 0, 1 or 2), an imino group which may be substituted, or a bond; and W¹ is an alkylene chain which may be substituted, an alkenylene chain which may be substituted, or a bond;
   W is a group represented by 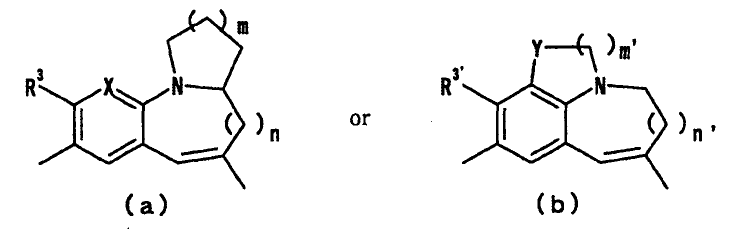 wherein R³ and R^{3'} are each a hydrogen atom, a lower alkyl group which may be substituted, or a lower alkoxy group which may be substituted; X is CH or N; n and n' are each an integer of 0 or 1 to 4; m and m' are each 1 or 2; Y is O, S(O)ₚ (wherein p is 0, 1 or 2), CH₂ or NR⁴ (wherein R⁴ is a hydrogen atom, a lower alkyl group which may be substituted, or a lower acyl group which may be substituted); and
   R² is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula: 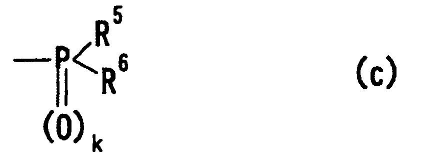 wherein k is 0 or 1, and when k is 0, the phosphorus atom may form a phosphonium salt; R⁵ and R⁶ are each a hydrocarbon group which may be substituted, a hydroxy group which may be substituted, or an amino group which may be substituted; or R⁵ and R⁶ may be bonded to each other to form a cyclic group with the adjacent phosphorus atom, (4) an amidino group which may be substituted, or (5) a guanidino group which may be substituted; or a salt thereof;
[2] A prodrug of the compound according to the above [1];
[3] The compound according to the above [1], wherein R¹ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted;
[4] The compound according to the above [1], wherein R¹ is a benzene which may be substituted;
[5] The compound according to the above [1], wherein n is 2;
[6] The compound according to the above [1], wherein Z¹ is a benzene which may be substituted with a substituent selected from (1) a halogen atom, (2) a C₁₋₄ alkyl group which may be substituted with a halogen atom, and (3) a C₁₋₄ alkoxy group which may be substituted with a halogen atom;
[7] The compound according to the above [1], wherein Z¹ is a benzene which may be substituted with a methyl group or a trifluoromethyl group;
[8] The compound according to the above [1], wherein Z² is a group represented by -Z^{2a}-W²-Z^{2b}-, wherein Z^{2a} and Z^{2b} are each O, S(O)_{q} (wherein q is 0, 1 or 2), an imino group which may be substituted, or a bond, and W² is an alkylene chain which may be substituted;
[9] The compound according to the above [1], wherein Z² is -CH₂-, -CH(OH)- or -S(O)_{q}-CH₂- (wherein q is 0, 1 or 2);
[10] The compound according to the above [1], wherein Z² is a group represented by -S(O)_{q}-CH₂- (wherein q is 0, 1 or 2) ;
[11] The compound according to the above [1], wherein R² is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted;
[12] The compound according to the above [1], wherein R² is an amino group which may be substituted, or a nitrogen-containing heterocyclic group which may be substituted and may contain a sulfur atom or an oxygen atom as the ring-constituting atom;
[13] The compound according to the above [1], wherein R² is -NRR', wherein R and R' are each an aliphatic hydrocarbon group which may be substituted or an alicyclic heterocyclic group which may be substituted;
[14] The compound according to the above [1], wherein R² is a nitrogen-containing heterocyclic aromatic group which may be substituted;
[15] The compound according to the above [1], wherein R² is an imidazolyl group which may be substituted or a triazolyl group which may be substituted;
[16] The compound according to claim 1, wherein W is a group represented by 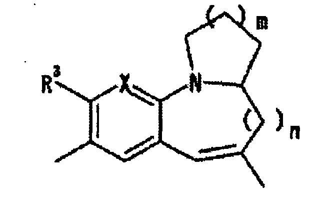 wherein each symbol has the same meaning as defined in [1];
[17] The compound according to the above [1], wherein R¹ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted with a halogen, a nitro, a cyano, a C₁₋₆ alkyl, a C₁₋₆ alkoxy, a C₁₋₆ alkoxy-C₁₋₆ alkyl or a C₁₋₆ alkoxy-C₁₋₆ alkoxy,
   Z¹ is a benzene which may be substituted with a substituent selected from (1) a halogen atom, (2) a C₁₋₄ alkyl group which may be substituted with a halogen atom and (3) a C₁₋₄ alkoxy group which may be substituted with a halogen atom,
   Z² is -Z^{2a}-W¹-Z^{2b}-, wherein Z^{2a} and Z^{2b} are each O, S(O)_{q} (wherein q is 0, 1 or 2), an imino group which may be substituted with a C₁₋₄ alkyl group, or a bond, and W¹ is a bond, or a C₁₋₄ alkylene chain or a C₂₋₄ alkenylene chain, each of which may be substituted with a C₁₋₆ alkyl, a hydroxy group, a hydroxyimino or a C₁₋₆ alkoxyimino, and
   R² is an amino group which may be substituted with a C₁₋₄ alkyl group, or a nitrogen-containing heterocyclic group which may contain a sulfur atom or an oxygen atom as the ring-constituting atom and may be substituted with a C₁₋₄ alkyl group;
[18] A compound represented by the formula: 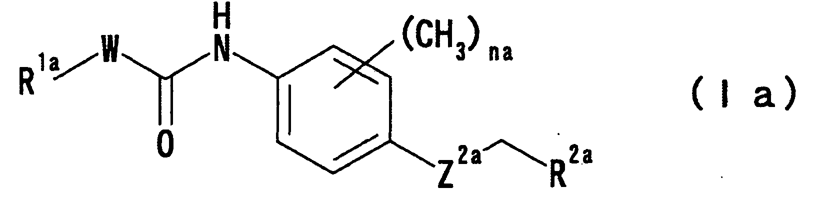 wherein R^{1a} is a (C₁₋₆ alkoxy-C₁₋₆ alkoxy)phenyl,
   R^{2a} is (1) an N-C₁₋₆ alkyl-N-tetrahydropyranylamino, (2) an imidazolyl which may be substituted with a C₁₋₆ alkyl which may be substituted, or (3) a triazolyl which may be substituted with C₁₋₆ alkyl which may be substituted,
   R³ is a hydrogen atom, a lower alkyl group which may be substituted, or a lower alkoxy group which may be substituted,
   na is 0 or 1,
   Z^{2a} is a bond, S, SO or SO₂, and
   W is the group as defined in the formula (a) or (b), or a salt thereof;
[19] The compound according to the above [17], wherein Z^{2a} is SO;
[20] The compound according to the above [18], wherein Z^{2a} is SO having a configuration of (S);
[21] The compound according to the above [17], wherein n and n' are each 2;
[22] (Ss)-9-[4-(2butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]benzazocine-6-carboxamide and a diastereomer thereof;
[23] (Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide and a diastereomer thereof;
[24] A process for producing a compound represented by the formula: 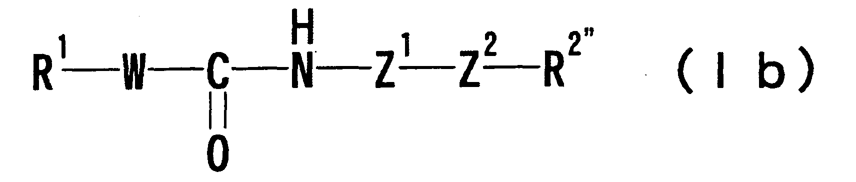 wherein R^{2"} is (1) an amino group which may be substituted, in which the nitrogen group may be converted to a quaternary ammonium, (2) a nitrogen-containing heterocyclic group which may be substituted and may contain a sulfur atom or an oxygen atom as the ring-constituting atom, in which the nitrogen atom may be converted to a quaternary ammonium, or (3) a group represented by the formula (c), and the other symbols have the same meanings as defined above, or a salt thereof, which comprises subjecting a compound represented by the formula: 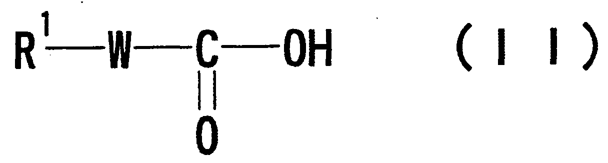 wherein each symbol has the same meaning as defined above, a salt thereof or a reactive derivative thereof, and a compound represented by the formula: 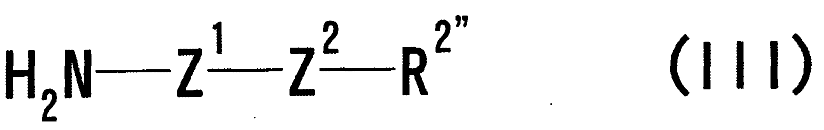 wherein each symbol has the same meaning as defined above, a salt thereof to a condensation reaction, and then optionally to deprotection, oxidation-reduction and/or quaternization reaction;
[25] A pharmaceutical composition comprising the compound represented by the formula (I), a salt thereof or a prodrug thereof;
[26] The pharmaceutical composition according to the above [25], which is a CCR antagonist;
[27] The pharmaceutical composition according to the above [26], wherein CCR is CCR5 and/or CCR2;
[28] The pharmaceutical composition according to the above [26], wherein CCR is CCR5;
[29] The composition according to the above [25], which is a prophylactic and/or therapeutic agent for HIV infection, chronic rheumatoid arthritis, autoimmune diseases, allergic diseases, ischemic brain cell disorder, cardiac infarction, nephritis/nephropathy or arteriosclerosis;
[30] The pharmaceutical composition according to the above [25], which is a prophylactic and/or therapeutic agent for HIV infection;
[31] The pharmaceutical composition according to the above [25], which is a prophylactic and/or therapeutic agent for AIDS;
[32] The pharmaceutical composition according to the above [25], which is a suppressive agent for disease progression of AIDS;
[33] A method for preventing or treating HIV infection, chronic rheumatoid arthritis, autoimmune diseases, allergic diseases, ischemic brain cell disorder, cardiac infarction, nephritis/nephropathy, arteriosclerosis or graft-versus-host diseases, which comprises administering an effective amount of the compound according to the above [1], a salt thereof or a prodrug thereof to a subject in need thereof; and
[34] Use of the compound according to the above [1], a salt thereof or a prodrug thereof, for the manufacture of a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.

### Best Mode for Carrying Out the Invention

In the above-described formula (I), the "5- or 6-membered ring" in the "5- or 6-membered ring group which may be substituted" represented by R¹ may be exemplified by a group which is formed by eliminating one hydrogen atom from 6-membered aromatic hydrocarbon such as benzene, etc.; 5- or 6-membered aliphatic hydrocarbon such as cyclopentane, cyclohexane, cyclopentene, cyclohexene, cyclopentanediene, cyclohexanediene, etc.; a 5- or 6-membered heterocyclic aromatic group containing 1 to 4 heteroatoms of one or two kinds selected from a nitrogen atom, a sulfur atom and an oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; a 5- or 6-membered non-heterocyclic aromatic group containing 1 to 4 heteroatoms of one or two kinds selected from a nitrogen atom, a sulfur atom and an oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.; and the like. Among them, the "5- or 6-membered ring" is preferably benzene, furan, thiophene, pyridine, cyclopentane, cyclohexane, pyrrolidine, piperidine, piperazine, morpholine, thiomorpholine, tetrahydropyran (preferably a 6-membered ring), and the like, it being particularly preferably benzene.

The "substituent" of the "5- or 6-membered ring" of the "5- or 6-membered ring group which may be substituted" represented by R¹ may be exemplified by a halogen atom, nitro, cyano, alkyl which may be substituted, cycloalkyl which may be substituted, hydroxy group which may be substituted, a thiol group which may be substituted (wherein the sulfur atom may be oxidized, or may form a sulfinyl group which may be substituted or a sulfonyl group which may be substituted), an amino group which may be substituted, acyl which may be substituted, a carboxyl group which may be esterified, an aromatic group which may be substituted, and the like.

Examples of the "halogen" as the substituent of R¹ include fluorine, chlorine, bromine, iodine and the like, it being preferably fluorine and chlorine.

The "alkyl" of the "alkyl which may be substituted" as the substituent of R¹ may be exemplified by linear or branched alkyl having 1 to 10 carbon atoms, for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl. Examples of the substituent of said "alkyl which may be substituted" include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

Examples of the "cycloalkyl" of the "cycloalkyl which may be substituted" as the substituent of R¹ include C₃₋₇ cycloalkyl such as cyclopropyl, cyclobutyl, cyclopentyl, cyclohexyl, cycloheptyl, etc., and the like. Examples of the substituent in the "cycloalkyl which may be substituted" include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The substituent of the "the hydroxy group which may be substituted" as the substituent of R¹ may be exemplified by
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl; and the like) ;
(2) cycloalkyl which may be substituted and may contain heteroatom(s) (for example, C₃₋₇ cycloalkyl such as cyclopropyl, cyclobutyl, cyclopentyl, cyclohexyl, cycloheptyl, etc.; a 5- or 6-membered saturated heterocyclic group containing 1 or 2 heteroatoms, such as tetrahydrofuranyl, tetrahydrothienyl, pyrrolidinyl, pyrazolidinyl, piperidinyl, piperazinyl, morpholinyl, thiomorpholinyl, tetrahydropyranyl, tetrahydrothiopyranyl, etc., and the like, and preferably tetrahydropyranyl, etc.; and the like);
(3) alkenyl which may be substituted (for example, alkenyl having 2 to 10 carbon atoms, such as allyl, crotyl, 2-pentenyl, 3-hexenyl, etc., and preferably lower (C₂₋₆) alkenyl; and the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.; and the like);
(5) aralkyl which may be substituted (for example, phenyl-C₁₋₄ alkyl such as benzyl, phenethyl, etc.; and the like) ;
(6) formyl, or acyl which may be substituted (for example, alkanoyl having 2 to 4 carbon atoms, such as acetyl, propionyl, butyryl, isobutyryl, etc.), alkylsulfonyl having 1 to 4 carbon atoms (for example, methanesulfonyl, ethanesulfonyl, etc.), and the like);
(7) aryl which may be substituted (for example, phenyl, naphthyl, etc.); and the like.

The substituent of the above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, and (7) aryl which may be substituted, may be exemplified by halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkyl which may be halogenated (for example, trifluoromethyl, methyl, ethyl, etc.), C₁₋₆ alkoxy which may be halogenated (for example, methoxy, ethoxy, propoxy, butoxy, trifluoromethoxy, trifluoroethoxy, etc.; and preferably C₁₋₄ alkoxy which may be halogenated), formyl, C₂₋₄ alkanoyl (for example, acetyl, propionyl, etc.), C₁₋₄ alkylsulfonyl (for example, methanesulfonyl, ethanesulfonyl, etc.), 5- or 6-membered heterocyclic aromatic group which may be substituted [for example, 5- or 6-membered heterocyclic aromatic group containing 1 to 4 heteroatoms of one or two kinds selected from a nitrogen atom, a sulfur atom and an oxygen atom such as furan, thiophene, pyrrole, imidazole, pyrazole, thiazole, oxazole, isothiazole, isoxazole, tetrazole, pyridine, pyrazine, pyrimidine, pyridazine, triazole, etc.; examples of the substituent of said heterocyclic group include halogen (for example, fluorine, chlorine, bromine, iodine, etc.), nitro, cyano, hydroxy group, thiol, an amino group, a carboxyl group, C₁₋₄ alkyl which may be halogenated (for example, trifluoromethyl, methyl, ethyl, etc.), C₁₋₄ alkoxy which may be halogenated (for example, methoxy, ethoxy, propoxy, butoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3], and the like, and the number of the substituents is preferably 1 to 3.

The substituent of the "thiol group which may be substituted" as the substituent of R¹ may be exemplified by the same ones as the "substituent of the hydroxy group which may be substituted as the substituent of R¹" as described above. Among them, the substituent is preferably:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl; and the like) ;
(2) cycloalkyl which may be substituted (for example, C₃₋₇ cycloalkyl such as cyclopropyl, cyclobutyl, cyclopentyl, cyclohexyl, cycloheptyl, etc., and the like);
(3) aralkyl which may be substituted (for example, phenyl-C₁₋₄ alkyl (for example, benzyl, phenethyl, etc.) and the like) ;
(4) aryl which may be substituted (for example, phenyl, naphthyl, etc.); and the like.

The substituent of the above-described (1) alkyl which may be substituted, (2) cycloalkyl which may be substituted, (3) aralkyl which may be substituted and (4) aryl which may be substituted may be exemplified by halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The substituent of the "amino group which may be substituted" as the substituent of R¹ may be exemplified by the same ones as the "substituent of the hydroxy group which may be substituted as the substituent of R¹" as described above, and the number of substituents on the amino group may be 1 or 2. Among them, the substituent is preferably:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₁₀) alkyl; and the like) ;
(2) cycloalkyl which may be substituted (for example, C₃₋₇ cycloalkyl such as cyclopropyl, cyclobutyl, cyclopentyl, cyclohexyl, cycloheptyl, etc.);
(3) alkenyl which may be substituted (for example, alkenyl having 2 to 10 carbon atoms, such as allyl, crotyl, 2-pentenyl, 3-hexenyl, etc., and preferably lower (C₂₋₆) alkenyl; and the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.; and the like);
(5) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.) and the like);
(6) aryl which may be substituted (for example, phenyl, naphthyl, etc.); and the like.

Examples of the substituent of the above-described (1) alkyl which may be substituted, (2) cycloalkyl which may be substituted, (3) alkenyl which may be substituted, (4) cycloalkenyl which may be substituted, (5) acyl which may be substituted, and (6) aryl which may be substituted, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), a carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

Further, the substituents of the "amino group which may be substituted" as the substituent of R¹ may be bonded to each other to form a cycloamino group (for example, a cycloamino group which is formed by eliminating a hydrogen atom from the ring-constituting nitrogen atom of a 5- or 6-membered ring and which has a bond on the nitrogen atom, such as tetrahydropyrrole, piperazine, piperidine, morpholine, thiomorpholine, pyrrole, imidazole, etc. so that a linking bond is made available on the nitrogen atom, and the like). This cycloamino group may be substituted, and examples of the substituent include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl), and the like, while the number of the substituents is preferably 1 to 3.

The "acyl group which may be substituted" as the substituent of R¹ may be exemplified by a group in which
(1) hydrogen;
(2) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl; and the like) ;
(3) cycloalkyl which may be substituted (for example, C₃₋₇ cycloalkyl such as cyclopropyl, cyclobutyl, cyclopentyl, cyclohexyl, cycloheptyl, etc.);
(4) alkenyl which may be substituted (for example, alkenyl having 2 to 10 carbon atoms, such as allyl, crotyl, 2-pentenyl, 3-hexenyl, etc., and preferably lower (C₂₋₆) alkenyl; and the like);
(5) cycloalkenyl which may be substituted (for example, cycloalkenyl of 3 to 7 carbon atoms, such as 2-cyclopentenyl, 2-cyclohexenyl, 2-cyclopentenylmethyl, 2-cyclohexenylmethyl, etc.);
(6) 5- or 6-membered monocyclic aromatic group which may be substituted (for example, phenyl, pyridyl, etc.);
   and the like
   is bonded to a carbonyl group or a sulfonyl group, (for example, formyl, acetyl, propionyl, butyryl, isobutyryl, valeryl, isovaleryl, pivaloyl, hexanoyl, heptanoyl, octanoyl, cyclobutanecarbonyl, cyclopentanecarbonyl, cyclohexanecarbonyl, cycloheptanecarbonyl, crotonyl, 2-cyclohexenecarbonyl, benzoyl, nicotinoyl, methanesulfonyl, ethanesulfonyl, etc.). Examples of the substituent of the above-described (2) alkyl which may be substituted, (3) cycloalkyl which may be substituted, (4) alkenyl which may be substituted, (5) cycloalkenyl which may be substituted, and (6) 5- or 6-membered monocyclic aromatic group which may be substituted, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkyl-carbamoyl, etc.), C₁₋₄ alkoxy which may be halogenated (for example, methoxy, ethoxy, propoxy, butoxy, trifluoromethoxy, trifluoroethoxy, etc.); C₁₋₄ alkoxy-C₁₋₄ alkoxy which may be halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The "carboxyl group which may be esterified" as the substituent of R¹ may be exemplified by a group in which
(1) hydrogen;
(2) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., and preferably lower (C₁₋₆) alkyl; and the like);
(3) cycloalkyl which may be substituted (for example, C₃₋₇ cycloalkyl such as cyclopropyl, cyclobutyl, cyclopentyl, cyclohexyl, cycloheptyl, etc.);
(4) alkenyl which may be substituted (for example, alkenyl having 2 to 10 carbon atoms, such as allyl, crotyl, 2-pentenyl, 3-hexenyl, etc., and preferably lower (C₂₋₆) alkenyl; and the like);
(5) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc.)
(6) aryl which may be substituted (for example, phenyl, naphthyl, etc.);
   and the like
   is bonded to a carbonyloxy group, preferably carboxyl, lower (C₁₋₆) alkoxycarbonyl, aryloxycarbonyl (for example, methoxycarbonyl, ethoxycarbonyl, propoxycarbonyl, phenoxycarbonyl, naphthoxycarbonyl, etc.), and the like.

Examples of the substituent of the above-described (2) alkyl which may be substituted, (3) cycloalkyl which may be substituted, (4) alkenyl which may be substituted, (5) cycloalkenyl which may be substituted, and (6) aryl which may be substituted, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkoxy which may be halogenated (for example, methoxy, ethoxy, propoxy, butoxy, trifluoromethoxy, trifluoroethoxy, etc.), C₁₋₄ alkoxy-C₁₋₄ alkoxy which may halogenated (for example, methoxymethoxy, methoxyethoxy, ethoxyethoxy, trifluoromethoxyethoxy, trifluoroethoxyethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The "aromatic group" of the "aromatic group which may be substituted" as the substituent of R¹ may be exemplified by a 5- or 6-membered homocyclic or heterocyclic aromatic group such as phenyl, pyridyl, furyl, thienyl, pyrrolyl, imidazolyl, pyrazolyl, thiazolyl, oxazolyl, isothiazolyl, isoxazolyl, tetrazolyl, pyrazinyl, pyrimidinyl, pyridazinyl, triazolyl, etc.; a fused-ring heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; and the like. Examples of the substituent of the aromatic group include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.); C₁₋₄ alkyl which may be halogenated (for example, trifluoromethyl, methyl, ethyl, etc.); C₁₋₄ alkoxy which may be halogenated (for example, methoxy, ethoxy, propoxy, butoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The number of the above substituents of R¹ may be 1 to 4, and preferably 1 to 2, and the substituents which may be the same or different from each other, and may be present at any possible positions of the ring. When the "5- or 6-membered ring" of the "5- to 6-membered ring which may be substituted" represented by R¹ has two or more substituents, two of the substituents may be bonded to each other to form, for example, lower (C₁₋₆) alkylene (for example, trimethylene, tetramethylene, etc.), lower (C₁₋₆) alkyleneoxy (for example, -CH₂-O-CH₂-, -O-CH₂-CH₂-, -O-CH₂-CH₂-CH₂-, -O-CH₂-CH₂-CH₂-CH₂-, -O-C(CH₃) (CH₃)-CH₂-CH₂-, etc.), lower (C₁₋₆) alkylenethio (for example, -CH₂-S-CH₂-, -S-CH₂-CH₂-, -S-CH₂-CH₂-CH₂-, -S-CH₂-CH₂-CH₂-CH₂-, -S-C(CH₃)(CH₃)-CH₂-CH₂-, etc.), lower (C₁₋₆) alkylenedioxy (for example, -O-CH₂-O-, -O-CH₂-CH₂-O-, -O-CH₂-CH₂-CH₂-O-, etc.), lower (C₁₋₆) alkylenedithio (for example, - S-CH₂-S-, -S-CH₂-CH₂-S-, -S-CH₂-CH₂-CH₂-S-, etc.), lower (C₁₋₆) alkyleneamino (for example, -O-CH₂-NH-, -O-CH₂-CH₂-NH-, etc.), oxy-lower (C₁₋₆) alkylenethio (for example, -O-CH₂-S-, -O-CH₂-CH₂-S-, etc.), lower (C₁₋₆) alkylenediamino (for example, -NH-CH₂-NH-, -NH-CH₂-CH₂-NH-, etc.), thialower (C₁₋₆) alkyleneamino (for example, -S-CH₂-NH-, -S-CH₂-CH₂-NH-, etc.), lower (C₂₋₆) alkenylene (for example, -CH₂-CH=CH-, -CH₂-CH₂-CH=CH-, -CH₂-CH=CH-CH₂-, etc.), lower (C₄₋₆) alkadienylene (for example, -CH=CH-CH=CH-, etc.), and the like.

Further, the divalent group formed by the binding of two substituents of R¹ may contain 1 to 3 substituents which are the same as the "substituents" of the "5- or 6-membered ring" of the "5- or 6-membered ring which may be substituted" represented by R¹ (halogen, nitro, cyano, alkyl which may be substituted, cycloalkyl which may be substituted, hydroxy group which may be substituted, thiol group which may be substituted (wherein the sulfur atom may be oxidized, or may form sulfinyl group which may be substituted or sulfonyl group which may be substituted), amino group which may be substituted, acyl which may be substituted, carboxyl group which may be esterified or amidated, an aromatic group which may be substituted, and the like).

The "substituent" of the "5- or 6-membered ring" of the "5- or 6-membered ring group which may be substituted" represented by R¹ may be exemplified by, in particular, lower (C₁₋₄) alkyl which may be halogenated or lower (C₁₋₄) alkoxylated (for example, methyl, ethyl, t-butyl, trifluoromethyl, methoxymethyl, ethoxymethyl, propoxymethyl, butoxymethyl, methoxyethyl, ethoxylethyl, propoxyethyl, butoxyethyl, etc.), lower (C₁₋₄) alkoxy which may be halogenated or lower (C₁₋₄) alkoxylated (for example, methoxy, ethoxy, propoxy, butoxy, t-butoxy, trifluoromethoxy, methoxymethoxy, ethoxymethoxy, propoxymethoxy, butoxymethoxy, methoxyethoxy, ethoxyethoxy, propoxyethoxy, butoxyethoxy, methoxypropoxy, ethoxypropoxy, propoxypropoxy, butoxypropoxy, etc.), halogen (for example, fluorine, chlorine, etc.), nitro, cyano, amino which may be substituted with one or two of lower (C₁₋₄) alkyl, formyl or lower (C₂₋₄) alkanoyl (for example, amino, methylamino, dimethylamino, formylamino, acetylamino, etc.), 5- or 6-membered cycloamino (for example, 1-pyrrolidinyl, 1-piperazinyl, 1-piperidinyl, 4-morpholino, 4-thiomorpholino, 1-imidazolyl, 4-tetrahydropyranyl, etc.), and the like.

Examples of the lower alkyl group of the "lower alkyl group which may be substituted" represented by R³ and R^{3'} above include C₁₋₆ alkyl such as methyl, ethyl, propyl, isopropyl, butyl, isobutyl, sec-butyl, tert-butyl, pentyl, isopentyl, neopentyl, hexyl, etc., and the like.

Examples of the lower alkoxy group of the "lower alkoxy group which may be substituted" represented by R³ and R^{3'} above include C₁₋₆ alkoxy such as methoxy, ethoxy, propoxy, butoxy, etc., and the like.

Examples of the substituent which may be carried by the "lower alkyl group which may be substituted" and "lower alkoxy group which may be substituted" include halogen (for example, fluorine, chlorine, bromine, iodine), hydroxy group, amino, mono(lower alkyl)amino, di(lower alkyl)amino, lower alkanoyl and the like.

The lower alkyl carried by the mono(lower alkyl)amino and di(lower alkyl)amino may be exemplified by the same lower alkyl group of the "lower alkyl group which may be substituted" represented by R³ and R^{3'} above.

The lower alkanoyl may be exemplified by C₂₋₆ alkanoyl such as acetyl, propionyl, butyryl, isobutyryl, etc.

Among them, for R³ and R^{3'}, the lower C₁₋₆ alkyl group which may be substituted is preferred, and particularly a methyl group which may be substituted is preferred.

With respect to the group represented by NR⁴ shown in Y above, the "lower alkyl group" of the "lower alkyl group which may be substituted" represented by R⁴ may be exemplified by C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, and more preferably lower (C₁₋₄) alkyl). Examples of the substituent which may be carried by the above-described lower alkyl group, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc.), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkyl which may be halogenated (for example, trifluoromethyl, methyl, ethyl, etc.), C₁₋₄ alkoxy which may be halogenated (for example, methoxy, ethoxy, propoxy, butoxy, trifluoromethoxy, trifluoroethoxy, etc.), C₁₋₄ alkylenedioxy (for example, -O-CH₂-O-, -O-CH₂-CH₂-O-, etc.), sulfonamide which may be substituted [for example, a group formed by bonding amino group which may be substituted (for example, amino, mono-C₁₋₄ alkylamino, di-C₁₋₄ alkylamino, 5- or 6-membered cycloamino such as terahydropyrrole, piperazine, piperidine, morpholine, thiomorpholine, pyrrole, imidazole, etc.) with -SO₂-, etc.], formyl, C₂₋₄ alkanoyl (for example, acetyl, propionyl, etc.), C₁₋₄ alkylsulfonyl (for example, methanesulfonyl, ethanesulfonyl, etc.), heterocyclic group which may be substituted, and the like, and the number of the substituents is preferably 1 to 3.

The "heterocyclic group" of the above-described "heterocyclic group which may be substituted" may be exemplified by a group formed by eliminating one hydrogen atom from a heterocyclic aromatic group or a non-heterocyclic aromatic group, and the like. Examples of such heterocyclic aromatic group include 5- or 6-membered heterocyclic aromatic group containing 1 to 4 heteroatoms of one or two kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole and the like, while examples of such non-heterocyclic aromatic group include 5- or 6-membered non-aromatic heterocyclic group containing 1 to 4 heteroatoms of one or two kinds selected from a nitrogen atom, a sulfur atom and an oxygen atom, such as tetrahydrofuran, tetrahydrothiophene, dioxolane, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc., and non-heterocyclic aromatic group in which all or part of the bonds in the above-mentioned heterocyclic aromatic groups are saturated bonds (preferably, a heterocyclic aromatic group such as pyrazole, thiazole, oxazole, tetrazole, etc.).

The lower alkyl group which may be substituted is preferably C₁₋₆ alkyl which may be halogenated or hydroxylated, or C₂₋₆ alkenyl which may be halogenated or hydroxylated.

The "lower acyl group which may be substituted" represented by R⁴ may be exemplified by the same ones as the "acyl group which may be substituted" as the substituent which may be carried by the "5- or 6-membered cyclic group" of the "5- or 6-membered group which may be substituted" represented by R¹, and among these, C₁₋₄ alkylsulfonyl which may be halogenated or hydroxylated, formyl, C₂₋₅ alkanoyl which may be halogenated or hydroxylated, and the like are preferred.

For R⁴, C₁₋₄ alkyl which may be halogenated or hydroxylated, formyl, C₂₋₅ alkanoyl which may be halogenated or hydroxylated, and the like are more preferred.

n and n' are preferably each 1 or 2, and particularly preferably 2.

In the formula (I) above, the "5- or 6-membered aromatic ring which may be substituted" represented by Z¹ may be exemplified by 6-membered aromatic hydrocarbon such as benzene, etc.; a 5- to 6-membered aromatic heterocycle containing 1 to 4 heteroatoms of one or two kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, etc.; a fused aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; and the like. Among them, preferred are benzene, furan, thiophene, pyridine, pyridazine, pyrimidine, benzimidazole and the like, and particularly preferably used are benzene, pyridine, pyridazine and benzimidazole (preferably benzene).

The "5- or 6-membered aromatic ring which may be substituted" represented by Z¹ may have the same substituents as the "substituent" which may be carried by the "5- or 6-membered ring" of the "5- or 6-membered cyclic group which may be substituted" represented by R¹, and among them, a halogen atom (for example, fluorine, chlorine, bromine, etc.), a C₁₋₄ alkyl group which may be halogenated (for example, methyl, ethyl, trifiluoromethyl, trifluoroethyl, etc.), a C₁₋₄ alkoxy group which may be halogenated (for example, methoxy, ethoxy, propoxy, trifluoromethoxy, trifluoroethoxy, etc.) and the like are preferred as the substituent. However, it is preferred that there is no other substituent than X² and Z², and it is preferred that when Z¹ is a 6-membered ring (preferably benzene), the position of substitution of Z² is para to X². Further, for Z¹, benzene which may be substituted with 1) a halogen atom, 2) a C₁₋₄ alkyl group which may be substituted with a halogen atom or 3) a C₁₋₄ alkoxy group which may be substituted with a halogen atom is preferred, and in particular, benzene which may be substituted with a methyl group or a trifluoromethyl group is preferred.

In the formula (I) above, with respect to the formulas -Z^{2a}-W¹-Z^{2b}- and -Z^{2a}-W²-Z^{2b}- represented by Z², the substituent (R^{a}) of the "imino group which may be substituted" represented by each of Z^{2a} and Z^{2b} may be exemplified by a hydrogen atom, lower (C₁₋₆) alkyl which may be substituted [for example, methyl, ethyl, propyl, isopropyl, butyl, isobutyl, sec-butyl, tert-butyl, pentyl, isopentyl, neopentyl, hexyl, hydroxy-C₁₋₆ alkyl (for example, hydroxyethyl, hydroxypropyl, hydroxybutyl, etc.), halogenated C₁₋₆ alkyl (for example, trifluoromethyl, trifluoroethyl, etc.), cyanated C₁₋₆ alkyl (for example, cyanoethyl, cyanopropyl, etc.), carboxyl-C₁₋₆ alkyl which may be esterified or amidated, etc.], formyl, lower (C₂₋₅) alkanoyl (for example, acetyl, propionyl, butyryl, etc.), lower (C₁₋₅) alkylsulfonyl (methylsulfonyl, ethylsulfonyl, etc.), and the like.

The alkylene chain of the "alkylene group which may be substituted" represented by W¹ and W² may be exemplified by the alkylene chain represented by -(CH₂)ₖ₁- (wherein k1 is an integer of 1 to 4) and the like. The alkenylene group of the "alkenylene group which may be substituted" represented by W¹ may be exemplified by the alkenylene chain represented by -(CH₂)ₖ₂-(CH=CH)-(CH₂)ₖ₃- (wherein k2 and k3 are the same or different from each other, and represent 0, 1 or 2, respectively, provided that the sum of k2 and k3 is 2 or less) and the like.

The alkylene group and the alkenylene group represented by the above-described W¹ and W² may be substituted at any arbitrary position (preferably on a carbon atom), and such substituent may be any substituent capable of bonding to the alkylene chain or the alkenylene chain which constitutes the straight chain moiety. Examples thereof include lower (C₁₋₆) alkyl (for example, methyl, ethyl, propyl, isopropyl, butyl, isobutyl, sec-butyl, tert-butyl, pentyl, isopentyl, neopentyl, hexyl, etc.), lower (C₃₋₇)cycloalkyl (for example, cyclopropyl, cyclobutyl, cyclopentyl, cyclohexyl, cycloheptyl, etc.), formyl, lower (C₂₋₇) alkanoyl (for example, acetyl, propionyl, butyryl, etc.), a phosphono group which may be esterified, carboxyl group which may be esterified or amidated, hydroxy group, oxo, hydroxyimino group, lower (C₁₋₆) alkoxyimino group which may be substituted and the like, and preferably lower alkyl having 1 to 6 carbon atoms (preferably, C₁₋₃ alkyl), hydroxy group, oxo, hydroxyimino group, lower (C₁₋₆) alkoxyimino group (which may be substituted with polar group such as hydroxy group, cyano group, carboxy group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), and the like) and the like.

The phosphono group which may be esterified may be exemplified by a group represented by P(O)(OR⁷)(OR⁸), wherein R⁷ and R⁸ are each hydrogen, an alkyl group having 1 to 6 carbon atoms, or a cycloalkyl group having 3 to 7 carbon atoms, or R⁷ and R⁸ may be bonded to each other to form a 5-to 7-membered ring.

In the above-described formula, the alkyl group having 1 to 6 carbon atoms represented by R⁷ and R⁸ may be exemplified by methyl, ethyl, propyl, isopropyl, butyl, isobutyl, sec-butyl, tert-butyl, pentyl, isopentyl, neopentyl, hexyl and the like, and the cycloalkyl having 3 to 7 carbon atoms may be exemplified by cyclopropyl, cyclobutyl, cyclopentyl, cyclohexyl, cycloheptyl and the like. Preferred is linear lower alkyl having 1 to 6 carbon atoms, and more preferred is lower alkyl having 1 to 3 carbon atoms. R⁷ and R⁸ may be the same or different from each other, preferably the same. Further, when R⁷ and R⁸ are bonded to each other to form a 5- to 7-membered ring, R⁷ and R⁸ are bonded to each other to form a linear C₂₋₄ alkylene side chain represented by -(CH₂)₂-, -(CH₂)₃- or -(CH₂)₄-. This side chain may be substituted, and examples of the substituent include hydroxy group, halogen and the like.

The ester product of the above-described carboxyl group which may be esterified may be exemplified by a product in which a carboxyl group is bonded to an alkyl group having 1 to 6 carbon atoms or a cycloalkyl group having 3 to 7 carbon atoms, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl, cyclopentyloxycarbonyl, cyclohexyloxycarbonyl and the like.

The amide product of the above-described carboxyl group which may be amidated may be exemplified by a product in which a carboxyl group is bonded to an alkylamino group having 1 to 6 carbon atoms, a cycloalkylamino group having 3 to 7 carbon atoms or 5- to 8-membered cyclic amine (for example, pyrrolidine, piperidine, morpholine, etc.), for example, carbamoyl, mono-C₁₋₆ alkylcarbamoyl, di-C₁₋₆ alkylcarbamoyl, cyclopentylaminocarbonyl, cyclohexylaminocarbonyl, pyrrolidinocarbonyl, piperidinocarbonyl, morpholinocarbonyl, thiomorpholinocarbonyl and the like.

For Z², preferably, one of Z^{2a} and Z^{2b} is O, S(O)_{q} (wherein q is 0, 1 or 2), or -N(R^{a})- (wherein R^{a} is a hydrogen atom or a lower C₁₋₄ alkyl which may be substituted), the other being a bond, and W is -(CH₂)ᵣ- (wherein r is an integer of 1 to 3), or Z² is a divalent group of the formula -CH(OH)-. More preferably, one of Z^{2a} and Z^{2b} is O or S(O)_{q} (wherein q is 0, 1 or 2), the other being a bond, and W is -(CH₂)ᵣ- (wherein r is an integer of 1 to 3), or Z² is a divalent group of the formula -CH(OH)-. Even more preferably, Z² is -CH₂-, -CH(OH)-, -S(O)_{q}-CH₂- (wherein q is 0, 1 or 2), with -S(O)_{q}-CH₂- (wherein q is 0, 1 or 2) being particularly preferred. In particular, when Z^{2a} is bonded to Z¹, Z² is preferably -SOCH₂-.

Z^{2a} represents a bond, S, SO or SO₂, and among them, SO is preferred. In this case, the configuration of SO is preferably (S).

The bonding position of Z² with respect to Z¹ is such that when Z¹ is a benzene ring for example, any position may be selected, but the para position is preferred.

In the above formula (I), the "amino group which may be substituted, in which the nitrogen atom may be converted to a quaternary ammonium or an oxide" represented by R² may be exemplified by an amino group which may have 1 or 2 substituents, an amino group which has 3 substituents, in which the nitrogen atom is converted to a quaternary ammonium, and the like. When the amino group has two or more substituents on its nitrogen atom, the substituents may be the same or different from each other; and when the nitrogen atom has 3 substituents, the amino group may be of any type among the following formulas, -N⁺R^{p}R^{p}R^{p,} -N⁺R^{p}R^{p}R^{q}, and -N⁺R^{p}R^{q}R^{r}, wherein R^{p}, R^{q}, and R^{r} are different from each other and represent hydrogen or a substituent, respectively. Examples of the counter anion of the amino group, in which the nitrogen atom is converted to a quaternary ammonium include, in addition to anions of the halogen atom (for example, Cl⁻, Br⁻, I⁻, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; anions derived from acidic amino acids such as aspartic acid, glutamic acid, etc.; and the like, and among them, Cl⁻, Br⁻, I⁻ and the like are preferred.

Examples of the substituent of the above-described amino group include:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, and the like); and
(2) cycloalkyl which may be substituted (for example, C₃₋₈ cycloalkyl such as cyclopropyl, cyclobutyl, cyclopentyl, cyclohexyl, cycloheptyl, cyanooctyl, etc., and the like);
   (2-1) the cycloalkyl may contain one heteroatom selected from a sulfur atom, an oxygen atom and a nitrogen atom, forming oxirane, thiolane, aziridine, tetrahydrofuran, tetrahydrothiophene, pyrrolidine, tetrahydropyran, tetrahydrothiopyran, tetrahydrothiopyran 1-oxide, piperidine, etc. (preferably, a 6-membered ring such as tetrahydropyran, tetrahydrothiopyran, piperidine, etc.), and the bond with the amino group may be preferably present at the 3- or 4-position (preferably at the 4-position);
   (2-2) also, the cycloalkyl may be fused to a benzene ring, forming indane (for example, indan-1-yl, indan-2-yl, etc.), tetrahydronaphthalene (for example, tetrahydronaphthalen-5-yl, tetrahydronaphthalen-6-yl, etc.), and the like (preferably, indane, etc.);
   (2-3) further, the cycloalkyl may be bridged via a straight atomic chain having 1 or 2 carbon atoms, forming a bridged cyclic hydrocarbon residue such as bicyclo[2.2.1]heptyl, bicyclo[2.2.2]octyl, bicyclo[3.2.1]octyl, bicyclo[3.2.2]nonyl, etc. (preferably, cyclohexyl bridged via a straight atomic chain having 1 or 2 carbon atoms, and more preferably, bicyclo[2.2.1]heptyl, etc.);
(3) alkenyl which may be substituted (for example, alkenyl having 2 to 10 carbon atoms, such as allyl, crotyl, 2-pentenyl, 3-hexenyl, etc., preferably lower (C₂₋₆) alkenyl, and the like) ;
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cylcohexenylmethyl, etc.);
(5) aralkyl which may be substituted (for example, phenyl-C₁₋₄ alkyl (for example, benzyl, phenethyl, etc.), and the like);
(6) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.), alkoxycarbonyl having 1 to 4 carbon atoms (for example, methoxycarbonyl, ethoxycarbonyl, tert-butoxycarbonyl, etc.), aralkyloxycarbonyl having 7 to 10 carbon atoms (for example, benzyloxycarbonyl, etc.), and the like) ;
(7) aryl which may be substituted (for example, phenyl, naphthyl, etc.);
(8) a heterocyclic group which may be substituted (for example, a group formed by eliminating a hydrogen atom from a 5- or 6-membered aromatic heterocycle containing 1 to 4 heteroatoms of one or two kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc., or from a fused heterocyclic aromatic group such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a group formed by eliminating a hydrogen atom from a 5- or 6-membered non-aromatic heterocycle containing 1 to 4 heteroatoms of one or two kinds selected from a nitrogen atom, a sulfur atom and an oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, etc.; and the like; preferably, a group formed by eliminating a hydrogen atom from a 5- or 6-membered non-aromatic heterocycle, etc.; more preferably, a group formed by eliminating a hydrogen atom from a 5- or 6-membered non-aromatic heterocycle containing one heteroatom, such as tetrahydrofuran, piperidine, tetrahydropyran, tetrahydrothiopyran, etc.); and the like. The substituents on the amino group may be bonded to each other to form 5- to 7-membered cycloamino such as piperidine, piperazine, morpholine, thiomorpholine, etc.

Examples of the substituent which may be carried by the above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, (7) aryl which may be substituted, and (8) a heterocyclic group which may be substituted, include halogen (for example, fluorine, chlorine, bromine, iodine, etc.); lower (C₁₋₄) alkyl which may be halogenated; lower (C₁₋₄) alkyl which may be substituted with a polar group such as a hydroxy group, a cyano group, a carboxyl group which may be esterified or amidated, etc. (for example, hydroxy-C₁₋₄ alkyl, cyano-C₁₋₄ alkyl, carboxyl-C₁₋₄ alkyl, C₁₋₄ alkoxycarbonyl-C₁₋₄ alkyl, carbamoyl-C₁₋₄ alkyl, mono-C₁₋₄ alkylcarbamoyl-C₁₋₄ alkyl, di-C₁₋₄ alkylcarbamoyl, di-C₁₋₄ alkylcarbamoyl-C₁₋₄ alkyl, pyrrolidinocarbonyl-C₁₋₄ alkyl, piperidinocarbonyl-C₁₋₄ alkyl, morpholinocarbonyl-C₁₋₄ alkyl, thiomorpholinocarbonyl-C₁₋₄ alkyl, etc.); C₁₋₄ alkoxy which may be halogenated (for example, methoxy, ethoxy, propoxy, butoxy, trifluoromethoxy, trifluoroethoxy, etc.); C₁₋₄ alkylenedioxy (for example, -O-CH₂-O-, -O-CH₂-CH₂-O-, etc.); formyl; C₂₋₄ alkanoyl (for example, acetyl, propionyl, etc.); C₁₋₄ alkylsulfonyl (for example, methanesulfonyl, ethanesulfonyl, etc.); phenyl-lower (C₁₋₄) alkyl; C₃₋₇ cycloalkyl; cyano; nitro; hydroxy group; thiol group which may be substituted (for example, thiol, C₁₋₄ alkylthio, etc.); amino group which may be substituted (for example, amino, mono-C₁₋₄ alkylamino, di-C₁₋₄ alkylamino, 5- or 6-membered cycloamino such as tetrahydropyrrole, piperazine, piperidine, morpholine, thiomorpholine, pyrrole, imidazole, etc.); carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.); lower (C₁₋₄) alkoxy-carbonyl; lower (C₇₋₁₀) aralkyloxycarbonyl; oxo group (preferably, halogen, lower (C₁₋₄) alkyl which may be halogenated, lower (C₁₋₄) alkoxy which may be halogenated, phenyl-lower (C₁₋₄) alkyl, C₃₋₇ cycloalkyl, cyano, hydroxy group, etc); and the like. The number of the substituents is preferably 1 to 3.

In the above formula (I), the "amino group which may be substituted, in which the nitrogen atom may be converted to a quaternary ammonium or an oxide" represented by R² is preferably an amino group which may have 1 to 3 substituents selected from:
(1) linear or branched lower (C₁₋₆) alkyl which may be substituted with one to three of halogen, cyano, hydroxy group or C₃₋₇ cycloalkyl;
(2) C₅₋₈ cycloalkyl which may be substituted with one to three of halogen, lower (C₁₋₄) alkyl which may be halogenated, or phenyl-lower (C₁₋₄) alkyl, which may contain one heteroatom selected from a sulfur atom, an oxygen atom and a nitrogen atom, which may be fused to a benzene ring, and which may be bridged via a straight atomic chain having 1 or 2 carbon atoms (for example, cyclopentyl, cyclohexyl, cycloheptyl, cyclooctyl, tetrahydropyranyl, tetrahydrothiapyranyl, piperidinyl, indanyl, tetrahydronaphthalenyl, bicyclo[2.2.1]heptyl, etc., each of which may be substituted);
(3) phenyl-lower (C₁₋₄) alkyl which may have one to three of halogen, lower (C₁₋₄) alkyl which may be halogenated, or lower (C₁₋₄) alkoxy which may be halogenated;
(4) phenyl which may have one to three of halogen, lower (C₁₋₄) alkyl which may be halogenated, or lower (C₁₋₄) alkoxy which may be halogenated; and
(5) a 5- to 6-membered heterocyclic aromatic group which may have one to three of halogen, lower (C₁₋₄) alkyl which may be halogenated, lower (C₁₋₄) alkoxy groups which may be halogenated, lower (C₁₋₄) alkoxy-lower (C₁₋₄) alkoxy which may be halogenated, phenyl-lower (C₁₋₄) alkyl, cyano, or hydroxy group (for example, a group formed by eliminating one hydrogen atom from furan, thiophene, pyrrole, pyridine, etc.).

In the above formula (I), the "nitrogen-containing heterocycle" of the "nitrogen-containing heterocyclic group which may be substituted and may contain a sulfur atom or an oxygen atom as the ring-constituting atom, in which the nitrogen atom may be converted to a quaternary ammonium or an oxide" represented by R², may be exemplified by a 5- to 6-membered aromatic heterocycle containing 1 to 4 heteroatoms of one or two kinds selected from a nitrogen atom, a sulfur atom and an oxygen atom, such as pyrrole, imidazole, pyrazole, thiazole, oxazole, isothiazole, isoxazole, tetrazole, pyridine, pyrazine, pyrimidine, pyridazine, triazole, oxadiazole, thiadiazole, etc.; a fused aromatic heterocycle such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a 5- to 8-membered non-aromatic heterocycle containing 1 to 3 heteroatoms of one or two kinds selected from a nitrogen atom, a sulfur atom and an oxygen atom in addition to one nitrogen atom, such as pyrrolidine, pyrroline, imidazolidine, imidazoline, pyrazolidine, pyrazoline, piperidine, piperazine, oxazine, oxadiazine, thiazine, thiadiazine, morpholine, thiomorpholine, azacycloheptane, azacyclooctane (azocane), etc.; and the like, and these nitrogen-containing heterocycles may be bridged via a straight atomic chain having 1 or 2 carbon atoms, forming a bridged-ring nitrogen-containing heterocycle such as azabicyclo[2.2.1]heptane, azabicyclo[2.2.2]octane (quinuclidine), etc. (preferably, piperidine bridged via a straight atomic chain having 1 or 2 carbon atoms, etc.).

Among specific examples of the above-described nitrogen-containing heterocycle, preferred are pyridine, pyridazine, pyrazole, imidazole, triazole, tetrazole, imidazopyridine, pyrrolidine, piperidine, piperazine, morpholine, thiomorpholine and azabicyclo[2. 2.2]octane (preferably, pyridine, imidazole, triazole, imidazopyridine, pyrrolidine, piperidine and morpholine).

The nitrogen atom of the "nitrogen-containing heterocycle" may be converted to a quaternary ammonium or may be oxidized. When the nitrogen atom of the "nitrogen-containing heterocycle" is converted to a quaternary ammonium, the counter anion of the "nitrogen-containing heterocyclic group in which the nitrogen atom is converted to a quaternary ammonium" may be exemplified by, in addition to anions of a halogen atom (for example, Cl⁻, Br⁻, I⁻, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; anions derived from acidic amino acids such as aspartic acid, glutamic acid, etc.; and the like, and among them, Cl⁻, Br⁻, I⁻ and the like are preferred.

The "nitrogen-containing heterocyclic group" may be bonded to a divalent group represented by Z² via a carbon atom or a nitrogen atom, and may be bonded to a ring-constituting carbon atom as in 2-pyridyl, 3-pyridyl, 2-piperidinyl, etc., or to a ring-constituting nitrogen atom as in the following:

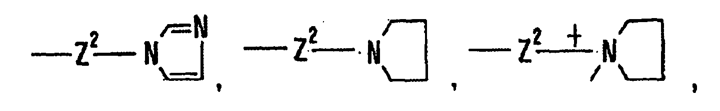

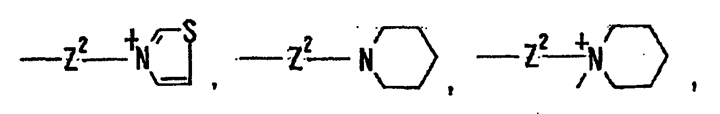

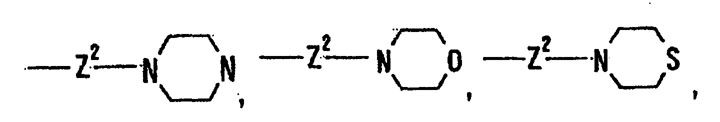

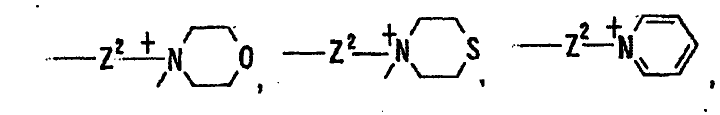

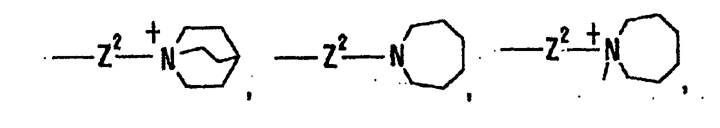

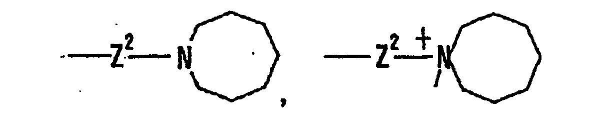

The substituent which may be carried by the "nitrogen-containing heterocycle" may be exemplified by a halogen atom (for example, fluorine, chlorine, bromine, iodine, etc.), lower (C₁₋₄) alkyl which may be substituted, lower (C₁₋₄) alkoxy which may be substituted, phenyl which may be substituted, mono- or diphenyl-lower (C₁₋₄) alkyl which may be substituted, C₃₋₇ cycloalkyl which may be substituted, cyano, nitro, hydroxy group, thiol group which may be substituted (for example, thiol, C₁₋₄ alkylthio, etc.), amino group group which may be substituted (for example, amino, mono-C₁₋₄ alkylamino, di-C₁₋₄ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), lower (C₁₋₄) alkoxy-carbonyl, formyl, lower (C₂₋₄) alkanoyl, lower (C₁₋₄) alkylsulfonyl, a heterocyclic group which may be substituted (for example, a group formed by eliminating a hydrogen atom from a 5- to 6-membered heterocyclic aromatic group containing 1 to 4 heteroatoms of one or two kinds selected from a nitrogen atom, a sulfur atom and an oxygen atom, such as furan, thiophene, pyrrole, imidazole, pyrazole, thiazole, oxazole, isothiazole, isoxazole, tetrazole, pyridine, pyrazine, pyrimidine, pyridazine, triazole, oxadiazole, thiadiazole, etc., or from a fused heterocyclic aromatic group containing 1 to 4 heteroatoms of one or two kinds selected from a nitrogen atom, a sulfur atom and an oxygen atom, such as benzofuran, indole, benzothiophene, benzoxazole, benzothiazole, indazole, benzimidazole, quinoline, isoquinoline, quinoxaline, phthalazine, quinazoline, cinnoline, imidazopyridine, etc.; a group formed by eliminating a hydrogen atom from a 5- to 6-membered non-aromatic heterocycle containing 1 to 4 heteroatoms of one or two kinds selected from a nitrogen atom, a sulfur atom and an oxygen atom, such as tetrahydrofuran, tetrahydrothiophene, dithiolane, oxathiolane, pyrrolidine, pyrroline, imidazolidine, imidazoline, pyrazolidine, pyrazoline, piperidine, piperazine, oxazine, oxadiazine, thiazine, thiadiazine, morpholine, thiomorpholine, pyran, tetrahydropyran, tetrahydrothiopyran, etc.), and the like, and the number of the substituents is preferably 1 to 3. The nitrogen atom in the "nitrogen-containing heterocycle" may be oxidized.

Examples of the substituent which may be carried respectively by the "lower (C₁₋₄) alkyl which may be substituted", the "lower (C₁₋₄) alkoxy which may be substituted", the "phenyl which may be substituted", the "mono- or diphenyl-lower (C₁₋₄) alkyl which may be substituted", the "C₃₋₇ cycloalkyl which may be substituted", and the "heterocyclic group which may be substituted", all of which are the substituents that may be carried by the "nitrogen-containing heterocycle", include a halogen atom (for example, fluorine, chlorine, bromine, iodine, etc.); lower (C₁₋₄) alkyl which may be halogenated; lower (C₁₋₄) alkyl which may be substituted by a polar group such as a hydroxy group, a cyano group, a carboxyl group which may be esterified or amidated, etc. (for example, hydroxy-C₁₋₄ alkyl, cyano-C₁₋₄ alkyl, carboxyl-C₁₋₄ alkyl, C₁₋₄ alkoxycarbonyl-C₁₋₄ alkyl, carbamoyl-C₁₋₄ alkyl, mono-C₁₋₄ alkylcarbamoyl-C₁₋₄ alkyl, di-C₁₋₄ alkylcarbamoyl-C₁₋₄ alkyl, pyrrolidinocarbonyl C₁₋₄ alkyl, piperidinocarbonyl C₁₋₄ alkyl, morpholinocarbonyl C₁₋₄ alkyl, thiomorpholinocarbonyl C₁₋₄ alkyl, etc.); lower (C₃₋₁₀) cycloalkyl; lower (C₃₋₁₀) cycloalkenyl; C₁₋₄ alkoxy which may be halogenated (for example, methoxy, ethoxy, trifluoromethoxy, trifluoroethoxy, etc.); formyl; C₂₋₄ alkanoyl (for example, acetyl, propionyl, etc.); C₁₋₄ alkylsulfonyl (for example, methanesulfonyl, ethanesulfonyl, etc.); C₁₋₃ alkylenedioxy (for example, methylenedioxy, ethylenedioxy, etc.); cyano; nitro; hydroxy group; thiol group which may be substituted (for example, thiol, C₁₋₄ alkylthio, etc.); amino group which may be substituted (for example, amino, mono-C₁₋₄ alkylamino, di-C₁₋₄ alkylamino, 5-to 6-membered cycloamino such as tetrahydropyrrole, piperazine, piperidine, morpholine, thiomorpholine, pyrrole, imidazole, etc.); carboxyl group which may be esterified or amidated (for example, carboxyl, C₁₋₄ alkoxy-carbonyl, carbamoyl, mono-C₁₋₄ alkylcarbamoyl, di-C₁₋₄ alkylcarbamoyl, etc.); lower (C₁₋₄) alkoxycarbonyl; and the like, and the number of the substituents is preferably 1 to 3.

In the above formula (I), the substituent which may be carried by the "nitrogen-containing heterocycle" of the "nitrogen-containing heterocyclic group which may be substituted and may contain a sulfur atom or an oxygen atom as the ring-constituting atom, in which the nitrogen atom may be a quaternary ammonium or an oxide" is preferably (1) halogen, (2) cyano, (3) hydroxy group, (4) carboxyl group, (5) carbamoyl group, (6) lower (C₁₋₄) alkoxy-carbonyl, (7) lower (C₁₋₄) alkylcarbamoyl, or 5- or 6-membered cycloamino (piperidino, morpholino, etc.)-carbonyl, (8) lower (C₁₋₄) alkyl which may be substituted with halogen, hydroxy group, cyano group, lower (C₁₋₄) alkoxy, or carboxyl which may be esterified or amidated, (9) lower (C₁₋₄) alkoxy which may be substituted with halogen, hydroxy group or lower (C₁₋₄) alkoxy, (10) phenyl which may be substituted with halogen, lower (C₁₋₄) alkyl, hydroxy group, lower (C₁₋₄) alkoxy or C₁₋₃ alkylenedioxy, (11) mono- or diphenyl-lower (C₁₋₄) alkyl which may be substituted with halogen, lower (C₁₋₄) alkyl, hydroxy group, lower (C₁₋₄) alkoxy or C₁₋₃ alkylenedioxy, or (12) a group formed by eliminating a hydrogen atom from a 5-or 6-membered aromatic heterocycle, such as furan, thiophene, pyrrole, pyridine, etc., and the like.

In the above formula (I), the "hydrocarbon group which may be substituted" represented by the formula (c) represented by R² may be exemplified by:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, and the like) ;
(2) cycloalkyl which may be substituted (for example, C₃₋₇ cycloalkyl such as cyclopropyl, cyclobutyl, cyclopentyl, cyclohexyl, cycloheptyl, etc., and the like);
(3) alkenyl which may be substituted (for example, alkenyl having 2 to 10 carbon atoms, such as allyl, crotyl, 2-pentenyl, 3-hexenyl, etc., preferably lower (C₂₋₆) alkenyl, and the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cylcohexenylmethyl, etc., and the like);
(5) alkynyl which may be substituted (for example, alkynyl having 2 to 10 carbon atoms, such as ethynyl, 1-propynyl, 2-propynyl, 1-butynyl, 2-pentynyl, 3-hexynyl, etc., preferably lower (C₂₋₆) alkynyl, and the like) ;
(6) aralkyl which may be substituted (for example, phenyl-C₁₋₄ alkyl (for example, benzyl, phenethyl, etc.) and the like) ;
(7) aryl which may be substituted (for example, phenyl, naphthyl, etc.); and the like. Examples of the substituents which may be carried by the above-described (1) alkyl which may be substituted, (2) cycloalkyl which may be substituted, (3) alkenyl which may be substituted, (4) cycloalkenyl which may be substituted, (5) alkynyl which may be substituted, (6) aralkyl which may be substituted, and (7) aryl which may be substituted, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkyl which may be halogenated (for example, trifluoromethyl, methyl, ethyl, etc.), C₁₋₄ alkoxy which may be halogenated (for example, methoxy, ethoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The "hydroxy group which may be substituted" represented by R⁵ and R⁶ may be exemplified by a hydroxy group which may have:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, and the like);
(2) cycloalkyl which may be substituted (for example, C₃₋₇ cycloalkyl such as cyclopropyl, cyclobutyl, cyclopentyl, cyclohexyl, cycloheptyl, etc., and the like);
(3) alkenyl which may be substituted (for example, alkenyl having 2 to 10 carbon atoms, such as allyl, crotyl, 2-pentenyl, 3-hexenyl, etc., preferably lower (C₂₋₆) alkenyl, and the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc., and the like);
(5) aralkyl which may be substituted (for example, phenyl-C₁₋₄ alkyl (for example, benzyl, phenethyl, etc.), and the like);
(6) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.), and the like);
(7) aryl which may be substituted (for example, phenyl, naphthyl, etc.); and the like.

Examples of the substituent which may be carried by the above-described (1) alkyl which may be substituted, (2) cycloalkyl which may be substituted, (3) alkenyl which may be substituted, (4) cycloalkenyl which may be substituted, (5) aralkyl which may be substituted, (6) acyl which may be substituted, and (7) aryl which may be substituted, include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- to 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkyl which may be halogenated (for example, trifluoromethyl, methyl, ethyl, etc.), C₁₋₄ alkoxy which may be halogenated (for example, methoxy, ethoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

Further, in the above-described formula, R⁵ and R⁶ may be bonded to each other to form a cyclic group (preferably, a 5- to 7-membered ring) together with the adjacent phosphorus atom. Such cyclic group may be substituted, and examples of the substituents include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- to 6-membered cycloamino such as tetrahydropyrrole, piperazine, piperidine, morpholine, thiomorpholine, pyrrole, imidazole, etc., and the like), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc.), C₁₋₄ alkyl which may be halogenated (for example, trifluoromethyl, methyl, ethyl, etc.), C₁₋₄ alkoxy which may be halogenated (for example, methoxy, ethoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

In the above formula (I), the counter anion for the case where the phosphorus atom forms a phosphonium salt, may be exemplified by, in addition to anions of a halogen atom (for example, Cl⁻, Br⁻, I⁻, etc.), anions derived from inorganic acids such as hydrochloric acid, hydrobromic acid, nitric acid, sulfuric acid, phosphoric acid, etc.; anions derived from organic acids such as formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.; and anions derived from acidic amino acids such as aspartic acid, glutamic acid, etc., and among them, Cl⁻, Br⁻, I⁻ and the like are preferred.

The amino group which may be substituted represented by R⁵ and R⁶ may be exemplified by an amino group which may have one or two of:
(1) alkyl which may be substituted (for example, C₁₋₁₀ alkyl such as methyl, ethyl, propyl, isopropyl, butyl, isobutyl, sec-butyl, tert-butyl, pentyl, isopentyl, neopentyl, hexyl, heptyl, octyl, nonyl, decyl, etc., preferably lower (C₁₋₆) alkyl, and the like);
(2) cycloalkyl which may be substituted (for example, C₃₋₇ cycloalkyl such as cyclopropyl, cyclobutyl, cyclopentyl, cyclohexyl, cycloheptyl, etc., and the like);
(3) alkenyl which may be substituted (for example, alkenyl having 2 to 10 carbon atoms, such as allyl, crotyl, 2-pentenyl, 3-hexenyl, etc., preferably lower (C₂₋₆) alkenyl and the like);
(4) cycloalkenyl which may be substituted (for example, cycloalkenyl having 3 to 7 carbon atoms, such as 2-cyclopentenyl, 2-cyclohexenyl, 2-cyclopentenylmethyl, 2-cyclohexenylmethyl, etc., and the like);
(5) formyl, or acyl which may be substituted (for example, alkanoyl having 2 to 4 carbon atoms (for example, acetyl, propionyl, butyryl, isobutyryl, etc.), alkylsulfonyl having 1 to 4 carbon atoms (for example, methanesulfonyl, ethanesulfonyl, etc.), and the like);
(6) aryl which may be substituted (for example, phenyl, naphthyl, etc.); and the like.

Examples of the substituent of the above-described (1) alkyl which may be substituted, (2) cycloalkyl which may be substituted, (3) alkenyl which may be substituted, (4) cycloalkenyl which may be substituted, (5) acyl which may be substituted, and (6) aryl which may be substituted include halogen (for example, fluorine, chlorine, bromine, iodine, etc.), nitro, cyano, hydroxy group, thiol group which may be substituted (for example, thiol, C₁₋₄ alkylthio, etc.), amino group which may be substituted (for example, amino, mono-C₁₋₄ alkylamino, di-C₁₋₄ alkylamino, 5- to 6-membered cycloamino such as tetrahydropyrrole, piperazine, piperidine, morpholine, thiomorpholine, pyrrole, imidazole, etc.), carboxyl group which may be esterified or amidated (for example, carboxyl, C₁₋₄ alkoxycarbonyl, carbamoyl, mono-C₁₋₄ alkylcarbamoyl, di-C₁₋₄ alkylcarbamoyl, etc,), C₁₋₄ alkyl which may be halogenated (for example, trifluoromethyl, methyl, ethyl, etc.); C₁₋₄ alkoxy which may be halogenated (for example, methoxy, ethoxy, trifluoromethoxy, trifluoroethoxy, etc.), formyl, C₂₋₄ alkanoyl (for example, acetyl, propionyl, etc.), C₁₋₄ alkylsulfonyl (for example, methanesulfonyl, ethanesulfonyl, etc.), and the like, and the number of the substituents is preferably 1 to 3.

The substituent of the "amidino group which may be substituted" and the "guanidino group which may be substituted" represented by R², may be exemplified by the same ones as the substituent of the above-described "amino which may be substituted in which the nitrogen atom may be converted to a quaternary ammonium or an oxide" represented by R².

R² is preferably (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted. R² is more preferably an amino group which may be substituted, in which the nitrogen group may be converted to a quaternary ammonium; a nitrogen-containing heterocyclic group which may be substituted, may contain a sulfur atom or an oxygen atom as the ring-constituting atom, in which the nitrogen group may be converted to an oxide; and the like, and particularly preferably an amino group which may be substituted; a nitrogen-containing heterocyclic group which may be substituted and may contain a sulfur atom or an oxygen atom as the ring-constituting atom; and the like.

R² is more preferably a group represented by the formula -NRR" or -N⁺RR'R'', wherein R, R' and R'' are each an aliphatic hydrocarbon group (a chained aliphatic hydrocarbon group and a cyclic aliphatic hydrocarbon group) which may be substituted, or an alicyclic (non-aromatic) heterocyclic group which may be substituted, or a nitrogen-containing heterocyclic aromatic group which may be substituted, in which the nitrogen atom may be converted to oxide.

In the above formulas, the "aliphatic hydrocarbon group which may be substituted" and the "alicyclic heterocyclic group which may be substituted" represented by R, R' and R'' may be exemplified by the same ones as the "aliphatic hydrocarbon group which may be substituted (for example, alkyl, cycloalkyl, alkenyl, cycloalkenyl, etc., each of which may be substituted)" and the "alicyclic heterocyclic group which may be substituted (for example, a 5- or 6-membered non-aromatic heterocycle which may be substituted, etc.)" that are exemplified as the substituent which may be carried by the "amino which may be substituted" represented by the substituent R².

Among them, R and R' are each preferably a chained hydrocarbon group which may be substituted (for example, alkyl, alkenyl, etc., each of which may be substituted), more preferably a C₁₋₆ alkyl group which may be substituted, and particularly preferably a methyl group which may be substituted.

R" is preferably an alicyclic hydrocarbon group which may be substituted (preferably, a C₃₋₈ cycloalkyl group which may be substituted; more preferably, cyclohexyl which may be substituted) or an alicyclic heterocyclic group which may be substituted (preferably, a saturated alicyclic heterocyclic group which may be substituted (preferably, a 6-membered cyclic group); more preferably, tetrahydropyranyl which may be substituted, tetrahydrothiopyranyl which may be substituted, or piperidyl which may be substituted; and particularly preferably, tetrahydropyranyl which may be substituted).

Furthermore, as the "nitrogen-containing heterocyclic aromatic group" of the "nitrogen-containing heterocyclic aromatic group which may be substituted, in which the nitrogen atom may be converted to an oxide" represented by R², pyridine, imidazole, triazole and imidazopyridine are exemplified as preferred, and among these, imidazole and triazole are particularly preferred.

The "amino group which may be substituted, in which the nitrogen atom may be converted to a quaternary ammonium or an oxide" represented by R^{2'} and R^{2"} and the like may be exemplified respectively by the same ones as the group corresponding to the above-described R².

The compound represented by the formula (I) is preferably the compound of the following:
(Ss)-8-[4-(2-butoxyethoxy)phenyl]-N-[[4-[(1-propyl-1H-imidazol-5-yl)-methyl]sulfinyl]phenyl]-2,3,3a,4-tetrahydro-1H-pyrrolo[1,2-a][1]benzazepine-5-carboxamide,
(Ss)-9-[4-(2-butoxyethoxy)phenyl]-N-[[4-[(1-propyl-1H-imidazol-5-yl)methyl]sulfinyl]phenyl]-1,2,3,4,4a,5-hexahydropyrido[1,2-a][1]benzazepine-6-carboxamide,
(Ss)-9-[4-(2-butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]benzazocine-6-carboxamide,
(Ss)-9-[4-(2-butoxyethoxy)phenyl]-N-[[4-[(1-propyl-1H-imidazol-5-yl)methyl]sulfinyl]phenyl]-1,2,3,4,4a,5-hexahydropyrido[1,2-a][1]benzazepine-6-carboxamide,
(Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide,
and diastereomers of the above five compounds,
(S)-10-[4-(2-butoxyethoxy)phenyl]-N-[[4-[(1-propyl-1H-imidazol-5-yl)-methyl]sulfinyl]phenyl]-2,3,5,6-tetrahydro-1H-azepino[3,2,1-ij]quinoline-7-carboxamide,
(S)-10-[4-(2-butoxyethoxy)phenyl]-N-[4-[[(1-propyl-1H-imidazol-5-yl)methyl]sulfinyl]phenyl]-1,2,5,6-tetrahydro-4H-azocino[3,2,1-hi]indole-7-carboxamide,
(S)-11-[4-(2-butoxyethoxy)phenyl]-N-[4-[[(1-propyl-1H-imidazol-5-yl)methyl]sulfinyl]phenyl]-2,3,6,7-tetrahydro-1H,5H-azocino[3,2,1-ij]quinoline-8-carboxamide,
(S)-11-[4-(2-butoxyethoxy)phenyl]-N-[4-[[(1-propyl-1H-imidazol-5-yl)methyl]sulfinyl]phenyl]-2,3,6,7-tetrahydro-5H-[1,4]oxazino[2,3,4-kl][1]benzoazocine-8-carboxamide, and the like.

The salt of the compound represented by the formula (I) of the present invention is preferably a pharmaceutically acceptable salt and may be exemplified by salts with inorganic bases, salts with organic bases, salts with inorganic acids, salts with organic acids, salts with basic or acidic amino acids, and the like. Preferred examples of the salt with inorganic base include alkali metal salts such as a sodium salt, a potassium salt, etc.; alkaline earth metal salts such as a calcium salt, a magnesium salt, etc.; an aluminum salt, an ammonium salt and the like. Preferred examples of the salt with organic base include salts with trimethylamine, triethylamine, pyridine, picoline, ethanolamine, diethanolamine, triethanolamine, dicyclohexylamine, N,N'-dibenzylethylenediamine and the like. Preferred examples of the salt with inorganic acid include salts with hydrochloric acid, hydrobromic acid, nitric acid, sulfuric acid, phosphoric acid and the like. Preferred examples of the salt with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like. Preferred examples of the salt with basic amino acid include salts with arginine, lysine, ornithine and the like. Preferred examples of the salt with acidic amino acid include salts with aspartic acid, glutamic acid and the like. The compound represented by the formula (I) of the present invention may be either hydrate or anhydrate. When the compound represented by the formula (I) of the present invention exists as a configurational isomer, a diastereomer, a conformer or the like, each form can be isolated by the separation and purification means known per se in the art, if desired. Further, when the compound represented by the formula (I) is present as a racemate, each of (S) and (R) isomer may be isolated by common means for optical resolution. Each of the optical isomers as well as the racemates is included in the scope of the present invention.

The prodrug of the compound represented by the formula (I) used in the present invention or a salt thereof [hereinafter, may be sometimes referred to as Compound (I)] refers to a compound which is converted to Compound (I) by an in vivo reaction caused by an enzyme, gastric acid or the like under physiological conditions, that is, a compound which is converted to Compound (I) upon occurrence of enzymatic oxidation, reduction, hydrolysis or the like, or a compound which is converted to Compound (I) upon occurrence of hydrolysis or the like by gastric acid or the like. The prodrug of Compound (I) may be exemplified by compounds resulting from acylation, alkylation or phosphorylation of the amino group of Compound (I) (for example, the compounds in which the amino group of Compound (I) is in the form of eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, tert-butyl or the like); the compounds resulting from acylation, alkylation, phosphorylation or boration of the hydroxy group of Compound (I) (for example, the compounds in which the hydroxy group of Compound (I) is in the form of acetyl, palmitoyl, propanoyl, pivaloyl, succinyl, fumaryl, alanyl, dimethylaminomethylcarbonyl or the like); the compounds resulting from esterification or amidation of the carboxyl group of Compound (I) (for example, the compounds in which the carboxyl group of Compound (I) is in the form of ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester, methyl amide or the like); or the like. These compounds can be prepared from Compound (I) by methods known per se in the art.

Furthermore, the prodrug of Compound (I) may be also a compound which is converted to Compound (I) under physiological conditions, as described in "Development of Pharmaceutical Products", Vol.7, Molecular Design, Hirokawa Publisher, pp.163-198 (1990).

Also, Compound (I) may be labeled with isotopes (for example, ³H, ¹⁴C, ³⁵S, ¹²⁵I, etc.).

Hereinafter, a process for producing the compound represented by the formula (I) or a salt thereof will be explained.

The compound represented by the formula (I) or the salt thereof can be produced by processes known per se. For example, it can be produced by the following processes. Alternatively, the compound represented by the formula (I) or the salt thereof can be produced by the process described in JP-A No. 8-73476 or a similar manner thereto.

The compounds which will be used in each of the following processes may form salts similar to the salt of Compound (I), as far as the salts do not interfere with the reactions.

Further, in the following reactions, when the starting compounds have an amino group, a carboxyl group or a hydroxy group as substituents, these groups may be protected by protective groups which are commonly used in peptide chemistry and the like, and if necessary, the protective groups can be removed after the reactions to obtain desired compounds.

Examples of the protective group to be used for an amino group include a C₁₋₆ alkylcarbonyl which may be substituted (for example, acetyl, propionyl, etc.), formyl, phenylcarbonyl, a C₁₋₆ alkyloxycarbonyl (for example, methoxycarbonyl, ethoxycarbonyl, t-butoxycarbonyl, etc.), a phenyloxycarbonyl (for example, benzoxycarbonyl, etc.), a C₇₋₁₀ aralkyloxycarbonyl (for example, benzyloxycarbonyl, etc.), trityl, phthaloyl and the like. The substituents of these protective groups include a halogen atom (for example, fluorine, chlorine, bromine, iodine, etc.), a C₁₋₆ alkylcarbonyl (for example, acetyl, propionyl, butyryl, etc.), a nitro group and the like, and the number of the substituents is about 1 to 3.

Examples of the protective group to be used for a carboxyl group include a C₁₋₆ alkyl which may be substituted (for example, methyl, ethyl, propyl, isopropyl, butyl, tert-butyl, etc.), phenyl, trityl, silyl and the like. The substituents of these protective groups include a halogen atom (for example fluorine, chlorine, bromine, iodine, etc.), C₁₋₆ alkylcarbonyl (for example, acetyl, propionyl, butyryl, etc.), formyl, a nitro group and the like, and the number of the substituents is about 1 to 3.

Examples of the protective groups to be used for a hydroxy group include a C₁₋₆ alkyl which may be substituted (for example, methyl, ethyl, propyl, isopropyl, butyl, tert-butyl, etc.), phenyl, a C₇₋₁₀ aralkyl (for example, benzyl, etc.), a C₁₋₆ alkylcarbonyl (for example, acetyl, propionyl, etc.), formyl, phenyloxycarbonyl, a C₇₋₁₀ aralkyloxycarbonyl (for example, benzyloxycarbonyl, etc.), pyranyl, furanyl, silyl and the like. The substituents of these protective groups include a halogen atom (for example, fluorine, chlorine, bromine, iodine, etc.), a C₁₋₆ alkyl, phenyl, a C₇₋₁₀ aralkyl, a nitro group and the like, and the number of the substituents is about 1 to 4.

Introduction and removal of protective groups are carried out according to the methods known per se or similar manners thereto [for example, the method described in "Protective Groups in Organic Chemistry", (J.F.W. McOmie et al., Plenum Press)], and removal is carried out by, for example, the methods of treating with an acid, a base, reduction, ultraviolet light, hydrazine, phenylhydrazine, sodium N-methyldithiocarbamate, tetrabutylammonium fluoride, palladium acetate, and the like.

In the following description, the compounds represented by the formulas (I), (Ia), (Ib), (II) and (III) including their salts may be also simply referred to as Compound (I), Compound (Ia), Compound (Ib), Compound (II) and Compound (III).

### [Process A]

Compound (I) can be prepared by reacting Compound (II) with Compound (III) according to the following reaction: 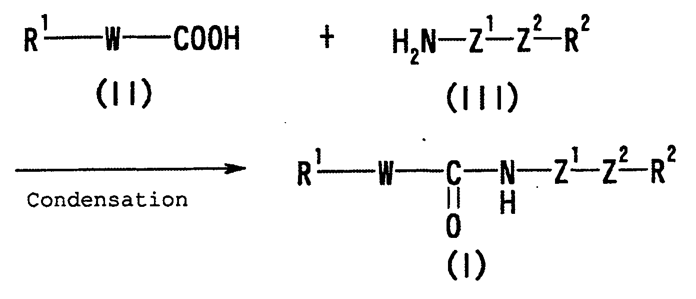 wherein each symbol has the same meaning as defined above.

In this reaction, a carboxylic acid derivative (II) is reacted with an amine derivative (III) to prepare Compound (I).

The condensation reaction of Compound (II) and Compound (III) may be carried out by a conventional means for peptide synthesis. The means for peptide synthesis may be carried out according to any method known in the art, for example, the methods described in M. Bodansky and M. A. Ondetti, Peptide Synthesis, Interscience, New York (1996); F. M. Finn and K. Hofmann, The Proteins, Vol. 2; H. Nenrath and R. L. Hill, Ed., Academic Press Inc., New York (1976); and Nobuo Izumiya et al. Foundations and Experiments in Peptide Synthesis, Maruzen Co. (1985), which include, for example, azide method, chloride method, acid anhydride method, mixed acid anhydride method, DCC method, activated ester method, method using Woodward's Reagent K, carbonyldiimidazole method, oxidation/reduction method, DCC/HONB method, as well as WSC method, diethyl cyanophosphate (DEPC) method and the like. In other words, examples of the reactive derivatives that may be used include acid halides (for example, acid chloride, acid bromide, etc.), acid azides, acid anhydrides, mixed acid anhydrides [for example, mixed acid anhydrides of mono-C₁₋₆ alkylcarbonic acid (for example, mixed acid anhydrides of a free acid with monomethylcarbonic acid, monoethylcarbonic acid, monoisopropylcarbonic acid, monoisobutylcarbonic acid, monotert-butylcarbonic acid, monobenzylcarbonic acid, mono(p-nitrobenzyl)carbonic acid, monoallylcarbonic acid, etc.), mixed acid anhydrides of C₁₋₆ aliphatic carboxylic acid (for example, mixed acid anhydrides of a free acid with acetic acid, trichloroacetic acid, cyanoacetic acid, propionic acid, butyric acid, isobutyric acid, valeric acid, isovaleric acid, pivalic acid, trifluoroacetic acid, trichloroacetic acid, acetoacetic acid, etc.), mixed acid anhydrides of C₇₋₁₂ aromatic carboxylic acid (for example, mixed acid anhydrides of a free acid with benzoic acid, p-toluic acid, p-chlorobenzoic acid, etc.), mixed acid anhydrides of organic sulfonic acid (for example, mixed acid anhydrides of a free acid with methanesulfonic acid, ethanesulfonic acid, benzenesulfonic acid, p-toluenesulfonic acid, etc.) and the like], activated amides, activated esters (for example, diethoxyphosphoric acid ester, diphenoxyphosphoric acid ester, p-nitrophenyl ester, 2,4-dinitrophenyl ester, etc.), activated thioesters (for example, 2-pyridylthiol ester, 2-benzothiazolylthiol ester, etc.) and the like. This condensation reaction can be carried out in a solvent. Examples of the solvent include N,N-dimethylformamide, dimethylsulfoxide, pyridine, chloroform, dichloromethane, tetrahydrofuran, dioxane, acetonitrile, each of which is dehydrated or hydrated, or appropriate mixtures thereof. The reaction temperature is usually about -20°C to about 50°C, and preferably about -10°C to about 30°C. The reaction time is about 1 to about 100 hours, and preferably about 2 to about 40 hours. The thus-obtained Compound (I) can be isolated and purified by known separation and purification means such as concentration, concentration under reduced pressure, solvent extraction, crystallization, recrystallization, resolubilization, chromatography and the like.

### [Process B]

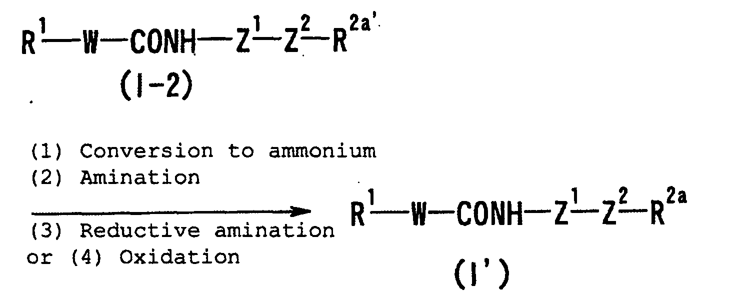

(1) When R^{2a'} as represented in Compound (I-2) is, for example, a tertiary amine, Compound (I-2) can be reacted with an alkyl halide or an aralkyl halide to preparequaternized Compound (I'). Herein, examples of the halogen atom include chlorine, bromine, iodine, etc., and the alkyl halide (for example, a lower (C₁₋₆) alkyl halide, etc.), or the aralkyl halide (for example, a lower (C₁₋₄) alkylphenyl halide, etc.) is typically used in an amount of about 1 to 5 moles with respect to 1 mole of Compound (I-2). The reaction may be carried out in an inert solvent, for example, toluene, benzene, xylene, dichloromethane, chloroform, 1,2-dichloroethane, dimethylformamide (DMF), dimethylacetamide, etc., or a mixture of the solvents above. The reaction temperature is in a range of about 10°C to about 160°C, and preferably about 20°C to about 120°C. The reaction time is about 1 to about 100 hours, and preferably about 2 to about 40 hours. The reaction is preferably carried out under an inert gas (for example, nitrogen, argon, etc.) atmosphere.
(2) When R^{2a'} as represented in Compound (I-2) is, for example, a secondary amine residue, Compound (I-2) can be reacted with an alkyl halide or an aralkyl halide to prepare tertiarized Compound (I'). Herein, examples of the halogen atom include chlorine, bromine, iodine, etc., and the alkyl halide or aralkyl halide is typically used in an amount of about 1 to 2 moles with respect to 1 mole of Compound (I-2). This reaction can be facilitated, if necessary, by adding about an equivalent to three-fold moles of a base such as triethylamine, diisopropylethylamine, pyridine, lithium hydride, sodium hydride, sodium methoxide, sodium ethoxide, sodium carbonate, potassium carbonate, sodium hydrogen carbonate and the like, and by further adding sodium iodide, potassium iodide, and the like.
   This reaction of tertiary amination may be carried out in an inert solvent such as methanol, ethanol, propanol, isopropanol, n-butanol, tetrahydrofuran, diethylether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature in a range of about 0°C to 180°C for about 1 to about 40 hours. Also, the reaction is preferably carried out under an inert gas (for example, nitrogen, argon, etc.) atmosphere.
(3) When R^{2a'} as represented in Compound (I-2) is, for example, a secondary amine residue, Compound (I-2) can be reacted with an aldehyde compound in the presence of a reductive amino reagent such as sodium triacetoxyborohydride, sodium cyanoborohydride, sodium borohydride and the like to prepare tertiarized Compound (I'). The reaction conditions for this reductive amination reaction are desirably varied depending on the reagent used. For example, when sodium triacetoxyborohydride is used, the reaction is preferably carried out in an inert solvent, for example, dichloromethane, chloroform, 1,2-dichloroethane, tetrahydrofuran (THF), diethylether, dioxane, acetonitrile, dimethylformamide (DMF), etc., or a mixture of the solvents above. The reagent is used in an amount of about 1 to 2 molar equivalents with respect to 1 mole of Compound (I-2). The reaction is usually carried out at a temperature in a range of about 0°C to about 80°C for about 1 to about 40 hours. This reaction is preferably carried out under an inert gas (for example, nitrogen, argon, etc.) atmosphere.
(4) When R^{2a'} as represented in Compound (I-2) is, for example, a sulfide residue or a tertiary amine residue, or when Z² is, for example, a sulfide, Compound (I-2) can be reacted with an oxidizing agent, for example, m-chloroperbenzoic acid, perbenzoic acid, p-nitroperbenzoic acid, magnesium monoperoxyphthalate, peracetic acid, hydrogen peroxide, sodium periodate, potassium periodate, etc., to prepare Compound (I') having a sulfinyl group, a sulfonyl group or an amine oxide group. The reaction conditions for this oxidation reaction are preferably varied depending on the oxidizing agent used. For example, when m-chloroperbenzoic acid is used, the reaction can be carried out in an inert solvent, for example, dichloromethane, chloroform, 1,2-dichloroethane, diethylether, tetrahydrofuran, acetone, ethyl acetate, etc., or a mixture of the solvents above. The oxidizing agent is used in an amount of about 1 to 3 molar equivalents with respect to 1 mole of Compound (I-2). The reaction is usually carried out at a temperature in a range of about -78°C to about 80°C (preferably from -50 to 25°C) for about 1 to about 40 hours.

When Z² as represented in Compound (I-2) is, for example, a sulfide residue, Compound (I') having an optically active sulfinyl group can be prepared according to the methods known per se in the art, for example, the method described in Ojima, I., Ed., Catalytic Asymmetric Synthesis, 2000, Wiley-VCH (New York), or a similar manner thereto.

### [Process C]

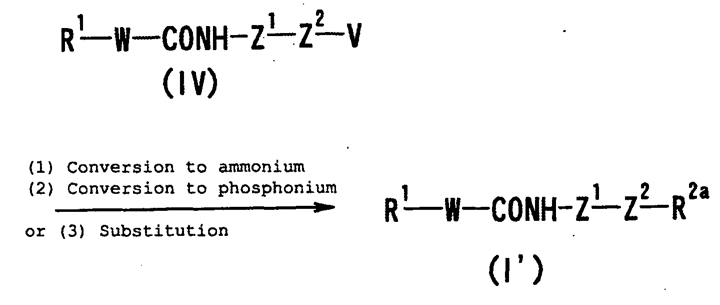

V as represented in Compound (IV) represents a halogen atom (chlorine, bromine, iodine, etc.), or a sulfonyloxy group (a methanesulfonyloxy group, a trifluoromethanesulfonyloxy group, a benzenesulfonyloxy group, a toluenesulfonyloxy group, etc.), and the other symbols have the same meanings as defined above.
(1) Compound (IV) can be reacted with a tertiary amine to prepare quaternized Compound (I'). This reaction may be carried out in an inert solvent, for example, toluene, benzene, xylene, dichloromethane, chloroform, 1,2-dichloroethane, dimethylformamide (DMF), dimethylacetamide, etc., or a mixture of the solvents above. The tertiary amine is used in an amount of about 1 to 3 moles with respect to 1 mole of Compound (IV). This reaction is carried out at a temperature in a range of about 10°C to about 120°C for about 1 to about 40 hours. The reaction is preferably carried out under an inert gas (for example, nitrogen, argon, etc.) atmosphere.
(2) Compound (IV) can be reacted with tertiary phosphine to prepare quaternized Compound (I'). This reaction may be carried out in an inert solvent, for example, toluene, benzene, xylene, dichloromethane, chloroform, 1,2-dichloroethane, acetonitrile, dimethylformamide (DMF), etc., or a mixture of the solvents above. The tertiary phosphine is used in an amount of about 1 to 2 moles with respect to 1 mole of Compound (IV). This reaction is carried out at a temperature in a range of about 20°C to about 150°C for about 1 to about 50 hours. This reaction is preferably carried out under an inert gas (for example, nitrogen, argon, etc.) atmosphere.
(3) Compound (IV) can be reacted with a primary or a secondary amine compound or a thiol compound to prepare Compound (I') having a secondary or tertiary amino group or a thio group. The primary or secondary amine compound or the thiol compound is usually used in an amount of about 1 to 3 moles with respect to 1 mole of Compound (IV). This reaction can be facilitated, if necessary, by adding about an equivalent to three-fold moles of a base such as triethylamine, diisopropylethylamine, pyridine, lithium hydride, sodium hydride, sodium methoxide, sodium ethoxide, sodium carbonate, potassium carbonate, sodium hydrogen carbonate and the like, and by further adding sodium iodide, potassium iodide and the like. This substitution reaction can be carried out in an inert solvent, for example, methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature in a range of about -10°C to about 180°C for about 1 to about 40 hours. This reaction is preferably carried out under an inert gas (for example, nitrogen, argon, etc.) atmosphere.

### [Process D]

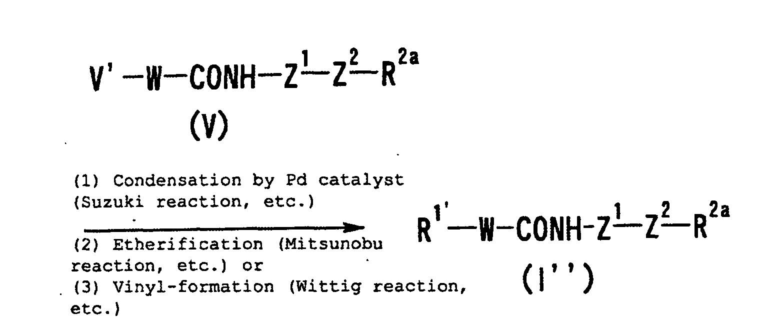

(1) Compound (V) [wherein V' represents a halogen atom (bromine, iodine, etc.) or a sulfonyloxy group (a trifluoromethanesulfonyloxy group, etc.), and the other symbols have the same meanings as described above] can be subjected to, for example, the Suzuki reaction [a cross-condensation reaction of aryl boric acid and, for example, aryl halide or aryloxytrifluoromethanesulfonate, catalyzed by a palladium catalyst; A. Suzuki et al., Synth. Commun., 11, 513 (1981)], to prepare Compound (I") in which R¹' is a 5- or 6-membered aromatic group. The aryl boric acid can be used in an amount of about an equivalent to 1.5-fold moles with respect to 1 mole of Compound (V) to prepare Compound (I").
   Further, Compound (V) can be subjected to, for example, a cross-condensation reaction with an arylacetylene compound in the presence of a palladium catalyst [dichlorobis(triphenylphosphine)palladium, etc.] [K. S. Y. Lau et al., J. Org. Chem., 46, 2280 (1981); J. W. Tilley, S. Zawoisky et al., J. Org. Chem., 53, 386 (1988)] to prepare Compound (I") having an acetylene bond, in which X¹ represents -C≡C-. The arylacetylene compound can be used typically in an amount of about an equivalent to two-fold moles with respect to 1 mole of Compound (V) to prepare Compound (I").
(2) Compound (V) [wherein V' represents a hydroxy group, and the other symbols have the same meanings as described above] can be subjected to, for example, the Mitsunobu reaction [an etherification reaction using, for example, triphenylphosphine and diethyl azodicarboxylate as the condensing agents; O. Mitsunobu et al., Synthesis, 1 (1981)] to prepare Compound (I") having an ether bond. The corresponding alcohol compound or phenol compound can be used in an amount of about an equivalent to three-fold moles with respect to 1 mole of Compound (V) to prepare Compound (I").
   Compound (I") having an ether bond can also be prepared by an etherification reaction of Compound (V) with a reactive compound such as a halide (chloride, bromide, iodide, etc.) compound, a tosylate compound, a mesylate compound, etc. The reactive compound is used typically in an amount of about an equivalent to two-fold moles with respect to 1 mole of Compound (V). This reaction can be facilitated, if necessary, by adding about an equivalent to three-fold moles of a base such as triethylamine, diisopropylethylamine, pyridine, lithium hydride, sodium hydride, sodium hydroxide, potassium hydroxide, sodium methoxide, sodium ethoxide, sodium carbonate, potassium carbonate, sodium hydrogen carbonate, etc., and by further adding sodium iodide, potassium iodide, etc. The reaction may be carried out in an inert solvent such as tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature in a range of about -10°C to 180°C for about 1 to about 40 hours. The reaction is preferably carried out under an inert gas (for example, nitrogen, argon, etc.) atmosphere.
(3) Compound (V), wherein V' represents a carbonyl group which may be substituted, a phosphonium salt or a phosphonic acid ester, and the other symbols have the same meanings as defined above, can be subjected to, for example, the Wittig reaction [A. Maercker, Org. React., 14, 270 (1965)] or the Wittig-Horner-Emmons reaction [J. Boutagy and R. Thomas, Chem. Rev., 74, 87 (1974)] to prepare Compound (I") having a vinyl bond. The corresponding carbonyl compound, a phosphonium salt or a phosphonic acid ester compound is used in an amount of about an equivalent to 1.5-fold moles with respect to 1 mole of Compound (V).

### [Process E]

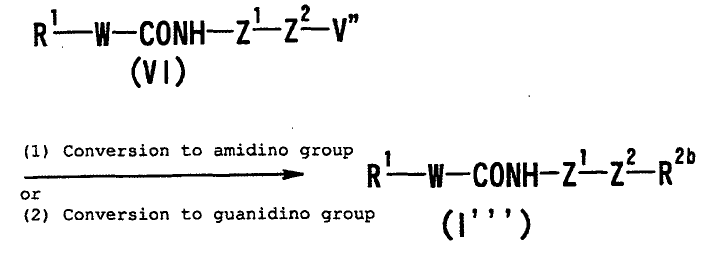

(1) First, Compound (VI), wherein V" represents a cyano group, and the other symbols have the same meanings as defined above, is reacted with a lower alcohol such as methanol, ethanol, propanol, etc. in the presence of an acid such as hydrochloric acid, etc. to prepare an imidate compound. This reaction is typically carried out using an excess of said alcohol at a temperature in a range of about -10°C to 50°C for about 1 hour to about 40 hours. The reaction may be carried out in an inert solvent such as diethyl ether, 1,4-dioxane, toluene, benzene, xylene, dichloromethane, chloroform, 1,2-dichloroethane, etc., or a mixture of the solvents above.
   Subsequently, the resulting imidate compound can be subjected to a substitution reaction with a primary or secondary amine compound to prepare an amidine compound [I''']. The primary or secondary amine compound is used typically in an amount of about 1 to 5 moles with respect to 1 mole of the imidate compound. The reaction can be facilitated, if necessary, by adding about an equivalent to three-fold moles of a demineralizing agent such as triethylamine, pyridine, sodium hydroxide, potassium hydroxide, sodium methoxide, sodium ethoxide, sodium carbonate, potassium carbonate, etc. This substitution reaction may be carried out in an inert solvent, for example, methanol, ethanol, propanol, isopropanol, n-butanol, tetrahydrofuran, diethylether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature in a range of about 0°C to 150°C for about 1 to about 50 hours. The reaction is also preferably carried out under an inert gas (for example, nitrogen, argon, etc.) atmosphere.
(2) Compound (VI), wherein V" is an amino group, and the other symbols have the same meanings as defined above, can be subjected to a substitution reaction with an S-alkyl (for example, methyl, ethyl, etc.)-isothiourea compound to prepare a guanidine Compound (I'''). The S-alkyl-isothiourea compound is typically used in an amount of about an equivalent to two-fold moles with respect to 1 mole of Compound (VI). This reaction can be facilitated, if necessary, by adding about an equivalent to three-fold moles of a demineralizing agent such as triethylamine, pyridine, sodium hydroxide, potassium hydroxide, sodium methoxide, sodium ethoxide, sodium carbonate, potassium carbonate, etc. This substitution reaction may be carried out in an inert solvent, for example, methanol, ethanol, propanol, isopropanol, n-butanol, tetrahydrofuran, diethyl ether, dimethoxyethane, 1,4-dioxane, toluene, benzene, xylene, dichloromethane, chloroform, 1,2-dichloroethane, dimethylformamide (DMF), dimethylsulfoxide (DMSO), pyridine, etc., or a mixture of the solvents above. The reaction is carried out at a temperature in a range of about 0°C to 150°C for about 1 to about 50 hours. This reaction is also preferably carried out under an inert gas (for example, nitrogen, argon, etc.) atmosphere.

The thus-obtained Compound (I) can be isolated and purified by known separation and purification means, for example, concentration, vacuum concentration, solvent extraction, crystallization, recrystallization, resolubilization, chromatography and the like.

Compound (II-1) which is used as the starting material can be prepared by any known methods (for example, the methods described in JP-A No. 8-73476; and JP-A No. 2001-058988, etc.) or a similar manner thereto, for example, the method of the reaction schemes I and II, the methods in Reference Examples described below and similar manners thereto.

### Reaction Scheme I

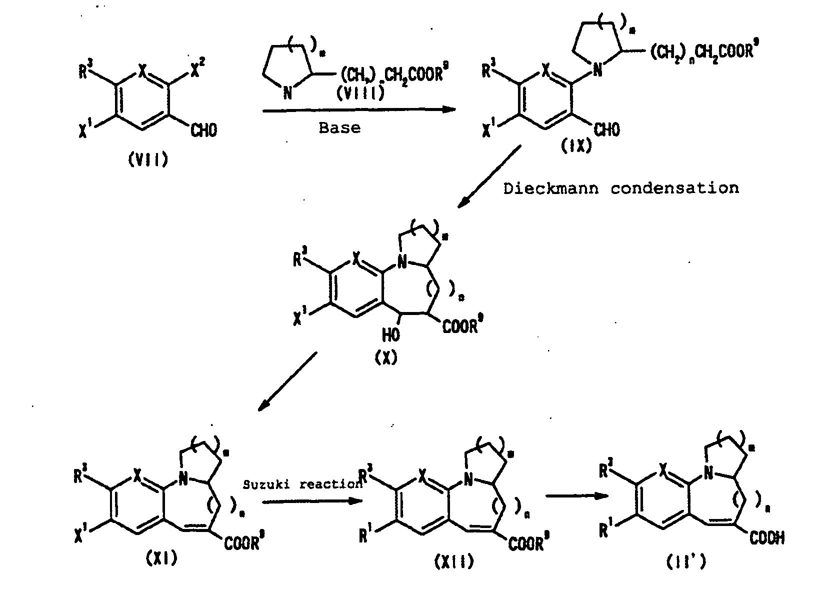

wherein R⁹ represents a C₁₋₄ alkyl group, X¹ and X² each represent a leaving group [a halogen atom (chlorine, bromine, iodine, etc.), methanesulfonyloxy, trifluoromethanesulfonyl, benzenesulfonyloxy, toluenesulfonyloxy, etc.], and the other symbols have the same meanings as defined above.

Compound (VII) can be subjected to a condensation reaction with a cyclic amine compound (VIII) in the presence of a base to prepare Compound (IX). Compound (IX) can be subjected to the Dieckmann condensation reaction (J.P. Schaefer and J.J. Bloomfield, Org. Reactions, 15, 1 (1967)) to prepare Compound (X), which is further dehydrated by a commonly-practiced method to prepare Compound (XI) which is unsaturated carboxylic acid ester. Compound (XI) can be subjected to, for example, a Suzuki reaction to prepare Compound (XII), which is subsequently subjected to an ester hydrolysis reaction to prepare unsaturated carboxylic acid Compound (II').

### Reaction Scheme II

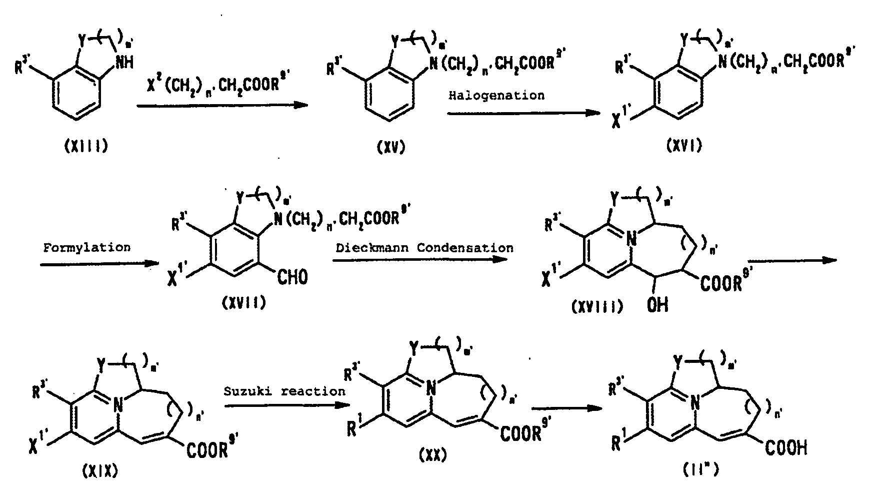

wherein R^{9'} represents a C₁₋₄ alkyl group, X^{1'} represents a halogen atom (chlorine, bromine, iodine, etc.), X^{2'} represents a leaving group [a halogen atom (chlorine, bromine, iodine, etc.), methanesulfonyloxy, trifluoromethanesulfonyl, benzenesulfonyloxy, toluenesulfonyloxy, etc.], and the other symbols have the same meanings as defined above.

Compound (XIII) can be subjected to a condensation reaction with Compound (XIV) in the presence of a base to prepare Compound (XV). Compound (XV) can be subjected to a halogenation reaction to prepare Compound (XVI), which is subsequently subjected to a formylation reaction to prepare Compound (XVII). Compound (XVII) can be subjected to a Dieckmann condensation reaction (J.P. Schaefer and J.J. Bloomfield, Org. Reactions, 15, 1 (1967)) to prepare Compound (XVIII), which is further dehydrated by a conventional method to prepare unsaturated carboxylic acid Compound (XIX). Compound (XIX) can be subjected to, for example, a Suzuki reaction to prepare Compound (XX), which is subsequently subjected to an ester hydrolysis reaction to prepare unsaturated carboxylic acid Compound (II").

Also, Compound (III-1) can be prepared by any known methods (for example, the methods described in JP-A No. 8-73476, etc.) or similar manners thereto, for example, the method of the reaction scheme III, the methods in Reference Examples described below and similar manners thereto. Reaction Scheme III 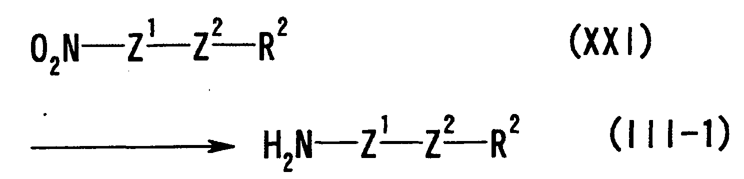 wherein each symbol has the same meaning as defined above.

Reduction of Compound (XXI) can be carried out by the methods known per se in the art. For example, reduction by metal, metal hydride or a metal hydrogen complex compound, reduction by diborane and substituted borane, catalytic hydrogenation, or the like is used. That is, this reaction is carried out by treating Compound (XXI) with a reducing agent. Examples of the reducing agent include metals such as reduced iron, zinc powder, etc.; metal hydrogen complex compounds such as alkali metal borohydrides (for example, sodium borohydride, lithium borohydride, etc.), aluminum lithium hydride, etc.; metal hydrides such as sodium hydride, etc.; organic tin compounds (triphenyltin hydride, etc.); metals and metal salts such as nickel compounds, zinc compounds, etc.; catalytic reducing agents using hydrogen and transition metal catalysts such as palladium, platinum, rhodium, etc.; diborane; and the like. The catalytic reduction using hydrogen and a transition metal such as palladium, platinum, rhodium, etc., and the reduction by a metal such as reduced iron are advantageously employed. This reaction is carried out in an organic solvent which does not interfere with the reaction. The solvent is appropriately selected for use from, for example, benzene, toluene, xylene, chloroform, carbon tetrachloride, dichloromethane, 1,2-dichloroethane, 1,1,2,2-tetrachloroethane, diethyl ether, tetrahydrofuran, dioxane, methanol, ethanol, propanol, isopropanol, 2-methoxyethanol, N,N-dimethylformamide, acetic acid or a mixture of the solvents above, depending on the type of the reducing agent. The reaction temperature is about -20°C to about 150°C, and particularly preferably about 0°C to about 100°C, and the reaction time is about 1 to about 24 hours.

The thus-obtained Compound (III-1) can be isolated and purified by known separation and purification means such as concentration, vacuum concentration, solvent extraction, crystallization, recrystallization, resolubilization, chromatography and the like.

The compound represented by the formula (I) of the present invention or a salt thereof including the above-mentioned Compound (I-1), Compound (I-2), Compound (I'), Compound (I'') and Compound (I''') (hereinafter, in the case where it is briefly referred to as "the compound represented by the formula (I)", it is intended to include a salt thereof and the compound represented by the formula (I) and a salt thereof) can be administered orally or parenterally alone or as a pharmaceutical composition comprising the compound mixed with a pharmaceutically acceptable carrier in the form of a solid preparation such as a tablet, a capsule, a granule, powders, etc., or a liquid preparation such as a syrup, an injectable solution, etc.

Examples of the dosage form for parenteral administration include an injectable solution, an infusion, a suppository, a vaginal suppository, etc., and in particular, the vaginal suppository is useful for prevention of HIV infection.

As the pharmaceutically acceptable carrier, a variety of organic or inorganic carriers that are commonly used as materials for pharmaceutical preparation may be used, and they are added as an excipient, a lubricant, a binder or a disintegrant in solid preparations, and as a solvent, a solubilizing agent, a suspending agent, an isotonic agent, a buffer, a soothing agent or the like in liquid preparations. Other additives for preparation such as an antiseptic agent, an antioxidant, a colorant, a sweetener or the like may also be used, if necessary. Preferred examples of the excipient include lactose, sucrose, D-mannitol, starch, crystalline cellulose, light anhydrous silica and the like. Preferred examples of the lubricant include magnesium stearate, calcium stearate, talc, colloidal silica and the like. Preferred examples of the binder include crystalline cellulose, sucrose, D-mannitol, dextrin, hydroxypropylcellulose, hydroxypropylmethylcellulose, polyvinylpyrrolidone and the like. Preferred examples of the disintegrant include starch, carboxymethylcellulose, calcium carboxymethylcellulose, sodium croscarmellose, sodium carboxymethylstarch and the like. Preferred examples of the solvent include water for injection, alcohol, propylene glycol, macrogol, sesame oil, corn oil and the like. Preferred examples of the solubilizing agent include polyethylene glycol, propylene glycol, D-mannitol, benzyl benzoate, ethanol, trisaminomethane, cholesterol, triethanolamine, sodium carbonate, sodium citrate and the like. Preferred examples of the suspending agent include surfactants such as stearyltriethanolamine, sodium laurylsulfate, laurylaminopropionic acid, lecithin, benzalkonium chloride, benzethonium chloride, glycerin monostearate, etc.; hydrophilic polymers such as polyvinyl alcohol, polyvinyl pyrrolidone, sodium carboxymethylcellulose, methylcellulose, hydroxymethylcellulose, hydroxyethylcellulose, hydroxypropylcellulose, etc.; and the like. Preferred examples of the isotonic agent include sodium chloride, glycerin, D-mannitol and the like. Preferred examples of the buffer include buffer solutions of salts such as phosphate, acetate, carbonate, citrate and the like. Preferred examples of the soothing agent include benzyl alcohol and the like. Preferred examples of the antiseptic agent include para-oxybenzoic acid esters, chlorobutanol, benzyl alcohol, phenethyl alcohol, dehydroacetic acid, sorbic acid and the like. Peferred examples of the antioxidant include sulfite salts, ascorbic acid and the like.

The compound represented by the formula (I) of the present invention or a salt thereof has excellent CCR antagonistic action, in particular, CCR5 and/or CCR2 antagonistic action, and especially, strong CCR5 antagonistic action, and therefore it may be used in prevention and treatment of human HIV infection, for example, AIDS, and also in prevention and treatment of other various diseases. Further, the compound represented by the formula (I) of the present invention or a salt thereof has low toxicity and can be used safely.

For example, the pharmaceutical composition containing the compound represented by the formula (I) of the present invention or a salt thereof can be used as a CCR5 antagonist, for example, a prophylactic and/or therapeutic agent for AIDS and a suppressive agent for disease progression of AIDS. Furthermore, the pharmaceutical composition containing the compound represented by the formula (I) of the present invention or a salt thereof may be used as a prophylactic and/or therapeutic agent for a variety of diseases, such as a prophylactic and/or therapeutic agent for graft versus host disease (GVHD) and/or rejection reaction, a prophylactic and/or therapeutic agent for chronic rheumatoid arthritis, autoimmune diseases, allergic diseases, ischemic brain cell disorder, cardiac infarction, chronic nephritis or arteriosclerosis, or a prophylactic and/or therapeutic agent for other diseases.

Examples of the diseases for which the prophylactic and/or therapeutic agent of the present invention is used, include graft rejection (post-transplantational rejection, post-transplantational polycythemia, hypertension, organ disorder, vascular hypertrophy, graft versus host disease, etc.); arthritic osteopathic diseases such as periostitis/meningitis, etc. (chronic rheumatoid arthritis, osteoarthritis deformans, rheumatoid myelitis, osteoporosis, abnormal growth of cell, fracture, refracture, osteomalacia, osseous Behcet's disease, ankylosing myelitis, articular tissue destruction by gonarthritis deformans and diseases similar thereto, etc.); autoimmune diseases (collagen disease, SLE (systemic lupus erythematosus), pachyderma, polyarteritis, myasthenia gravis, multiple sclerosis, etc.); allergic diseases (allergic nasal catarrh, conjunctivitis, gastrointestinal allergy, pollinosis, anaphylaxis, atopic dermatitis, bronchial asthma, etc.); inflammatory enteropathic diseases (ulcerative colitis, Crohn's disease, gastritis, gastric ulcer, gastric cancer, post-gastrotomic disorder, dyspepsia, esophageal ulcer, pancreatitis, polyp of the colon, cholelithiasis, hemorrhoids, peptic ulcer, situational ileitis, etc.); inflammatory diseases (retinopathy, post-operative and post-traumatic inflammation, remission of puffiness, pharyngitis, cystitis, meningitis, inflammatory ophthalmic diseases, etc.); respiratory diseases (cold syndrome, pneumonia, asthma, pulmonary hypertension, pulmonary thrombi/pulmonary obliteration, pulmonary sarcoidosis, pulmonary tuberculosis, interstitial pneumonia, silicosis, adult tachypnea syndrome, chronic obliterative pulmonary diseases, etc.); infectious diseases (viral infection caused by cytomegalovirus, influenzavirus, herpes virus or the like, rickettsia infection, bacterial infection, sexually transmitted diseases, carinii pneumonia, helicobacter pylori infection, systemic fungal infection, tuberculosis, invasive staphylococcal infection, acute viral encephalitis, acute bacterial meningitis, AIDS encephalopathy, septicemia, sepsis, sepsis gravis, septic shock, endotoxin shock, toxic shock syndromes, etc.); cancers and accompanying cachexia, cancer metastases (bladder cancer, breast cancer, cervical cancer, ovarian cancer, chronic lymphoblastic leukemia, chronic myeloid leukemia, colon cancer, rectal cancer, colic cancer, multiple myeloma, malignant myeloma, prostatic cancer, lung cancer, gastric cancer, Hodgkin's disease, malignant melanoma, malignant lymphoma, etc.); non-Hodgkin's lymphoma; non-small cell lung cancer; malignant melanoma, neurodegenerative diseases (Alzheimer's disease, Parkinson's disease, amyotrophic lateral sclerosis (ALS), Huntington's chorea, diabetic neural disorder, Creutzfeldt-Jakob disease, etc.); mental diseases (depression, epilepsia, alcoholism etc.); schizophrenia; venous dysfunction; central nerve disorder (disorder of intracerebral bleeding, brain infarction, etc. and aftereffect/complication therefrom, cephalic trauma, spinal damage, brain edema, sensory malfunction, sensory dysfunction, autonomic nervous malfunction, autonomic nervous dysfunction, and the like); central damage (cephalic trauma, spinal damage, whiplash injury, etc.); vascular dementia (multiinfarct dementia, Binswanger's disease, etc.); cerebrovascular accident (asymptomatic cerebrovascular accident, transient cerebral ischemic attack, stroke, cerebrovascular dementia, hypertensive encephalopathy, etc.); recurrence and aftereffect of cerebrovascular accident (neural symptom, mental symptom, subjective symptom, operational disorder in daily life, etc.); cerebral vascular dementia; post-cerebrovascular obliteration central hypofunction; disorder or abnormality of cerebral circulation and autoregulation of renal circulation; blood brain barrier disorder; anxiety symptom; acute coronary artery syndromes including unstable angina, etc.; anxious mental state; amnesia; prosopalgia; otolaryngological disease (Meniere's syndrome, tinnitus, gustation disorder, dizziness, dysequilibrium, dysphagia, etc.); migraine; chronic pain; dermatoses (keloid, angioma, psoriasis, etc.); arteriosclerosis obliterans; thromboangiitis obliterans; peripheral obstruction; postischemic reperfusion injury; Raynaud's disease; Buerger's disease; myocarditis; cardiac ischemia; cardiac infarction; progress of cardiac failure after cardiac infarction; cardiomyopathy; cardiac hypertrophy; acute cardiac failure and chronic (including estatic) cardiac failure; angina pectoris; arrhythmia; tachycardia; circadian rhythm disorder of blood pressure; abnormality in characteristic of blood haemocyte components (enhancement in platelet aggregation, abnormality of erythrocyte deformability, enhancement in leucocyte adhesiveness, increase in blood viscosity, polycythemia, vascular peliosis, autoimmune hemolytic anemia, disseminated intravascular coagulation syndrome, multiple myelopathy, etc.); arteriosclerosis including atherosclerosis (aneurysm, coronary arteriosclerosis, cerebro arteriosclerosis, peripheral arteriosclerosis, etc.); vascular reocclusion and restenosis after bypass operation; vascular hyperplasia or occlusion and organ malfunction after intervention (transdermal coronary arterioplasty, stent detention, coronary autoscope, vascular ultrasound therapy, coronary injection thrombolytic therapy, etc.); production and enhancement of vasoactive materials and thrombi inducing materials (endothelin, thromboxan A2, etc.); arterialization (including abnormal vasculogenesis in abnormal capillary vasoganglion formation in the atherosclerotic outer membrane); thrombosis; fat storage disease acceleration; ophthalmic diseases (glaucoma, ocular hypertension disease, etc.); hypertension; hypertensive tinnitus; dialysis hypotension; endothelial cell and organ disorders; endocrinopathy (Addison's disease, Cushing's syndrome, melanocytoma, primary aldosteronism, etc.); nephritis; renal diseases (nephritis, glomerulonephritis, glomerulosclerosis, renal failure, thrombotic microangiopathy, dialysis complications, organ disorders including nephropathy by radiation, diabetic nephropathy, etc.); diabetic diseases (insulin-dependent diabetes, diabetic complications, diabetic retinopathy, diabetic microangiopathy, diabetic neuropathy, etc.); glucose tolerance abnormality; hepatic diseases (hepatitis (including chronic hepatitis), hepatic cirrhosis, etc.); interstitial hepatic diseases; chronic pancreatitis; portal pressure enhancement; obesity; male sterility; gynecologic diseases (climacteric disorder, gestational toxicosis, endometriosis, hysteromyoma, ovarian disease, mammary disease, etc.); edema; chronic fatigue syndromes; prostatomegaly; Behcet's disease; Hodgkin's disease; lacunar infarction; consciousness disorder; psoriasis; diseases due to environmental or occupational factors (disorder caused by radiation, disorders caused by ultraviolet ray/infrared ray/laser ray, altitude sickness, etc.); intermittent claudication; and the like.

The pharmaceutical composition containing the compound represented by the formula (I) or a salt thereof may vary depending on the kind of disease to be treated and may be used in combination with other drugs. Examples of the other drugs include HDL-increasing drugs [a squalene synthase inhibitor, a CETP inhibitor, a LPL activator, etc.]; a prophylactic and/or therapeutic agent for HIV infection [nucleic acid reverse transcriptase inhibitors such as zidovudine, didanosine, zalcitabine, lamivudine, stavudine, abacavir, adefovir, adefovir dipivoxil, fozivudine tidoxil, etc., non-nucleic acid reverse transcriptase inhibitors such as nevirapine, delavirdine, efavirenz, loviride, immunocal, oltipraz, etc., protease inhibitors such as saquinavir, ritonavir, indinavir, nelfinavir, amprenavir, palinavir, lasinavir, lopinavir, etc.]; NMG-CoA reductase inhibitors [cerivastatin, atorvastatin, pravastatin, simvastatin, itavastatin, lovastatin, fluvastatin, (+)-3R,5S-7-[4-(4-fluorophenyl)-6-isopropyl-2-(N-methyl-N-methanesulfonylamino)pyrimidin-5-yl]-3,5-dihydroxy-6(E)-heptenoic acid, etc.]; atopic dermatitis drugs [sodium cromoglycate, etc.]; allergic nasal catarrh drugs [sodium cromoglycate, chlorpheniramine maleate, alimemazine tartrate, clemastine fumarate, homochlorcyclizine hydrochloride, terfenadine, mequitazine, etc.]; imipenem·cilastatin sodium; endotoxin antagonists or antibodies; oxidosqualene-lanosterol cyclase [e.g., decalin derivatives, azadecalin derivatives and indane derivatives]; calcium antagonists (diltiazem, etc.); glycerol; cholinesterase inhibitors (e.g., Aricept (donepezil), etc.); compounds suppressing cholesterol uptake [e.g., sitosterol, neomycin, etc.]; compounds inhibiting cholesterol biosyntheses [e.g., HMG-CoA reductase inhibitors such as lovastatin, simvastatin, pravastatin, etc.];
cyclooxygenase inhibitors [Cox-I, Cox-II inhibitors such as celecoxib, rofecoxib, salicylic acid derivatives such as aspirin and the like, diclofenac, indometacin, loxoprofen, etc.]; signal transduction inhibitors, squalene epoxidase inhibitors [e.g., NB-598 and the analogous compounds, etc.]; steroidal drugs [dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclomethasone propionate, estriol, etc.]; diacerin; nicotinic acid and derivatives and analogues thereof [e.g., acipimox and probucol]; nicergoline, nephrotic syndrome drugs: prednisolone (Predonine), prednisolone sodium succinate (Predonine), methylprednisolone sodium succinate (Solumedrol), betamethasone (Rinderon), dipyridamole (Persantine), dilazep hydrochloride (Comelian), ticlopidine, clopidogrel, antiplatelet drugs and anticoagulants such as FXa inhibitors, etc.; barpital-based anticonvulsants or anaesthetic drugs (phenobarbital, mephobarbital, metharbital, etc.); Parkinson's disease drugs (e.g., L-DOPA, etc.); histamine receptor blockers (cimetidine, famotidine, etc.); hydantoin-based anticonvulsant drugs (phenytoin, mephenytoin, ethotoin, etc.); piroxicam, fibrates [e.g., clofibrate, benzafibrate, gemfibrozil, etc.]; prostaglandins; megestrol acetate; gastric and intraduodenal ulcer drugs: antacids [e.g., histamine H2 antagonists (cimetidine, etc.), proton pump inhibitors (lansoprazole, etc.), and the like]; inflammatory mediator inhibitors; coronary vasodilators: nifedipine, diltiazem, nicoradil, nitrite drugs, etc.; infectious disease drugs: [e.g., antibiotic formulations (cefotiam hydrochloride, cefozopran hydrochloride, ampicillin, etc.), chemotherapeutic agents (sulfa drugs, synthetic antibacterial agents, antiviral agents, etc.), biologic formulations (vaccines, blood preparations including immunoglobulins) etc.] etc.; hepatic disease drugs: glycyrrhizin formulations [e.g., Stronger Minophagen, etc.]; liver hydrolysate; SH compounds [e.g., glutathione, etc.]; special amino acid formulations [e.g., aminoleban, etc.]; phospholipids [e.g., polyene-phosphatidylcholine, etc.]; vitamins [e.g., vitamin B₁, B₂, B₆, B₁₂, C, etc. ]; adrenocortical hormones [e.g., dexamethasone, betamethasone, etc.]; interferons [e.g., interferon α, β, etc.]; hepatic encephalopathy drugs [e.g., lactulose, etc.];
hemostatic agents used in cases of rupture of esophageal and gastric varices [e.g., vasopressin, somatostatin, etc.] etc.; arthritis drugs; muscle relaxants [pridinol, tubocurarine, pancuronium, tolperisone hydrochloride, chlorphenesin carbamate, baclofen, chlormezanone, mephenesin, chlorzoxazone, eperisone, tizanidine, etc.]; vasodilators [oxyfedrine, diltiazem, tolazoline, hexobendine, bamethan, clonidine, methyldopa, guanabenz, etc.]; vasoconstrictors [dopamine, dobutamine, denopamine, etc.]; platelet coagulation inhibitors (ozagrel, etc.); thrombogenesis prophylactic and/or therapeutic drugs: anticoagulant drugs [e.g., heparin sodium, heparin calcium, warfarin calcium (Warfarin), Xa inhibitors]; thrombolytic drugs [e.g., tPA, urokinase]; antiplatelet drugs [e.g., aspirin, sulfinpyrazone (Anturan), dipyridamole (Bersantine), ticlopidine (Panaldine), cilostazol (Pletal), GPIIb/IIIa antagonists (ReoPro)]; antidepressants [imipramine, clomipramine, noxiptiline, fenelzin, amitriptyline hydrochloride, nortriptyline hydrochloride, amoxapine, mianserin hydrochloride, maprotiline hydrochloride, sulpiride, fluvoxamine maleate, trazodone hydrochloride, etc.]; antiepileptic drugs [gabapentin, phenytoin, ethosuximide, acetazolamide, chlordiazepoxide, trimethadione, carbamazepine, phenobarbital, primidone, sultiame, sodium valproate, clonazepam, diazepam, nitrazepam, etc.]; antiallergic drugs [diphenhydramine, chlorpheniramine, tripelennamine, methdilamine, clemizole, diphenylpyraline, methoxyphenamine, sodium cromoglycate, tranilast, repirinast, amlexanox, ibudilast, ketotifen, terfenadine, mequitazine, azlastine, epinastine, ozagrel hydrochloride, pranlukast hydrate, seratrodast, fexofenadine, ebastine, bucillamine, oxatomide, Stronger Neo-Minophagen C, tranexamic acid, ketotifen fumarate, etc.]; anticholinergic drugs (e.g., ipratropium bromide, flutropium bromide, oxitropium bromide, etc.); anti-Parkinson drugs (dopamine, levodopa, etc.); antirheumatic drugs; anti-inflammatory drugs (e.g., aspirin, acetaminophen, diclofenac sodium, ibuprofen, indometacin, loxoprofen sodium, dexamethasone, etc.); anticoagulant and antiplatelet drugs [sodium citrate, activated protein C, tissue factor pathway inhibitors, antithrombin III, dalteparin sodium, argatroban, gabexate, ozagrel sodium, ethyl icosapentate, beraprost sodium, alprostadil, pentoxifylline, tisokinase, streptokinase, hebarin, etc.]; anticoagulant therapeutic drugs [dipyridamole (Bersantine), dilazep hydrochloride (Comelian), ticlopidine, clopidogrel, Xa inhibitors]; antibacterial drugs [(1) sulfa drugs [sulfamethizole, sulfisoxazole, sulfamonomethoxine, sulfamethizole, salazosulfapyridine, sulfadiazine silver, etc.], (2) quinoline-based antibacterial drugs [nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin, etc.], (3) antituberculous drugs [isoniazid, ethambutol (ethambutol hydrochloride), p-aminosalicylic acid (calcium p-aminosalicylate), pyrazinamide, ethionamide, prothionamide, rifampicin, streptomycin sulfate, kanamycin sulfate, cycloserine, etc.], (4) anti-acid fast bacterial drugs [diaphenylsulfone, rifampicilin, etc.], (5) antiviral drugs [idoxuridine, acyclovir, vidarabine, ganciclovir, etc.], (6) anti-HIV drugs [zidovudine, didanosine, zalcitabine, indinavir sulfate ethanolate, ritonavir, etc.], (7) spirocheticide, (8) antibiotics [tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotiam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or salts thereof, griseofulvin, lankacidins [J. Antibiotics, 38, 877-885 (1985)], etc.], cefixime, levofloxacin]; antithrombotic drugs (argatroban, etc.); antiprotozoal drugs [metronidazole, tinidazole, diethylcarbamazine citrate, quinine hydrochloride, quinine sulfate, etc.]; antitumor drugs [6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuproline acetate, buserelin acetate, etc.]; antifungal drugs [ (1) polyethylene-based antibiotics (e.g., amphotericin B, nystatin, trichomycin), (2) griseofulvin, pyrrolnitrin, etc., (3) cytosine metabolism antagonists (e.g., flucytosine), (4) imidazole derivatives (e.g., econazole, clotrimazole, miconazole nitrate, bifonazole and croconazole), (5) triazole derivatives (e.g., fluconazole, itoraconazole, azole compounds [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl-3-(2H,4H)-1,2,4-triazolone], (6) thiocarbamate derivatives [e.g., trinaphthol], (7) echinocandin-based derivatives (e.g., caspofungin, FK-463, V-echinocandin), etc.]; antipsychotic drugs [chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine, etc.];
antiulcer drugs [metoclopramide, histidine hydrochloride, lansoprazole, metoclopramide, pirenzepine, cimetidine, ranitidine, famotidine, urogastron, oxethazaine, proglumide, omeprazole, sucralfate, sulpiride, cetraxate, gefarnate, aldioxa, teprenone, prostaglandins, etc.]; antidiabetic drugs [e.g., pioglitazone, nateglinide, voglibose, acarbose, etc.]; antiobesity drugs (mazindol, etc.); antirheumatic drugs, etc.; antianxiety drugs [diazepam, lorazepam, oxazepam, chlordiazepoxide, medazepam, oxazolam, cloxazolam, clotiazepam, bromazepam, etizolam, fludiazepam, hydroxyzine, etc.]; antiarrhythmic drugs: disopyramide, lidocaine, quinidine sulfate, flecainide acetate, mexiletine hydrochloride, amiodarone hydrochloride, and β blockers, Ca antagonists, etc.; antiasthmatic drugs [isoprenaline hydrochloride, salbutamol sulfate, procaterol hydrochloride, terbutaline sulfate, trimetoxynol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglycate, tranilast, repirinast, amlexanox, ibudilast, ketotifen, terfenadine, mequitazine, azelastine, epinastine, ozagrel hydrochloride, pranlukast hydrate, seratrodast, dexamethasone, prednisolone, hydrocortisone, beclomethasone propionate, fluticasone propionate, beclomethasone propionate, procaterol, etc.]; anti-hypothyroidism drugs [dried thyroid (Thyreoid), levothyroxine sodium (Thyradin S), liothyronine sodium (thyronine, tyronamine)]; nephrotic syndrome drugs [prednisolone (Predonine), prednisolone sodium succinate (Predonine), methylprednisolone sodium succinate (Solumedrol), betamethasone (Rinderon)]; antihypertensive drugs [(1) sympathetic nerve inhibitors [α2 stimulants (e.g., clonidine, guanabenz, guanfacine, methyldopa, etc.), ganglionic blockers (e.g., hexamethonium, trimethaphan, etc.), presynaptic blockers (e.g., ARSA-Oxylone, dimethylaminoreserpinate, rescinnamine, reserpine, syrosingopine, etc.), neuronal blockers (e.g., betanidine, guanethidine, etc.), α1 blockers (e.g., bunazosin, doxazosin, prazosin, terazosin, urapidil, etc.),
β blockers (e.g., propranolol, nadolol, timolol, nipradilol, bunitrolol, indenolol, penbutolol, carteolol, carvedilol, pindolol, acebutolol, atenolol, pisoprolol, metoprolol, labetalol, amosulalol, arotinolol, etc.), and the like], (2) vasodilators [calcium channel antagonists (e.g., manidipine, nicardipine, nilvadipine, nisoldipine, nitrendipine, benidipine, amlodipine, aranidipine, etc.), phthalazine derivatives (e.g., budralazine, cadralazine, ecarazine, hydralazine, todralazine, etc.), and the like], (3) ACE inhibitors [alacepril, captopril, cilazapril, delapril, enalapril, lisinopril, temocapril, trandolapril, quinapril, imidapril, benazepril, perindopril, etc.)], (4) AII antagonists [losartan, candesartan, valsartan, telmisartan, irbesartan, forasartan, etc.], (5) diuretic drugs (e.g., diuretic drugs described above, etc.); antihypertensive drugs: diuretic drugs [e.g., furosemide (Lasix), bumetanide (Lunetoron), azosemide (DIART)], antihypertensive drugs [e.g., ACE inhibitors, (enalapril maleate (RENIVACE), etc.) and Ca antagonists (manidipine, amlodipine, etc.), α or β receptor blockers, etc.], antihyperlipemia drugs [HMG-CoA reductase inhibitors (e.g., fluvastatin, cerivastatin, atorvastatin, etc.), fibrates [e.g., simfibrate, aluminum clofibrate, clinofibrate, fenofibrate, etc.], anion exchange resin (e.g., cholestyramine, etc.), nicotinic acid drugs (e.g., nicomol, niceritrol, tocopherol nicotinate, etc.), polyvalent unsaturated fatty acid derivatives (e.g., ethyl icosapentate, polyene phosphatidylcholine, melinamide, etc.), phytosterols (e.g., gamma-oryzanol, soy sterol, etc.), elastase, sodium dextran sulfate, squalene synthase inhibitors, CETP inhibitors, ethyl 2-chloro-3-[4-(2-methyl-2-phenylpropoxy)phenyl]propionate [Chem. Pharm. Bull., 38, 2792-2796 (1990)], etc.]; osseous disease drugs: calcium formulations [e.g., calcium carbonate, etc.], calcitonin formulations, activated vitamin D₃ formulations [e.g., alfacalcidol (Alfarol, etc.), calcitriol (Rocaltrol), etc.],
sex hormones (e.g., estrogen, estrandiol, etc.), hormone formulations [e.g., conjugated estrogen (Premarin), etc.], ibriflavone formulations [Osten, etc.], vitamin K₂, vitamin K₂ formulations [e.g., menatetrenone (Glakay), etc.], bisphosphonate-based formulations (etidronate, etc.), prostaglandin E2, fluorine compounds (e.g., sodium fluoride, etc.), bone morphogenetic protein (BMP), fibroblast growth factor(FGF), platelet derived growth factor (PDGF), transforming growth factor (TGF-β), insulin-like growth factor-1 and -2 (IGF-1, -2), parathyroid adrenal hormones (PTH), and compounds described in EP-A1-376197, EP-A1-460488, and EP-A1-719782 (e.g., (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide, etc.), and the like, fat-soluble vitamin drugs [(1) vitamin A family: vitamin A₁, vitamin A₂, and retinol palmitate, (2) vitamin D family: vitamin D₁, D₂, D₃, D₄ and D₅, (3) vitamin E family: α-tocopherol, β-tocopherol, γ-tocopherol, δ-tocopherol, dl-α-tocopherol nicotinate, (4) vitamin K family: vitamin K₁, K₂, K₃ and K₄, (5) folic acids (vitamin M, etc.); vitamin derivatives [various vitamin derivatives, e.g., vitamin D₃ derivatives such as 5,6-trans-cholecalciferol, 2,5-hydroxycholecalciferol, 1-α-hydroxycholecalciferol, vitamin D₂ derivatives such as 5,6-trans-ergocalciferol, and the like]; disease-modifying antirheumatic and immunosuppressive drugs [e.g., methotrexate, leflunomide, prograf, sulfasalazine, D-penicillamine, oral gold drugs]; hypertensors [dopamine, dobutamine, denopamine, digitoxin, digoxin, methyldigoxin, lanatoside C, G-strophanthin, etc.]; myocardial protective drugs: heart ATP-K opener, Na-H exchange inhibitors, endothelin antagonists, urotensin antagonist, etc., cardiac failure drugs [cardiac stimulants (e.g., digitoxin, digoxin, methyldigoxin, lanatoside C, proscillaridin, etc.), α, β stimulants (e.g., epinephrine, norepinephrine, isoproterenol, dopamine, docarpamine, dobutamine, denopamine, etc.), phosphodiesterase inhibitors (e.g., amrinone, milrinone, olprinone hydrochloride, etc.),
calcium channel sensitivity enhancer (e.g., pimobentan, etc.), nitrate drugs (e.g., nitroglycerin, isosorbide nitrate, etc.), ACE inhibitors (e.g., the ACE inhibitor described above, etc.), diuretic drugs (e.g., diuretic drugs described above, etc.), calperitide, ubidecarenone, vesnarinone, aminophylline, etc.]; neurotrophic factors; renal failure and nephropathy drugs; biological formulations [e.g., monoclonal antibodies (e.g., anti-TNF-α antibodies, anti-IL-12 antibodies, anti-IL-6 antibodies, anti-ICAM-I antibodies, anti-CD4 antibodies, etc.), soluble receptors (e.g., soluble TNF-α receptors, etc.), protein ligands (IL-I receptor antagonist, etc.)]; bile acid binding resins [e.g., cholestyramine, colestipol, etc.]; biliary tract disease drugs : cholepoietic drugs [e.g., dehydrocholic acid, etc.], cholekinetic drugs [e.g., magnesium sulfate, etc.], and the like; central nervous system agonists: antianxiety drugs, hypnotic and sedative drugs, anesthetic drugs, spasmolytic drugs, autonomic drugs, anti-Parkinson drugs and other psychoneuro drugs, etc.; antitussive and expectorants [ephedrine hydrochloride, noscapine hydrochloride, codeine phosphate, dihydrocodeine phosphate, isoproterenol hydrochloride, ephedrine hydrochloride, methylephedrine hydrochloride, alloclamide, clofedanol, picoperidamine, cloperastine, protokylol, isoproterenol, salbutamol, terbutaline, oxymetebanol, morphine hydrochloride, dextromethorphan hydrobromide, oxycodone hydrochloride, dimemorfan phosphate, tipepidine hibenzate, pentoxyverine citrate, clofedanol hydrochloride, benzonatate, guaifenesin, bromhexine hydrochloride, ambroxol hydrochloride, acetylcysteine, ethylcysteine hydrochloride, carbocisteine, etc.], sedative drugs [chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium, etc.], analgesic and antiphlogistic drugs [e.g., central analgesic drugs (e.g., morphine, codeine, pentazocine, etc.), steroidal drugs (e.g., prednisolone, dexamethasone, betamethasone, etc.), antiphlogistic enzymatic drugs (e.g., bromelain, lysozymes, proctase, etc.)], diabetic drugs [sulfonylurea drugs (e.g., tolbutamide, chlorpropamide, glyclopyramide, acetohexamide, tolazamide, glibenclamide, glibuzole, etc.), biguanide drugs (e.g., metformin hydrochloride, buformin hydrochloride, etc.),
α-glucosidase inhibitors (e.g., voglibose, acarbose, etc.), insulin resistance improvers (e.g., pioglitazone, troglitazone, etc.), insulin, glucagon, diabetic complication drugs (e.g., epalrestat, thioctic acid, etc.), actos, rosiglitazone, kinedak, penfill, humulin, euglucon, glimicron, daonil, novolin, monotard, insulin family, glucobay, dimelin, rastinone, bacilcon, deamelin S, Iszilin acid, etc.]; brain function activating agents (e.g., idebenone, vinpocetine, etc.); urinary and male genital disease drugs [e.g., prostatomegaly drugs (tamsulosin hydrochloride, prazosin hydrochloride, chlormadinone acetate, etc.), prostate cancer drugs (leuprorelin acetate, goserelin acetate, chlormadinone acetate, etc.)], etc; nonsteroidal antiinflammatory drugs [acetaminophen, phenacetin, ethenzamide, sulpyrine, antipyrine, migrenin, aspirin, mefenamic acid, fulfenamic acid, diclofenac sodium, loxoprofen sodium, phenylbutazone, indometacin, ibuprofen, ketoprofen, naproxen, oxaprozin, flurbiprofen, fenbufen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, sodium aurothiomalate, sodium hyaluronate, sodium salicylate, morphine hydrochloride, salicylic acid, atropine, scopolamine, morphine, pethidine, levorphanol, ketoprofen, naproxen, oxymorphone or salts thereof, etc.]; frequent urination and incontinence drugs [flavoxate hydrochloride, etc.]; unstable plaque stabilizers [MMP inhibitors, chymase inhibitors, etc.]; arrhythmic drugs [sodium channel blockers (e.g., quinidine, procainamide, disopyramide, ajmaline, cibenzoline, lidocaine, diphenylhydantoin, mexiletine, propafenone, flecainide, pilsicainide, phenytoin, etc.), β blockers (e.g., propranolol, alprenolol, bufetolol, oxprenolol, atenolol, acebutolol, metoprolol, pisoprolol, pindolol, carteolol, arotinolol, etc.), potassium channel blockers (e.g., amiodarone, etc.), calcium channel blockers (e.g., verapamil, diltiazem, etc.), and the like];
gynecologic disease drugs [e.g., climacteric disorder drugs (conjugated estrogen, estradiol, testosterone enanthate, estradiol valerate, etc.), breast cancer drugs (tamoxifen citrate, etc.), endometriosis and hysteromyoma drugs (leuprorelin acetate, danazol, etc.)], etc.; anesthetic drugs [a. local anaesthetic drugs [cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine], etc.]; b. systemic anesthetic drugs [(1) inhalation anesthetic drugs (e.g., ether, halothane, nitrous oxide, influrane, enflurane), (2) intravenous anesthetic drugs (e.g., ketamine hydrochloride, droperidol, thiopental sodium, thiamylal sodium, pentobarbital), etc.]]; anesthetic antagonists [levallorphan, nalorphine, naloxone, or salts thereof, etc.]; chronic cardiac failure drugs: cardiac stimulants [e.g., cardiac glycoside (digoxin), etc., β receptor stimulants (catecholamine preparations such as denopamine, dobutamine), PDE inhibitors, etc.]; diuretic drugs [e.g., furosemide (Lasix), spironolactone (Aldactone), bumetanide (Lunetoron), azosemide (Diart), etc.]; ACE inhibitors [e.g., enalapril maleate (Renivace), etc.]; Ca antagonists [e.g., amlodipine, manidipine, etc.] and β receptor blockers, etc.; immunomodulators [cyclosporin, tacrolimus, gusperimus, azathioprine, antilymphocyte sera, dried sulfonated immunoglobulins, erythropoietins, growth promoting glycoproteins, interleukins, interferons, etc.]; diuretic drugs [thiazide-based diuretic drugs (benzylhydrochlorothiazide, cyclopenthiazide, ethiazide, hydrochlorothiazide, hydroflumethiazide, methyclothiazide, penfluthiazide, polythiazide, trichlormethiazide, etc.), loop diuretic drugs (chlortalidone, clofenamide, indapamide, mefruside, meticrane, sotrazone, tribamide, quinethazone, metolazone, furosemide, mefruside, etc.), potassium-sparing diuretic drugs (spironolactone, triamterene, etc.)]; erectile dysfunction drugs (Viagra, apomorphine, etc.); and the like.

These drugs may be formulated, separately or simultaneously, by mixing with pharmaceutically acceptable carriers, excipients, binders, diluents or the like, and can be administered either orally or parenterally. When the drugs are formulated separately, the separately prepared formulations may be mixed using a diluent or the like at the time of use and then administered, or each of the separately prepared formulations may be administered, simultaneously or separately with a time interval, to the same subject. Kit products that are to be used for mixing the separately prepared formulations using a diluent or the like at the time of use and administering (for example, an injection kit including ampoules for containing individual powdery drug, and a diluent for mixing and dissolving two or more drugs at the time of use, and the like), kit products that are to be used for administering each of the separately prepared formulations, simultaneously or separately with a time interval, to the same subject (for example, a tablet kit for administering two or more tablets, simultaneously or separately with a time interval, each tablet containing each of the drugs and placed in the same or separate bags, with space for memorandum provided, if necessary, on the bags for indication of the drug administration time, or the like), and the like are also included to the pharmaceutical composition of the present invention.

Dosage of the pharmaceutical composition of the present invention can be appropriately selected by taking into consideration of the subject to be administered, age and body weight of the subject, symptoms, administration time, administration route, dosage form and the like.

The dosage of a particular subject can be determined according to the subject's age, body weight, general health condition, gender, meal, administration time, administration route, excretion rate and the extent of disease condition of the patient at the time of treatment, by taking into consideration of these and other factors.

When the pharmaceutical composition described above is used as a prophylactic and therapeutic agent for AIDS and as a suppressive agent for disease progression of AIDS, the dosage may vary depending on the patient's condition, body weight or the method of administration. However, in the case of oral administration, the daily dosage is in a range of about 5 to 1000 mg, preferably about 10 to 600 mg, more preferably about 10 to 300 mg, and particularly preferably about 15 to 150 mg, as the active ingredient [i.e. as the compound of the formula (I)], for an adult having a body weight of 50 kg, and the composition is administered once, or in 2 or 3 divided doses a day.

When the pharmaceutical composition described above is used as a prophylactic and therapeutic agent for graft versus host disease and/or rejection associated with transplantation of organ such as heart, kidney, liver, bone marrow or the like, administration of the composition starts three days before the transplantation and is continued even after the transplantation. The daily dosage of the pharmaceutical composition of the present invention may vary depending on the patient's condition, body weight or method of administration, but in the case of oral administration, it is about 5 to 1000 mg, preferably about 10 to 600 mg, more preferably about 10 to 300 mg, and particularly preferably about 15 to 150 mg, as the active ingredient [i.e., as the compound represented by the formula (I)], for an adult having a body weight of 50 kg, and the composition is administered once, or in 2 or 3 divided doses a day. In this case, too, the composition may be used in combination with other suppressive agents for graft versus host disease and/or rejection associated with organ transplantation. Specific examples of the suppressive agent for graft versus host disease and/or rejection associated with organ transplantation, which are used in combination with the compound represented by the above formula (I) or a salt thereof, include cyclosporin, tacrolimus, rapamycin, steroids, azathioprine, mycophenolate mofetil, mizoribine, etc. In the case of using these drugs in combination, if one of the drugs interferes with metabolism of other drugs, the dosage of each drug is to be appropriately adjusted, but in general, the dosage for administration of a single drug is employed for each of the drugs.

When the compound represented by the formula (I) described above or a salt thereof is used for diseases other than the suppressive agents for graft versus host disease and/or rejection associated with organ transplantation, the daily dosage thereof may vary depending on the kind of the disease to be treated, the patient's condition, body weight, or method of administration. But, in the case of oral administration, the dosage is about 5 to 1000 mg, preferably about 10 to 600 mg, more preferably about 10 to 300 mg, and particularly preferably about 15 to 150 mg, as the active ingredient [i.e., as the compound represented by the formula (I)], for an adult having a body weight of 50 kg, and the composition is administered once, or in 2 or 3 divided doses a day. When the compound is used in combination with other drugs, the dosage of the other drugs is appropriately selected in a range of, for example, about 1/200 to 1/2 or more and about 2 to 3 times or less of a general dosage. Further, in the case of using the compound in combination with two or more drugs, if one of the drugs interferes with metabolism of the other drugs, the dosage of each drug is to be appropriately adjusted, but in general, the dosage for administration of a single drug is employed for each of the drugs.

Furthermore, the compound represented by the formula (I) or a salt thereof can be also included in or used in combination with blood for transfusion or blood derivatives. Blood for transfusion or blood derivatives are usually produced by mixing blood obtained from a plurality of persons, and in some cases, cells infected by HIV virus may be co-present with HIV-uninfected cells. In such a case, there is fear for infection of the uninfected cells. Thus, when the compound represented by the formula (I) of the present invention is added to blood for transfusion or a blood derivative, it is possible to prevent or control infection and proliferation of the virus. Especially, upon storage of blood derivatives, addition of the compound represented by the formula (I) of the present invention is effective for prevention or control of infection and proliferation of the virus. In addition, when blood for transfusion or a blood derivative contaminated with HIV virus is administered to a person, infection and proliferation of the HIV virus in the body of the person administered with blood for transfusion or a blood derivative can be prevented by the compound represented by the formula (I) that has been added to the blood for transfusion or the blood derivative. For example, in the case of orally administering the compound to an adult (body weight of about 60 kg) for preventing HIV infection upon blood transfusion and use of blood derivatives, the dosage for a single administration is usually in a range of about 0.02 to 50 mg/kg, preferably 0.05 to 30 mg/kg, and more preferably 0.1 to 10 mg/kg, as the CCR antagonist, and the compound is preferably administered in about 1 to about 3 doses a day. As a matter of fact, the range of dosage can be adjusted on the basis of the unit dosage necessary for dividing the daily dosage; however, as described above, the dosage can be determined by taking into consideration of the nature and severity of the disease, the patient's age, the body weight, the general health condition, the gender, the meal, the administration time, the method of administration, the excretion rate and other factors. In this case, the method of administration can be also appropriately selected, and the above-described prophylactic agent for HIV infection of the present invention may be added directly to the blood or blood derivative to be transfused, prior to blood transfusion or use of blood derivative. In such a case, the addition of the compound is preferably carried out immediately before to 24 hours before, preferably immediately before to 12 hours before, and more preferably immediately before to 6 hours before, the transfusion or use of blood derivative.

When the prophylactic agent for HIV infection of the present invention is further administered in addition to the blood or blood derivative to be transfused, at the time of blood transfusion or use of blood derivative, the agent is preferably administered from 1 hour before to simultaneously with transfusion or use of blood derivative, and more preferably, the agent is administered 1 to 3 times per day, continuously for 4 weeks.

Moreover, when the compound represented by the formula (I) or a salt thereof is used in combination with a reverse transcriptase inhibitor and/or a protease inhibitor, the dosage of the reverse transcriptase inhibitor or the protease inhibitoror is properly selected in a range of about 1/200 to 1/2 or more, to about 2 to 3 times or less of the usual dosage.

The usual dosages of the representative reverse transcriptase inhibitors and protease inhibitors are, for example, as follows:
zidovudine: 100 mg
didanosine: 125 to 200 mg
zalcitabine: 0.75 mg
lamivudine: 150 mg
stavudine: 30 to 40 mg
saquinavir: 600 mg
ritonavir: 600 mg
indinavir: 800 mg
nelfinavir: 750 mg

A typical embodiment of combined use of the compound of the formula [I] or a salt thereof, and a reverse transcriptase and/or a protease inhibitor will be described below.
(1) About 10 to 300 mg of the compound of the formula [I] or the salt thereof and about 50 to 200 mg of zidovudine, per an adult of body weight of 50 kg, are administered in combination to the same object. Each medicine may be administered simultaneously or separately in a time interval of less than 12 hours.
(2) About 10 to 300 mg of the compound of the formula [I] or the salt thereof and about 300 to 1200 mg of saquinavir, per an adult of body weight of 50 kg, are administered in combination to the same object. Each medicine may be administered simultaneously or separately in a time interval of less than 12 hours.

The following Examples, Reference Examples, Experimental Example and Formulation Examples further illustrate the present invention in detail but are not to be construed to limit the scope thereof.

### Example 1 (Preparation of Compound 1)

To (S)-4-[[(1-propyl-1H-imidazol-5-yl)methyl]sulfinyl]aniline di-p-toluoyl-D-tartrate monohydrate (1.66 g) was added 1 N hydrochloric acid (9 ml), and the mixture was extracted with ethyl acetate. To the aqueous layer was added an aqueous 25% potassium carbonate solution (9 ml), followed by extraction with ethyl acetate-2-propanol (4 : 1) three times. The organic layer was washed with saturated brine, and dried over magnesium sulfate, which was concentrated under reduced pressure to give (S)-4-[[(1-propyl-1H-imidazol-5-yl)methyl]sulfinyl]aniline as a colorless amorphous material.

To a solution of 8-[4-(2-butoxyethoxy)phenyl]-2,3,3a,4-tetrahydro-1H-pyrrolo[1,2-a][1]benzazepine-5-carboxylic acid (700 mg) in THF (10 ml) were added thionyl chloride (0.18 ml) and DMF (one drop), and the mixture was stirred at room temperature for 1.5 hours. After concentration under reduced pressure, a solution of the residue in THF (30 ml) was added dropwise to a suspension of (S)-4-[[(1-propyl-1H-imidazol-5-yl)methyl]sulfinyl]aniline and triethylamine (1.4 ml) in THF (10 ml) at room temperature. After stirring the resulting mixture at room temperature for 18 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (ethyl acetate) using a basic silica gel, and further recrystallized (ethyl acetate-diisopropyl ether) to give (Ss)-8-[4-(2-butoxyethoxy)phenyl]-N-[[4-[(1-propyl-1H-imidazol-5-yl)methyl]sulfinyl]phenyl]-2,3,3a,4-tetrahydro-1H-pyrrolo[1,2-a][1]benzazepine-5-carboxamide (Compound 1) (488.3 mg) as yellow crystals.

¹H-NMR (200MHz, CDCl₃) δ 0.91 (3H, t, J=7.3 Hz), 0.93 (3H, t, J=7.3 Hz), 1.28-1.27 (2H, m), 1.52-1.78 (4H, m), 1.92-2.09 (4H, m), 2.25-2.45 (1H, m), 2.62-2.81 (1H, m), 3.13-3.22 (1H, m), 3.41-3.54 (2H, m), 3.55 (2H, t, J=6.6 Hz), 3.74-3.83 (2H, m), 3.98-4.18 (4H, m), 6.57 (1H, s), 6.78 (1H, d, J=8.4 Hz), 6.98 (2H, d, J=8.8 Hz), 7.33-7.50 (8H, m), 7.73 (2H, d, J=8.4 Hz), 7.80 (1H, s).

IR (KBr) 3032, 1657, 1605, 1590, 1520, 1497, 1397, 1356, 1310, 1244, 1179, 1117, 1040, 820 cm⁻¹

Elementary analysis C₃₉H₄₆N₄O₄S, Calcd. C, 70.24 ; H, 6.95 ; N, 8.40 : Found. C, 70.05 ; H, 6.84 ; N, 8.23.

### Example 2 (Preparation of Compounds 2 and 3)

(Ss)-8-[4-(2-butoxyethoxy)phenyl]-N-[[4-[(1-propyl-1H-imidazol-5-yl)methyl]sulfinyl]phenyl]-2,3,3a,4-tetrahydro-1H-pyrrolo[1,2-a][1]benzazepine-5-carboxamide (Compound 1) (350 mg) was optically resolved by using CHIRALPAK AD (50 mm ID x 500 mmL) (elution solvent, methanol). The fraction was concentrated into dry solid, and the residue was dissolved in ethanol, which was filtered by a 0.45 µm filter. The filtrate was concentrated to give two diastereomers of Compound 1 [the former fraction: diastereomer 1 (Compound 2) (170 mg, >99.9%de) and the latter fraction: diastereomer 2 (Compound 3) (170 mg, >99.9%de)].

Compound 2: [α]_{D} = -503.3° (c = 0.546%, chloroform solution)

Compound 3: [α]_{D} = +249.1° (c = 0.470%, ethanol solution)

### Example 3 (Preparation of Compound 4)

To (S)-4-[[(1-propyl-1H-imidazol-5-yl)methyl]sulfinyl]aniline di-p-toluoyl-D-tartrate monohydrate (1.0 g) was added 1 N hydrochloric acid (9 ml), and the mixture was extracted with ethyl acetate. To the aqueous layer was added an aqueous 25% potassium carbonate solution (9 ml), followed by extraction with ethyl acetate-2-propanol (4 : 1) three times. The organic layer was washed with saturated brine, and dried over magnesium sulfate, which was concentrated under reduced pressure to give (S)-4-[[(1-propyl-1H-imidazol-5-yl)methyl]sulfinyl]aniline as a colorless amorphous material.

To a solution of 9-[4-(2-butoxyethoxy)phenyl]-1,2,3,4,4a,5-hexahydropyrido[1,2-a][1]benzazepine-6-carboxylic acid (500 mg) in THF (10 ml) were added thionyl chloride (0.126 ml) and DMF (one drop) at room temperature, and the mixture was stirred for 1 hour. After concentration under reduced pressure, a solution of the residue in THF (30 ml) was added dropwise to a suspension of (S)-4-[[(1-propyl-1H-imidazol-5-yl)methyl]sulfinyl]aniline and triethylamine (0.96 ml) in THF (10 ml) at room temperature. After stirring the resulting mixture at room temperature for 16 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (Ss)-9-[4-(2-butoxyethoxy)phenyl]-N-[[4-[(1-propyl-1H-imidazol-5-yl)methyl]sulfinyl]phenyl]-1,2,3,4,4a,5-hexahydropyrido[1,2-a][1]benzazepine-6-carboxamide (Compound 4) (487 mg) as a yellow amorphous material.

[α]_{D} = -129.4° (c = 0.498%, ethanol solution)

¹H-NMR (300MHz, CDCl₃) δ 0.92 (3H, t, J=7.4 Hz), 0.94 (3H, t, J=7.4 Hz), 1.31-1.44 (2H, m), 1.46-1.89 (10H, m), 2.54-2.60 (1H, m), 2.73-2.79 (1H, m), 3.11-3.23 (1H, m), 3.25-3.34 (1H, m), 3.45-3.54 (1H, m), 3.55 (2H, t, J=6.6 Hz), 3.78-3.82 (4H, m), 4.04 (1H, d, J=14.4 Hz), 4.11 (1H, d, J=14.4 Hz), 4.16 (2H, t, J=5.0 Hz), 6.58 (1H, s), 6.99 (2H, d, J=8.7 Hz), 7.12 (1H, d, J=8.4 Hz), 7.36-7.40 (4H, m), 7.47-7.50 (4H, m), 7.77 (2H, d, J=9.0 Hz), 8.04 (1H, s).

IR (KBr) 1667, 1590, 1518, 1491, 1397, 1316, 1246, 1221, 1123, 1046, 824 cm⁻¹

Elementary analysis C₄₀H₄₈N₄O₄S·0.5H₂O, Calcd. C, 69.64 ; H, 7.16 ; N, 8.12 : Found. C, 69.24 ; H, 7.09 ; N, 8.05.

### Example 4 (Preparation of Compound 5)

To (S)-4-[(1-propyl-1H-imidazol-5-yl)methyl)sulfinyl)aniline di-p-toluoyl-D-tartrate monohydrate (2.99 g) was added 1 N hydrochloric acid (20 ml), and the mixture was extracted with ethyl acetate. To the aqueous layer was added an aqueous 25% potassium carbonate solution (20 ml), followed by extraction with ethyl acetate-2-propanol (4 : 1) three times. The organic layer was washed with saturated brine, and dried over magnesium sulfate, which was concentrated under reduced pressure to give (S)-4-[(1-propyl-1H-imidazol-5-yl)methyl]sulfinyl]aniline as a colorless amorphous material. To a solution of 9-[4-(2butoxyethoxy)phenyl]-1,2,3,3a,4,5-hexahydropyrrolo[1,2-a][1]benzazocine-6-carboxylic acid (1.50 g) and DMF (0.1 ml) in THF (30 ml) was added oxalyl chloride (0.33 ml) at 0°C, and the mixture was stirred at room temperature for 2 hours. The reaction mixture was added dropwise to a suspension of (S)-4-[(1-propyl-1H-imidazol-5-yl)methyl]sulfinyl]aniline and pyridine (2.8 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (Ss)-9-[4-(2butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]benzazocine-6-carboxamide (Compound 5) (2.28 g) as a yellow amorphous material.

¹H-NMR (200MHz, CDCl₃) δ 0.89 (3H, t, J=7.4 Hz), 0.93 (3H, t, J=7.4 Hz), 1.32-1.79 (9H, m), 1.91-2.11 (3H, m), 2.64-2.76 (2H, m), 3.08-3.30 (1H, m), 3.42-3.59 (3H, m), 3.73-3.83 (4H, m), 4.01 (1H, d, J=14.2 Hz), 4.08-4.17 (3H, m), 4.37-4.51 (1H, m), 6.55 (1H, s), 6.68 (1H, d, J=8.4 Hz), 6.95 (2H, d, J=8.4 Hz), 7.26-7.46 (7H, m), 760 (1H, s), 7.76 (2H, d, J=8.4 Hz), 8.17 (1H, s).

IR (KBr) 3100, 1661, 1605, 1588, 1518, 1497, 1395, 1358, 1315, 1248, 1182, 1123, 912, 831, 727 cm⁻¹

Elementary analysis C₄₀H₄₈N₄O₄S·0.5H₂O, Calcd. C, 69.64 ; H, 7.16 ; N,8.12 : Found. C, 69.49 ; H, 7.23 ; N, 7.86.

### Example 5 (Preparation of Compounds 6 and 7)

(Ss)-9-[4-(2butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]bensazocine-6-carboxamide (Compound 5) (1.97 g) was optically resolved by using CHIRALCEL OD (50 mmID × 500 mmL) (elution solvent, hexane : ethanol = 7 : 3). The fraction was concentrated into dry solid, and the residue was dissolved in ethanol, which was filtered by a 0.45 µm filter. The filtrate was concentrated to give two diastereomers of Compound 5 [the former fraction: diastereomer 1 (Compound 6) (0.95 g, 99.9%de) and the latter fraction: diastereomer 2 (Compound 7) (0.95 g, 99.2%de)].

Compound 6: [α]_{D} = -169.5° (c = 0.519%, ethanol solution)

Compound 7: [α]_{D} = -91.8° (c = 0.527%, in ethanol solution)

### Example 6 (Preparation of Compound 8)

To (S)-4-[(1-propyl-1H-imidazol-5-yl)methyl)sulfinyl)aniline di-p-toluoyl-D-tartrate monohydrate (0.96 g) was added 1 N hydrochloric acid (5 ml), and the mixture was extracted with ethyl acetate. To the aqueous layer was added an aqueous 25% potassium carbonate solution (5 ml), followed by extraction with ethyl acetate-2-propanol (4 : 1) three times. The organic layer was washed with saturated brine, and dried over magnesium sulfate, which was concentrated under reduced pressure to give (S)-4-[(1-propyl-1H-imidazol-5-yl)methyl]sulfinyl]aniline as a colorless amorphous material.

To a solution of 10-[4-(2-butoxyethoxy)phenyl]-2,3,4,4a,5,6-hexahydro-1H-pyrido[1,2-a][1]benzazocine-7-carboxylic acid (0.50 g) in THF (10 ml) were added thionyl chloride (0.12 ml) and DMF (one drop) at room temperature, and the mixture was stirred for 1.5 hours. After concentration under reduced pressure, a solution of the residue in THF (30 ml) was added dropwise to a suspension of (S)-4-[(1-propyl-1H-imidazol-5-yl)methyl]sulfinyl]aniline and triethylamine (0.96 ml) in THF (30 ml) at room temperature. After stirring the resulting mixture at room temperature for 20 hours, water was added thereto and the mixture was extracted with ethyl acetate. The organic layer was washed with an aqueous 5% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate) to give (Ss)-10-[4-(2-butoxyethoxy)phenyl]-N-[[4-[(1-propyl-1H-imidazol-5-yl)methyl]sulfinyl]phenyl]-2,3,4,4a,5,6-hexahydropyrido[1,2-a][1]benzazocine-6-carboxamide (Compound 8) (483.8 mg, 63%) as a yellow amorphous material.

¹H-NMR (300MHz, CDCl₃) δ 0.91 (3H, t, J=7.8 Hz), 0.93 (3H, t, J=7.2 Hz), 1.31-1.80 (14H, m), 1.95-2.09 (1H, m), 2.25-2.39 (1H, m), 2.63-2.72 (1H, m), 2.90-3.04 (1H, m), 3.55 (2H, t, J=6.6 Hz), 3.61-3.82 (5H, m), 4.02 (1H, d, J=13.8 Hz), 4.08-4.17 (3H, m), 6.55-6.56 (1H, m), 6.97 (2H, d, J=8.7 Hz), 7.07 (1H, d, J=8.4 Hz), 7.34-7.46 (8H, m), 7.76 (2H, d, J=8.4 Hz), 8.03 (1H, s).

IR (KBr) 3031, 1663, 1605, 1590, 1518, 1495, 1316, 1246, 1182, 1117, 1046, 831 cm⁻¹

Elementary analysis C₄₁H₅₀N₄O₄S·0.5H₂O, Calcd. C, 69.95 ; H, 7.30 ; N, 7.96 : Found. C, 70.03 ; H, 7.30 ; N, 7.74.

### Example 7 (Preparation of Compounds 9 and 10)

(Ss)-10-[4-(2-butoxyethoxy)phenyl]-N-[4-[[(1-propyl-1H-imidazol-5-yl)methyl]sulfinyl]phenyl]-2,3,4,4a,5,6-hexahydro-1H-pyrido[1,2-a][1]benzazocine-7-carboxamide (Compound 8) (330 mg) was optically resolved by using CHIRALCEL OD (50 mmID × 500 mmL) (elution solvent, hexane : ethanol = 8 : 2). The fraction was concentrated into dry solid, and the residue was dissolved in ethanol, which was filtered by a 0.45 µm filter. The filtrate was concentrated to give two diastereomers of Compound 8 [the formerfraction: diastereomer 1 (Compound 9) (161 mg, 99.8%de) and the latter fraction: diastereomer 2 (Compound 10) (162 mg, 99.8%de)].

Compound 9: [α]_{D} = -118.5° (c = 0.497%, chloroform solution)

Compound 10: [α]_{D} = -22.9° (c = 0.352%, chloroform solution)

### Example 8 (Preparation of Compound 11)

To (S)-4-[[(1-propyl-1H-imidazol-5-yl)methyl]sulfinyl]aniline di-p-toluoyl-D-tartrate monohydrate (1.65 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine, and dried over magnesium sulfate, which was concentrated under reduced pressure to give (S)-4-[[(1-propyl-1H-imidazol-5-yl)methyl]sulfinyl]aniline as a colorless amorphous material. To a solution of 10-[4-(2-butoxyethoxy)phenyl]-2,3,5,6-tetrahydro-1H-azepino[3,2,1-ij]quinoline-7-carboxylic acid (800 mg) in THF (20 ml) were added oxalyl chloride (0.19 ml) and DMF (0.1 ml) at room temperature, and the mixture was stirred for 1.5 hours. The reaction solution was added dropwise to a suspension of (S)-4-[[(1-propyl-1H-imidazol-5-yl)methyl]sulfinyl]aniline and pyridine (1.54 ml) in THF (20 ml) at 0°C. After stirring the resulting mixture at room temperature for 16 hours, water was added thereto and the mixture was extracted with ethyl acetate. The organic layer was washed with an aqueous 10% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-10-[4-(2-butoxyethoxy)phenyl]-N-[[4-[(1-propyl-1H-imidazol-5-yl)methyl]sulfinyl]phenyl]-2,3,5,6-tetrahydro-1H-azepino[3,2,1-ij]quinoline-7-carboxamide (Compound 11) (988 mg, 78%) as a yellow amorphous material.

[α]_{D} = -139.5° (c = 0.466%, ethanol solution)
¹H-NMR (200MHz, CDCl₃) δ 0.90 (3H, t, J=7.6 Hz), 0.94 (3H, t, J=7.0 Hz), 1.29-1.48 (2H, m), 1.55-1.78 (4H, m), 1.90-2.07 (2H, m), 2.78-2.84 (2H, m), 2.88-2.99 (2H, m), 3.31-3.44 (4H, m), 3.55 (2H, t, J=6.6 Hz), 3.73-3.83 (4H, m), 4.01 (1H, d, J=14.4 Hz), 4.06-4.18 (3H, m), 6.57 (1H, s), 6.97 (2H, d, J=8.8 Hz), 7.18 (1H, d, J=1.8 Hz), 7.33-7.48 (7H, m), 7.73 (2H, d, J=8.8 Hz), 7.89 (1H, s).

### Example 9 (Preparation of Compound 12)

To (S)-4-[[(1-propyl-1H-imidazol-5-yl)methyl]sulfinyl]aniline di-p-toluoyl-D-tartrate monohydrate (1.03 g) was added 1 N hydrochloric acid (8.0 ml), and the mixture was extracted with ethyl acetate. To the aqueous layer was added 25% potassium carbonate (8.0 ml), followed by extraction with ethyl acetate-2-propanol (4 : 1). The organic layer was washed with saturated brine, and dried over magnesium sulfate, which was concentrated under reduced pressure to give (S)-4-[[(1-propyl-1H-imidazol-5-yl)methyl]sulfinyl]aniline. To a solution of 10-[4-(2-butoxyethoxy)phenyl]-1,2,5,6-tetrahydro-4H-azocino[3,2,1-hi]indole-7-carboxylic acid (0.50 g) and DMF (0.1 ml) in THF (10 ml) was added oxalyl chloride (0.1 ml) at 0°C, and the mixture was stirred at 0°C for 3 minutes. The resulting solution was added dropwise to a mixture of (S)-4-[[(1-propyl-1H-imidazol-5-yl)methyl]sulfinyl]aniline and pyridine (0.96 ml) in THF (20 ml) at 0°C. After stirring the resulting mixture at room temperature for 2 hours, water was added thereto and the mixture was extracted with ethyl acetate. The organic layer was washed with an aqueous 5% acetic acid solution, water, an aqueous sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 99) to give (S)-10-[4-(2-butoxyethoxy)phenyl]-N-[4-[[(1-propyl-1H-imidazol-5-yl)methyl]sulfinyl]phenyl]-1,2,5,6-tetrahydro-4H-azocino[3,2,1-hi]indole-7-carboxamide (Compound 12) (357 mg) as a yellow amorphous material.

[α]_{D} = -142.1° (c = 0.521%, ethanol solution)
¹H-NMR (200MHz, CDCl₃) δ 0.80-0.96 (6H, m), 1.19-1.85 (8H, m), 2.72-2.85 (2H, m), 2.96-3.10 (2H, m), 3.43-3.61 (6H, m), 3.75-3.82 (4H, m), 3.98-4.17 (4H, m), 6.57 (1H, s), 6.94 (2H, d, J=8.8 Hz), 7.03 (1H, d, J=1.8 Hz), 7.21 (1H, s), 7.ss-7.48 (6H, m), 7.76 (2H, d, J=8.4 Hz), 8.00 (1H, s).

### Example 10 (Preparation of Compound 13)

To (S)-4-[(1-propyl-1H-imidazol-5-yl)methyl)sulfinyl)aniline di-p-toluoyl-D-tartrate monohydrate (1.59 g) was added 1 N hydrochloric acid (10 ml), and the mixture was extracted with ethyl acetate. To the aqueous layer was added an aqueous 25% potassium carbonate solution (10 ml), followed by extraction with ethyl acetate-2-propanol (4 : 1) three times. The organic layer was washed with saturated brine, and dried over magnesium sulfate, which was concentrated under reduced pressure to give (S)-4-[(1-propyl-1H-imidazol-5-yl)methyl]sulfinyl]aniline as a colorless amorphous material. To a solution of 11-[4-(2-butoxyethoxy)phenyl]-2,3,6,7-tetrahydro-1H,5H-azocino[3,2,1-ij]quinoline-8-carboxylic acid (0.80 g) and DMF (0.1 ml) in THF (20 ml) was added oxalyl chloride (0.177 ml) at 0°C, and the mixture was stirred at room temperature for 1.5 hours. The reaction mixture was added dropwise to a suspension of (S)-4-[(1-propyl-1H-imidazol-5-yl)methyl]sulfinyl]aniline and pyridine (1.49 ml) in THF (20 ml) at 0°C. After stirring the resulting mixture at room temperature for 20 hours, water was added thereto and the mixture was extracted with ethyl acetate. The organic layer was washed with an aqueous 10% acetic acid solution, an aqueous saturated sodium hydrogen carbonate solution and saturated brine, and dried over magnesium sulfate. After concentration under reduced pressure, the residue was separated and purified by column chromatography (basic silica gel, ethyl acetate → ethanol : ethyl acetate 1 : 49) to give (S)-11-[4-(2-butoxyethoxy)phenyl]-N-[4-[[(1-propyl-1H-imidazol-5-yl)methyl]sulfinyl]phenyl]-2,3,6,7-tetrahydro-1H,5H-azocino[3,2,1-ij]quinoline-8-carboxamide (Compound 13) (485.5 mg, 39%) as a yellow amorphous material.

[α]_{D} = -130.2° (c = 0.472%, ethanol solution)
¹H-NMR (300MHz, CDCl₃) δ 0.90 (3H, t, J=7.2 Hz), 0.93 (3H, t, J=7.2 Hz), 1.32-1.45 (2H, m), 1.51-1.78 (6H, m), 1.95-2.04 (2H, m), 2.51-2.62 (2H, m), 2.80 (2H, t, J=5.9), 3.22 (2H, t, J=6.0 Hz), 3.47-3.57 (4H, m), 3.74-3.81 (4H, m), 4.02 (1H, d, J=13.8 Hz), 4.07-4.15 (3H, m), 6.56 (1H, s), 6.94 (2H, d, J=8.9 Hz), 7.14-7.18 (2H, m), 7.34 (2H, d, J=8.9 Hz), 7.41 (2H, d, J=8.9 Hz), 7.45 (1H, s), 7.55 (1H, s), 7.75 (2H, d, J=8.9 Hz), 8.00 (1H, s).

IR (KBr) 3196, 1659, 1605, 1588, 1516, 1497, 1316, 1244, 1177, 1121, 1044, 829, 743 cm⁻¹
Elementary analysis C₄₀H₄₈N₄O₄S·0.25H₂O, Calcd. C, 70.09 ; H, 7.13 ; N, 8.17 : Found. C, 70.05 ; H, 7.28 ; N, 7.93.

### Example 11 (Preparation of Compound 14)

To (S)-4-[[(1-propyl-1H-imidazol-5-yl)methyl]sulfinyl]aniline di-p-toluoyl-D-tartrate monohydrate (0.99 g) was added 1 N hydrochloric acid (8.0 ml), and the mixture was extracted with ethyl acetate. To the aqueous layer was added 25% potassium carbonate (8.0 ml), and the mixture was extracted with ethyl acetate-2-propanol (4 : 1). The organic layer was washed with saturated brine, and dried over magnesium sulfate, which was concentrated under reduced pressure to give (S)-4-[[(1-propyl-1H-imidazol-5-yl)methyl]sulfinyl]aniline. To a solution of 11-[4-(2-butoxyethoxy)phenyl]-2,3,6,7-tetrahydro-5H-[1,4]oxazino[2,3,4-kl][1]benzazocine-8-carboxylic acid (0.50 g) and DMF (0.05 ml) in THF (10 ml) was added oxalyl chloride (0.099 ml) at 0°C, and the mixture was stirred at 0°C for 5 minutes. The resulting solution was added dropwise to a mixture of (S)-4-[[(1-propyl-1H-imidazol-5-yl)methyl]sulfinyl]aniline and pyridine (0.92 ml) in THF (20 ml) at 0°C. After stirring the resulting mixture at 0°C for 1 hour, an aqueous sodium hydrogen carbonate solution was added thereto and the mixture was extracted with ethyl acetate. The organic layer was washed with water and saturated brine, and dried over magnesium sulfate. After concentration under reduced pressure, the residue was separated and purified by column chromatography (basic silica gel, ethyl acetate → ethanol : ethyl acetate 1 : 49) to give (S)-11-[4-(2-butoxyethoxy)phenyl]-N-[4-[[(1-propyl-1H-imidazol-5-yl)methyl]sulfinyl]phenyl]-2,3,6,7-tetrahydro-5H-[1,4]oxazino[2,3,4-kl][1]benzazocine-8-carboxamide (Compound 14) (133 mg) as a yellow amorphous material.

[α]_{D} = -130.3° (c = 0.498%, ethanol solution)
¹H-NMR (200MHz, CDCl₃) δ 0.90 (3H, t, J=7.3 Hz), 0.93 (3H, t, J=7.2 Hz), 1.29-1.46 (2H, m), 1.51-1.82 (6H, m), 2.61-2.73 (2H, m), 3.40 (2H, t, J=4.4 Hz), 3.52-3.61 (4H, m), 3.75-3.82 (4H, m), 4.01 (1H, d, J=14.0 Hz), 4.08-4.17 (3H, m), 4.24 (2H, t, J=4.4 Hz), 6.55 (1H, s), 6.94 (1H, d, J=2.4 Hz), 6.95 (2H, d, J=8.8 Hz), 7.03 (1H, d, J=2.4 Hz), 7.32-7.46 (5H, m), 7.56 (1H, s), 7.76 (2H, d, J=8.8 Hz), 8.10 (1H, s).

### Example 12 (Preparation of Compound 15)

(S)-4-(((1-propylimidazol-5-yl)methyl)sulfinyl)aniline di-p-toluoyl-D-tartrate monohydrate (1.2 g) was dissolved in ethyl acetate (10 ml) and 1 N hydrochloric acid (6.09 ml), followed by separation. To the aqueous layer was added an aqueous 25% potassium carbonate solution (6.09 ml), followed by extraction with 2-propanol-ethyl acetate (1 : 4) twice. The organic layers were combined, washed with saturated brine and dried over magnesium sulfate, and then the solvent was distilled off under reduced pressure. To the resulting residue was added tetrahydrofuran, and then the solvent was again distilled off under reduced pressure to give (S)-4-(((1-propylimidazol-5-yl)methyl)sulfinyl)aniline. To a solution of 3-[4-(2-butoxyethoxy)phenyl]-7,8,8a,9,10,11-hexahydropyrido[3,2-g]pyrrolo[1,2-a]azocine-6-carboxylic acid (600 mg) in dichloromethane (15 ml) was added a drop of DMF, and then oxalyl chloride (0.156 ml) was added thereto at 0°C. The mixture was returned to room temperature and stirred for 30 minutes under a nitrogen atmosphere. The resulting solution was then added dropwise to a solution of (S)-4-(((1-propylimidazol-5-yl)methyl)sulfinyl)aniline and pyridine (2.9 ml) in tetrahydrofuran (20 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. After distilling off the solvent under reduced pressure, the residue was separated and purified by basic silica gel column chromatography (ethyl acetate → methanol : ethyl acetate = 3 : 100) to give (Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide (759 mg) (Compound 15) as a yellow amorphous material.

¹H-NMR (300MHz, CDCl₃) δ 0.88-0.96 (6H, m), 1.32-1.50 (3H, m), 1.55-1.80 (6H, m), 1.90-2.05 (3H, m), 2.70-2.80 (2H, m), 3.53-3.82 (8H, m), 4.01 (1H, d, J=14.1 Hz), 4.10-4.17 (3H, m), 4.35-4.45 (1H, m), 6.53 (1H, s), 6.98 (2H, d, J=9.0 Hz), 7.33-7.50 (7H, m), 7.75 (2H, d, J=8.7 Hz), 8.14 (1H, s), 8.35 (1H, d, J=2.4 Hz).

Elementary analysis C₃₉H₄₇N₅O₄S·0.5H₂O, Calcd. C, 67.80 ; H, 7.00 ; N, 10.13 ; Found. C, 67.52 ; H, 6.99 ; N, 9.89.

[α]_{D} = -132.9° (C = 0.269%, ethanol solution)

### Example 13 (Preparation of Compounds 16 and 17)

(Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide (590 mg) was resolved by using CHIRAKCEL OD (50 mmID x 500 mmL) (hexane : ethanol = 7 : 3) to give two diastereomers [the former fraction: (8aR,Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide (Compound 16) (294 mg, >99.9%de) and the latter fraction: (8aS,Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide (Compound 17) (291 mg, 99.8%de)].

Compound 16: [α]_{D} = -84.5° (C = 0.255% ethanol solution)

Compound 17: [α]_{D} = -193.0° (C = 0.291% ethanol solution)

### Example 14 (Preparation of Compound 16)

(S)-4-[[(1-propyl-1H-imidazol-5-yl)methyl]sulfinyl]aniline di-p-toluoyl-D-tartrate monohydrate (398 mg) was dissolved in ethyl acetate (5 ml) and 1 N hydrochloric acid (2.98 ml), followed by separation. To the aqueous layer was added an aqueous 25% potassium carbonate solution (2.98 ml), followed by extraction with 2-propanol-ethyl acetate (1 : 4) twice. The organic layers were combined, washed with saturated brine, dried over magnesium sulfate, and the solvent was distilled off under reduced pressure. To the resulting residue was added toluene, and the solvent was again distilled off under reduced pressure to give (S)-4-[[(1-propyl-1H-imidazol-5-yl)methyl]sulfinyl]aniline. To a solution of (8aR)-3-[4-(2-butoxyethoxy)phenyl]-7,8,8a,9,10,11-hexahydropyrido[3,2-g]pyrrolo[1,2-a]azocine-6-carboxylic acid (200 mg) in dichloromethane (10 ml) was added a drop of DMF, and then oxalyl chloride (0.052 ml) was added thereto at 0°C. The mixture was returned to room temperature and stirred for 30 minutes under a nitrogen atmosphere. Then, the resulting solution was added dropwise to a solution of (S)-4-[[(1-propyl-1H-imidazol-5-yl)methyl]sulfinyl]aniline and pyridine (0.96 ml) in tetrahydrofuran (10 ml) at 0°C under a nitrogen atmosphere. The mixture was returned to room temperature and stirred overnight. Then, water was added thereto and the mixture was extracted with ethyl acetate. The organic layer was washed with water twice and saturated brine once, and then dried over magnesium sulfate. After distilling off the solvent under reduced pressure, the residue was separated and purified by basic silica gel column chromatography (hexane : ethyl acetate = 1 : 3 → ethyl acetate) to give (8aR,Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide (246 mg) (Compound 16) as a yellow amorphous material.

### Reference Example 1

To a solution of 1-(tert-butoxycarbonyl)proline (16.6 g), 1-hydroxybenzotriazol monohydrate (17.7 g) and N,O-dimethylhydroxyamine hydrochloride (9.02 g) in DMF (100 ml) were added 1-ethyl-3-(3'-dimethylaminopropyl)carbodiimide hydrochloride (22.2 g) and triethylamine (21 ml) at room temperature, and the mixture was stirred for 20 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 1) to give tert-butyl 2-[[methoxy(methyl)amino]carbonyl]pyrrolidine-1-carboxylate (14.82 g) as a colorless oily material. To a solution of tert-butyl 2-[[methoxy(methyl)amino]carbonyl]pyrrolidine-1-carboxylate (14.82 g) in toluene (200 ml) was added dropwise diisobutylaluminum hydride (in 1.0 M toluene, 100 ml) at 0°C. After stirring the resulting mixture at 0°C for 1 hour, to the reaction system was added sodium sulfate decahydrate (48.3 g). After stirring the resulting mixture at room temperature for 20 hours, magnesium sulfate was added thereto, and the insolubles were removed by filtration. After concentration under reduced pressure, the residue was separated and purified by column chromatography (ethyl acetate : hexane 1 : 4) to give 7.23 g of colorless tert-butyl 2-formylpyrrolidine-1-carboxylate. To a suspension of sodium hydride (60%, 1.45 g) in toluene (60 ml) was added dropwise ethyl diethylphosphonoacetate (7.2 ml) at 0°C. The mixture was stirred at 0°C for 30 minutes, and then a solution of tert-butyl 2-formylpyrrolidine-1-carboxylate (7.23 g) in toluene (20 ml) was added dropwise thereto. The mixture was heated under reflux for 2 hours, water was added thereto, and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 9) to give ethyl (2E)-3-[1-(tert-butoxycarbonyl)pyrrolidin-2-yl]-2-propenoate (8.09 g). To a solution of ethyl (2E)-3-[1-(tert-butoxycarbonyl)pyrrolidin-2-yl]-2-propenoate (8.09 g) in ethanol (200 ml) was added a 1 N aqueous sodium hydroxide solution (45 ml) at room temperature. The mixture was stirred at room temperature for 6 hours, and then the reaction mixture was extracted with diethyl ether. To the aqueous layer was added 1 N hydrochloric acid (50 ml), and the mixture was extracted with ethyl acetate. The organic layer was washed with saturated brine, and dried over magnesium sulfate, which was concentrated under reduced pressure to give (2E)-3-[1-(tert-butoxycarbonyl)pyrrolidin-2-yl]-2-propenoic acid (5.31 g) as a colorless oily material.

¹H-NMR (200MHz, CDCl₃) δ 1.43 (9H, s), 1.68-2.24 (4H, m), 3.31-3.51 (2H, m), 4.30-4.59 (1H, m), 5.83 (1H, d, J=15.0 Hz), 6.84-7.01 (1H, m).

IR (neat) 1698, 1395, 1368, 1165 cm⁻¹

### Reference Example 2

A mixture of ethyl (2E)-3-[1-(tert-butoxycarbonyl)pyrrolidin-2-yl]-2-propenoate (5.31 g) and 10% Pd-C (0.50 g) in ethanol was stirred under a hydrogen atmosphere for 24 hours. After removing the catalyst by filtration, the resulting residue was concentrated under reduced pressure to give 3-[1-(tert-butoxycarbonyl)pyrrolidin-2-yl]-2-propionic acid (5.32 g, 98%) as a colorless oily material. To a solution of 3-[1-(tert-butoxycarbonyl)pyrrolidin-2-yl]propionic acid (5.32 g) in ethanol (50 ml) was added concentrated hydrochloric acid (50 ml), and the mixture was stirred at room temperature for 20 hours. After concentration under reduced pressure, to the residue were added water (25 ml) and concentrated hydrochloric acid (50 ml), and the mixture was stirred at 120°C for 20 hours. After concentration under reduced pressure, 2-propanol was added thereto and the mixture was concentrated. To the residue were added 2-propanol and diisopropyl ether. The precipitated crystals were collected by filtration. The crystals were washed with diisopropyl ether to give 3-(pyrrolidin-2-yl)propionic acid hydrochloride (1.91 g) as colorless crystals.
¹H-NMR (200MHz, DMSO-d₆) δ 1.43-1.67 (2H, m), 1.66-2.14 (4H, m), 2.37 (2H, t, J=7.5 Hz), 3.00-3.21 (2H, m), 3.29-3.50 (1H, m), 8.62-8.88 (1H, m), 9.24-9.28 (1H, m). IR (KBr) 3113, 1732, 1705, 1599, 1420, 1206, 1173, 878 cm⁻¹

### Reference Example 3

A mixture of 5-bromo-2-fluorobenzaldehyde (2.15 g), 3-(pyrrolidin-2-yl)propionic acid hydrochloride (1.91 g) and sodium carbonate (3.4 g) in DMSO (40 ml) and water (20 ml) was stirred at 135°C for 17 hours. After cooling to room temperature, 1 N hydrochloric acid was added thereto until the pH reached 4, and the resulting mixture was extracted with ethyl acetate. The organic layer was washed with saturated brine, and dried over magnesium sulfate. After concentration under reduced pressure, to a solution of the residue in DMF (30 ml) were added potassium carbonate (2.1 g) and iodomethane (0.75 ml), and the mixture was stirred at room temperature for 3 hours. To the reaction system was added water, and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 9 → 1 : 4) to give methyl 3-[1-(4-bromo-2-formylphenyl)pyrrolidin-2-yl]propionate (2.74 g) as a yellow oily material.

¹H-NMR (200MHz, CDCl₃) δ 1.56-1.82 (3H, m), 1.85-2.40 (5H, m), 3.02-3.11 (1H, m), 3.66 (3H, s), 3.69-3.97 (2H, m), 6.94 (1H, d, J=9.2 Hz), 7.47 (1H, dd, J=9.2, 2.6 Hz), 7.82 (1H, d, J=2.6 Hz), 10.02 (1H, s).

IR (neat) 1736, 1676, 1590, 1481, 1400, 1173, 1107 cm⁻¹

### Reference Example 4

To a solution of methyl 3-[1-(4-bromo-2-formylphenyl)pyrrolidin-2-yl]propionate (2.24 g) in dimethyl carbonate (45 ml) was added sodium methoxide (28% solution of 28% in methanol, 1.9 g) at room temperature, and the mixture was stirred for 2.5 hours. To the reaction system was added 1 N hydrochloric acid (10 ml), and the mixture was extracted with ethyl acetate. The organic layer was washed with saturated brine, and dried over magnesium sulfate. After concentration under reduced pressure, the precipitated crystals were collected by filtration. The crystals were washed with diisopropyl ether to give methyl 8-bromo-2,3,3a,4-tetrahydro-1H-pyrrolo[1,2-a][1]benzazepine-5-carboxylate (1.83 g) as yellow crystals.

m.p. 136-139°C

¹H-NMR (200MHz, CDCl₃) δ 1.86-2.05 (3H, m), 2.16-2.36 (1H, m), 2.52-2.68 (1H, m), 3.06 (1H, dd, J=17.6, 1.4 Hz), 3.28-2.45 (3H, m), 3.79 (3H, s), 6.54 (1H, d, J=9.2 Hz), 7.23 (1H, dd, J=9.2, 2.6 Hz), 7.37 (1H, d, J=2.6 Hz), 7.54 (1H, d, J=2.6 Hz).

IR (KBr) 1701, 1626, 1586, 1543, 1495, 1478, 1433, 1356, 1285, 1271, 1246, 12219, 1179, 1157, 1127, 1082, 878, 802 cm⁻¹

Elementary analysis C₁₅H₁₆NO₂Br, Calcd. C, 55.92 ; H, 5.01 ; N, 4.35 : Found. C, 55.85 ; H, 4.86 ; N, 4.16.

### Reference Example 5

Under an argon atmosphere, a mixture of methyl 8-bromo-2,3,3a,4-tetrahydro-1H-pyrrolo[1,2-a][1]benzazepine-5-carboxylate (1.0 g), 4-(2-butoxyethoxy)phenylboric acid (0.96 g) and potassium carbonate (1.11 g) in toluene (30 ml), ethanol (3 ml) and water (3 ml) was stirred at room temperature for 1 hour. Tetrakis(triphenylphosphine)-palladium (0.18 g) was added to the reaction system, and the mixture was heated under reflux for 5 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 9) to give methyl 8-bromo-2,3,3a,4-tetrahydro-1H-pyrrolo[1,2-a][1]benzazepine-5-carboxylate (1.00 g) as yellow crystals.

m.p. 102-103°C
¹H-NMR (300MHz, CDCl₃) δ 0.93 (3H, t, J=7.2 Hz), 1.32-1.46 (2H, m), 1.50-1.66 (2H, m), 1.89-2.04 (3H, m), 2.22-2.38 (1H, m), 2.59-2.69 (1H, m), 3.05-3.11 (1H, m), 3.37-3.51 (3H, m), 3.55 (2H, t, J=6.6 Hz), 3.79-3.82 (5H, m), 4.16 (2H, t, J=5.0 Hz), 6.74 (1H, d, J=8.4 Hz), 6.97 (2H, d, J=9.0 Hz), 7.41 (1H, dd, J=8.4, 2.1 Hz), 7.45-7.48 (3H, m), 7.74 (1H, d, J=2.7 Hz).

IR (KBr) 1705, 1607, 1499, 1453, 1356, 1264, 1240, 1186, 1128, 1182, 1069, 926, 837, 804 cm⁻¹
Elementary analysis C₂₇H₃₃NO₄, Calcd. C, 74.45 ; H, 7.64 ; N, 3.22 : Found. C, 74.48 ; H, 7.80 ; N, 3.09.

### Reference Example 6

To a mixed solution of methyl 8-[4-(2-butoxyethoxy)phenyl]-2,3,3a,4-tetrahydro-1H-pyrrolo[1,2-a][1]benzazepine-5-carboxylate (0.90 g) in ethanol (30 ml) and THF (10 ml) was added a 1 N sodium hydroxide acetic acid solution (4.0 ml) at room temperature. The mixture was stirred at 60°C for 20 hours and cooled to 0°C. 1 N Hydrochloric acid (4.0 ml) was added thereto and the mixture was extracted with ethyl acetate. The organic layer was washed with saturated brine, and dried over magnesium sulfate. After concentration under reduced pressure, the precipitated crystals were collected by filtration. The crystals were washed with hexane to give 8-[4-(2-butoxyethoxy)phenyl]-2,3,3a,4-tetrahydro-1H-pyrrolo[1,2-a][1]benzazepine-5-carboxylic acid (794 mg) as yellow crystals.

m.p. 186-187°C
¹H-NMR (200MHz, CDCl₃) δ 0.93 (3H, t, J=7.2 Hz), 1.29-1.76 (4H, m), 1.86-2.09 (3H, m), 2.22-2.42 (1H, m), 2.52-2.75 (1H, m), 3.03-3.15 (1H, m), 3.38-3.54 (3H, m), 3.56 (2H, t, J=6.6 Hz), 3.80 (2H, t, J=4.9 Hz), 4.16 (2H, t, J=4.9 Hz), 6.75 (1H, d, J=8.8 Hz), 6.98 (2H, d, J=8.8 Hz), 7.14-7.49 (4H, m), 7.85 (1H, d, J=2.2 Hz).

IR (KBr) 1669, 1607, 1497, 1426, 1356, 1250, 1184, 1132, 1065, 924, 837, 806 cm⁻¹
Elementary analysis C₂₆H₃₁NO₄, Calcd. C, 74.08 ; H, 7.41 ; N, 3.32 : Found. C, 73.94 ; H, 7.37 ; N, 3.17.

### Reference Example 7

To a solution of DL-pipecolic acid (10.0 g, 77.4 millimole) in a 1 N aqueous sodium hydroxide solution (150 ml, 150 millimole) and THF (200 ml) was added dropwise di-tert-butyl dicarbonate at room temperature. After stirring the resulting mixture at room temperature for 20 hours, 1 N hydrochloric acid (150 ml) was added thereto at 0°C and the mixture was extracted with ethyl acetate. The organic layer was washed with saturated brine, and dried over magnesium sulfate. After concentration under reduced pressure, the precipitated crystals were collected by filtration. The crystals were washed with hexane to give 1-(tert-butoxycarbonyl)piperidine-2-carboxylic acid (15.4 g) as colorless crystals.

¹H-NMR (200MHz, CDCl₃) δ 1.16-1.54 (12H, m), 1.59-1.80 (2H, m), 2.15-2.30 (1H, m), 2.79-3.08 (1H, m), 3.85-4.08 (1H, m), 4.72-4.99 (1H, m).

### Reference Example 8

To a solution of 1-(tert-butoxycarbonyl)piperidine-2-carboxylic acid (15.4 g), 1-hydroxy-1H-benzotriazol monohydrate (15.44 g) and N,O-dimethylhydroxyamine hydrochloride (9.83 g) in DMF (150 ml) were added 1-ethyl-3-(3-dimethylaminopropyl)carbodiimide hydrochloride (19.32 g) and triethylamine (28 ml) at room temperature, and the mixture was stirred at room temperature for 4 days. To the reaction system was added water and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 3) to give tert-butyl 2-[[methoxy(methyl)amino]carbonyl]piperidine-1-carboxylate (15.76 g) as colorless crystals.

¹H-NMR (300MHz, CDCl₃) δ 1.31-1.76 (14H, m), 1.93-2.04 (1H, m), 3.19 (3H, s), 3.30-3.54 (1H, m), 3.77 (3H, br s), 3.84-4.05 (1H, m), 4.82-5.11 (1H, m).

IR (KBr) 1684, 1667, 1456, 1385, 1368, 1279, 1258, 1181, 1167, 1127, 1094, 1046, 872 cm⁻¹

### Reference Example 9

To a solution of tert-butyl 2-[[methoxy(methyl)amino]carbonyl]piperidine-1-carboxylate (15.76 g) in THF (150 ml) was added dropwise a solution of diisobutylaluminum hydride in toluene (1.5 M, 57 ml) at -78°C. After stirring the resulting mixture at -78°C for 0.5 hour, the mixture was warmed to 0°C and further stirred for 0.5 hour. To the reaction system was added sodium sulfate decahydrate and the mixture was stirred at room temperature for 8 hours. Magnesium sulfate was added thereto, and the precipitates were removed by filtration. After concentration under reduced pressure, the residue was separated and purified by column chromatography (ethyl acetate : hexane 1 : 9) to give tert-butyl 2-formylpiperidine-1-carboxylate (9.25 g) as a colorless oily material.

¹H-NMR (200MHz, CDCl₃) δ 1.13-1.78 (14H, m), 2.09-2.23 (1H, m), 2.79-3.02 (1H, m), 3.81-4.06 (1H, m), 4.45-4.68 (1H, m), 9.59 (1H, s).

IR (neat) 1734, 1694, 1404, 1366, 1277, 1252, 1165 cm⁻¹

### Reference Example 10

Under a nitrogen atmosphere, to a suspension of sodium hydride (60%, 2.02 g) in toluene (50 ml) was added dropwise ethyl diethylphosphonoacetate (10.0 ml) at 0°C. After stirring the resulting mixture at 0°C for 30 minutes, a solution of tert-butyl 2-formylpiperidine-1-carboxylate (9.25 g) in toluene (50 ml) was added dropwise thereto. After heating under reflux for 3 hours, water was added thereto and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9) to give ethyl (2E)-3-[1-(tert-butoxycarbonyl)piperidin-2-yl]-2-propenoate (10.36 g). To a solution of ethyl (2E)-3-[1-(tert-butoxycarbonyl)piperidin-2-yl]-2-propenoate (10.36 g) in ethanol (100 ml) was added a 1 N aqueous sodium hydroxide solution (50 ml) at room temperature, and the mixture was stirred for 3 days. After extraction with diethyl ether, 1 N hydrochloric acid (50 ml) was added to the aqueous layer, and the mixture was extracted with ethyl acetate. The organic layer was washed with saturated brine, and dried over magnesium sulfate, which was concentrated under reduced pressure to give (2E)-3-[1-(tert-butoxycarbonyl)piperidin-2-yl]-2-propenoic acid (9.08 g) as a colorless oily material.

¹H-NMR (200MHz, CDCl₃) δ 1.28-1.76 (16H, m), 2.03-2.12 (1H, m), 2.28-2.36 (2H, m), 2.67-2.83 (1H, m), 3.91-4.05 (1H, m), 4.20-4.35 (1H, m).

IR (neat) 1736, 1711, 1690, 1424, 1366, 1275, 1161 cm⁻¹

### Reference Example 11

Under a hydrogen atmosphere, a mixture of (2E)-3-[1-(tert-butoxycarbonyl)piperidin-2-yl]-2-propenoic acid (9.08 g) and 10% Pd-C (0.5 g) in ethanol (200 ml) was stirred at room temperature for 22 hours. Pd-C was removed by filtration and the filtrate was concentrated under reduced pressure to give 3-[1-(tert-butoxycarbonyl)piperidin-2-yl]propionic acid (8.98 g) as a colorless oily material.

¹H-NMR (200MHz, CDCl₃) δ 1.28-1.76 (16H, m), 2.03-2.12 (1H, m), 2.28-2.36 (2H, m), 2.67-2.83 (1H, m), 3.91-4.05 (1H, m), 4.20-4.35 (1H, m).

### Reference Example 12

To 3-[1-(tert-butoxycarbonyl)piperidin-2-yl]propionic acid (8.98 g) was added 6 N hydrochloric acid (45 ml) at room temperature. The resulting mixture was stirred at room temperature for 1 hour, and concentrated under reduced pressure. To the residue was added 2-propanol and the mixture was further concentrated. The precipitated crystals were collected by filtration. The crystals were washed with diisopropyl ether to give 3-(piperidin-2-yl)propionic acid hydrochloride (5.72 g) as colorless crystals.

¹H-NMR (200MHz, DMSO-d₆) δ 1.22-1.98 (8H, m), 2.35-2.43 (2H, m), 2.65-3.25 (3H, m), 8.59-9.15 (2H, m).

IR (KBr) 1720, 1590, 1445, 1433, 1310, 1292, 1235 cm⁻¹

### Reference Example 13

A mixture of 5-bromo-2-fluorobenzaldehyde (5.24 g), 3-(piperidin-2-yl)propionic acid hydrochloride (5.0 g) and sodium carbonate (8.20 g) in DMSO (100 ml) and water (50 ml) was heated under reflux for 18 hours. After cooling to 0°C, 1 N hydrochloric acid was added thereto until the pH reached 4 and extracted with ethyl acetate. The organic layer was washed with saturated brine, and dried over magnesium sulfate. After concentration under reduced pressure, to a solution of the residue in DMF (50 ml) were added potassium carbonate (5.3 g) and iodomethane (1.2 ml), and the mixture was stirred at room temperature for 3 hours. To the reaction system was added water, and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9) to give a yellow oily material (1.3 g). To a solution of the resulting oily material (1.3 g) in dimethyl carbonate (10 ml) was added sodium methoxide (28% solution in methanol, 1.09 g) at room temperature, and the mixture was stirred at room temperature for 16 hours. To the reaction system was added 1 N hydrochloric acid (6 ml), and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 9) to give a yellow oily material (861 mg). Under an argon atmosphere, a mixture of the yellow oily material (861 mg), 4-(2-butoxyethoxy)phenylboric acid (0.73 g) and potassium carbonate (0.71 g) in toluene (25 ml), ethanol (2.5 ml) and water (2.5 ml) was stirred at room temperature for 1 hour. Tetrakis(triphenylphosphine)palladium (0.15 g) was added to the reaction system, and the mixture was heated under reflux for 5 hours. The resulting mixture was cooled to room temperature and then extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 15) to give a yellow oily material (940.8 mg). To a solution of the yellow oily material (940.8 mg) in THF (5 ml) and ethanol (10 ml) was added a 1 N aqueous sodium hydroxide solution (5.0 ml) at room temperature, and the mixture was stirred at 60°C for 20 hours. After cooling to 0°C, 1 N hydrochloric acid (5 ml) was added thereto and the mixture was extracted with ethyl acetate. The organic layer was washed with saturated brine, and dried over magnesium sulfate. After concentration under reduced pressure, the precipitated crystals were collected by filtration. The crystals were washed with diisopropyl ether-ethyl acetate to give 9-bromo-1,2,3,4,4a,5-hexahydropyrido[1,2-a][1]benzazepine-6-carboxylic acid (543 mg) as yellow crystals.

m.p. 150-152°C
¹H-NMR (300MHz, CDCl₃) δ 0.93 (3H, t, J=7.2 Hz), 1.32-1.45 (2H, m), 1.37-1.85 (8H, m), 2.60 (1H, dd, J=15.1, 3.9 Hz), 2.72 (1H, dd, J=15.1, 5.3 Hz), 3.23-3.27 (2H, m), 3.39-3.47 (1H, m), 3.55 (2H, t, J=6.8 Hz), 3.81 (2H, t, J=5.0 Hz), 4.16 (2H, t, J=5.0 Hz), 6.98 (2H, d, J=9.3 Hz), 7.08 (1H, d, J=8.4 Hz), 7.40 (1H, d, J=2.1 Hz), 7.45-7.49 (3H, m), 7.87 (1H, s).

IR (KBr) 1671, 1607, 1516, 1487, 1426, 1277, 1248, 1127, 820 cm⁻¹
Elementary analysis C₂₇H₃₃NO₄, Calcd. C, 74.45 ; H, 7.64 ; N, 3.22 : Found. C, 74.26 ; H, 7.81 ; N, 3.01.

### Reference Example 14

A solution of [2-(1,3-dioxolan-2-yl)ethyl]triphenylphosphonium bromide (7.61 g) and potassium tert-butoxide (1.92 g) in THF (50 ml) was stirred at room temperature for 1.5 hours. A solution of benzyl 2-formylpyrrolidine-1-carboxylate (1.0 g) in THF (20 ml) was added dropwise thereto, and the mixture was stirred at room temperature for 1 hour and further stirred at 50°C for 2 hours. To the reaction system was added water and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 3 → 1 : 2) to give benzyl 2-[3-(1,3-dioxolan-2-yl)-1-propenyl]pyrrolidine-1-carboxylate (1.59 g) as a colorless oily material. A mixture of benzyl 2-[3-(1,3-dioxolan-2-yl)-1-propenyl]pyrrolidine-1-carboxylate (1.59 g) and 10% Pd-C (0.15 g) in ethanol was stirred at room temperature for 14 hours under a hydrogen atmosphere. Pd-C was removed by filtration and the filtrate was concentrated under reduced pressure. To a solution of the residue in THF (20 ml) was added a 1 N aqueous sodium hydroxide solution (15 ml), and further added dropwise benzyl chlorocarbonate (0.92 ml) at 0°C. After stirring at room temperature for 2 hours, the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 3 → 1 : 2) to give benzyl 2-[3-(1,3-dioxolan-2-yl)propyl]pyrrolidine-1-carboxylate (1.28 g) as a colorless oily material.

¹H-NMR (200MHz, CDCl₃) δ 1.29-1.51 (4H, m), 1.56-1.99 (6H, m), 3.32-3.49 (2H, m), 3.79-4.02 (5H, m), 4.74-4.88 (1H, m), 5.03-5.22 (2H, m), 7.30-7.41 (5H, m).

IR (neat) 1699, 1412, 1358, 1142, 1107, 912, 743 cm⁻¹

### Reference Example 15

To a solution of benzyl 2-[3-(1,3-dioxolan-2-yl)propyl]pyrrolidine-1-carboxylate (0.5 g) in THF (5 ml) was added 2 N hydrochloric acid (12 ml) at room temperature. After stirring at room temperature for 3.5 hours, the resulting mixture was cooled to 0°C, a 1 N aqueous sodium hydroxide solution (24 ml) was added thereto, and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 2) to give colorless benzyl 2-(4-oxobutyl)pyrrolidine-1-carboxylate. To a solution of benzyl 2-(4-oxobutyl)pyrrolidine-1-carboxylate (388 mg) and 1-methyl-1-cyclohexene (2.2 ml) in tert-butanol (12 ml) was added dropwise an aqueous solution (10 ml) of sodium chlorite (1.77 g) and sodium dihydrogen phosphate dihydrate (1.96 g) at room temperature. After stirring at room temperature for 1 hour, the resulting mixture was acidified with 1 N hydrochloric acid and extracted with ethyl acetate. The organic layer was washed with an aqueous sodium thiosulfate solution and saturated brine, and dried over magnesium sulfate. After concentration under reduced pressure, a mixture of the residue and 10% Pd-C (0.07 g) in ethanol (20 ml) was stirred at room temperature for 2 hours under a hydrogen atmosphere. Pd-C was removed by filtration and the filtrate was concentrated under reduced pressure to give 4-(pyrrolidin-2-yl)butyric acid (222 mg) as colorless crystals.

¹H-NMR (200MHz, CDCl₃) δ 1.41-2.39 (10H, m), 3.15-3.48 (3H, m).

IR (KBr) 1559, 1399 cm⁻¹

### Reference Example 16

A mixture of 5-bromo-2-fluorobenzaldehyde (0.28 g), 4-(pyrrolidin-2-yl)butyric acid (222 mg) and potassium carbonate (0.44 g) in DMSO (10 ml) and water (2.5 ml) was stirred at 130°C for 12 hours. After cooling to 0°C, the resulting mixture was acidified with 1 N hydrochloric acid and extracted with ethyl acetate. The organic layer was washed with saturated brine, and dried over magnesium sulfate. After concentration under reduced pressure, to a solution of the residue in DMF (10 ml) were added potassium carbonate (0.39 g) and iodomethane (0.13 g), and the mixture was stirred at room temperature for 1 hour. To the reaction system was added water and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 4 → 1 : 3) to give methyl 4-[1-(4-bromo-2-formylphenyl)pyrrolidin-2-yl]butyrate (286.2 mg) as a yellow oily material.

¹H-NMR (200MHz, CDCl₃) δ 1.27-1.44 (1H, m), 1.54-1.88 (5H, m), 1.91-2.01 (1H, m), 2.18-2.38 (3H, m), 3.02-3.14 (1H, m), 3.65 (3H, s), 3.68-3.89 (2H, m), 6.79 (1H, d, J=9.2 Hz), 7.44 (1H, dd, J=9.2, 2.6 Hz), 7.82 (1H, d, J=2.6 Hz), 10.01 (1H, s).

IR (neat) 1705, 1607, 1487, 1273, 1244, 1225, 1132, 1117, 1067, 826 cm⁻¹

### Reference Example 17

To a solution of methyl 4-[1-(4-bromo-2-formylphenyl)pyrrolidin-2-yl]butyrate (3.62 g) in dimethyl carbonate (30 ml) was added sodium methoxide (28% solution in methanol, 3.0 g) at room temperature, and the mixture was stirred at 50°C for 4 hours. After cooling to 0°C, the mixture was neutralized with 1 N hydrochloric acid and extracted with ethyl acetate. The organic layer was washed with saturated brine, and dried over magnesium sulfate. After concentration under reduced pressure, the residue was separated and purified by column chromatography (ethyl acetate : hexane 1 : 9) to give methyl 9-bromo-1,2,3,3a,4,5-hexahydropyrrolo[1,2-a][1]benzazocine-6-carboxylate (2.49 g) as a yellow amorphous material.

¹H-NMR (200MHz, CDCl₃) δ 1.08-1.29 (1H, m), 1.32-1.54 (1H, m), 1.61-1.72 (1H, m), 1.81-2.08 (3H, m), 2.39-2.54 (1H, m), 2.69-2.83 (1H, m), 3.02-3.16 (1H, m), 3.29-3.43 (1H, m), 3.78 (3H, s), 4.23-4.34 (1H, m), 6.44 (1H, d, J=8.8 Hz), 7.18-7.26 (2H, m), 7.69 (1H, s).

### Reference Example 18

Under an argon atmosphere, a mixture of methyl 9-bromo-1,2,3,3a,4,5-hexahydropyrrolo[1,2-a][1]benzazocine-6-carboxylate (2.49 g), 4-(2-butoxyethoxy)phenylboric acid (2.29 g) and potassium carbonate (2.05 g) in toluene (70 ml), ethanol (7 ml) and water (7 ml) was stirred at room temperature for 1 hour. Tetrakis(triphenylphosphine)palladium (0.26 g) was added to the reaction system, and the mixture was heated under reflux for 6 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 9) to give methyl 9-[4-(2butoxyethoxy)phenyl]-1,2,3,3a,4,5-hexahydropyrrolo[1,2-a][1]benzazocine-6-carboxylate (2.00 g) as yellow crystals.

¹H-NMR (200MHz, CDCl₃) δ 0.93 (3H, t, J=7.1 Hz), 1.14-1.73 (7H, m), 1.81-2.11 (3H, m), 2.55 (1H, td, J=13.0, 4.4 Hz), 2.71-2.84 (1H, m), 3.12-3.26 (1H, m), 3.39-3.58 (3H, m), 3.76-3.82 (5H, m), 4.15 (2H, t, J=4.9 Hz), 4.28-4.42 (1H, m), 6.65 (1H, d, J=8.8 Hz), 6.95 (2H, d, J=8.8 Hz), 7.30 (1H, d, J=2.6 Hz), 7.37-7.47 (3H, m), 7.87 (1H, s).

### Reference Example 19

To a mixed solution of methyl 9-[4-(2butoxyethoxy)phenyl]-1,2,3,3a,4,5-hexahydropyrrolo[1,2-a][1]benzazocine-6-carboxylate (2.00 g) in ethanol (20 ml) and THF (10 ml) was added a 1 N aqueous sodium hydroxide solution (10 ml) at room temperature. After stirring at 60°C for 14 hours, the resulting mixture was cooled to 0°C, 1 N hydrochloric acid (10 ml) was added thereto, and the mixture was extracted with ethyl acetate. The organic layer was washed with saturated brine, and dried over magnesium sulfate. After concentration under reduced pressure, the precipitated crystals were collected by filtration. The crystals were washed with hexane to give 9-[4-(2butoxyethoxy)phenyl]-1,2,3,3a,4,5-hexahydropyrrolo[1,2-a][1]benzazocine-6-carboxylic acid (1.76 g) as yellow crystals.

¹H-NMR (200MHz, CDCl₃) δ 0.93 (3H, t, J=7.5 Hz), 1.18-1.76 (7H, m), 1.92-2.18 (3H, m), 2.45-2.65 (1H, m), 2.71-2.86 (1H, m), 3.08-3.29 (1H, m), 3.39-3.59 (3H, m), 3.80 (2H, t, J=4.8 Hz), 4.15 (2H, t, J=4.8 Hz), 4.28-4.44 (1H, m), 6.67 (1H, d, J=9.2 Hz), 6.96 (2H, d, J=8.4 Hz), 7.31 (1H, d, J=2.2 Hz), 7.39-7.46 (3H, m), 8.00 (1H, s).

### Reference Example 20

To a solution of 2-piperidineethanol (10.0 g) in THF (100 ml) was added dropwise di-tert-butyl dicarbonate (16.9 g) at room temperature. After stirring the resulting mixture at room temperature for 24 hours, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 3) to give tert-butyl 2-(2-hydroxyethyl)piperidine-1-carboxylate (17.92 g) as a colorless oily material.

¹H-NMR (200MHz, CDCl₃) δ 1.22-1.79 (15H, m), 1.85-2.04 (1H, m), 2.60-2.76 (1H, m), 3.23-3.47 (1H, m), 3.52-4.04 (3H, m), 4.35-4.52 (1H, m).

IR (neat) 3443, 1689, 1667, 1418, 1366, 1275, 1165, 1142, 1053 cm⁻¹

### Reference Example 21

To a solution of tert-butyl 2-(2-hydroxyethyl)piperidine-1-carboxylate (17.4 g, 75.9 millimole) and triethylamine (21.2 ml, 152.0 millimole) in THF (400 ml) was added dropwise methanesulfonyl chloride (8.8 ml, 113.7 millimole) at 0°C. After stirring the resulting mixture at 0°C for 30 minutes, to the reaction system was added an aqueous sodium hydrogen carbonate solution and the mixture was extracted with ethyl acetate. The organic layer was washed with water and saturated brine, and dried over magnesium sulfate, which was concentrated under reduced pressure to give tert-butyl 2-(2-methanesulfonyloxyethyl)piperidine-1-carboxylate. To a suspension of sodium hydride (60%, 5.76 g, 144.0 millimole) in THF (150 ml) was added dropwise diethyl malonate (23.06 g, 144.0 millimole) at 0°C. After stirring the resulting mixture at 0°C for 30 minutes, previously synthesized tert-butyl 2-(2-methanesulfonyloxyethyl)piperidine-1-carboxylate in THF (50 ml) was added dropwise thereto. After stirring the resulting mixture at 55°C for 24 hours, 1 N hydrochloric acid was added at 0°C thereto until the pH reached 4. The mixture was extracted with ethyl acetate, washed with saturated brine, dried over magnesium sulfate, and then concentrated under reduced pressure. The residue was separated and purified by column chromatography (ethyl acetate : hexane 1 : 19 → 1 : 9 → 1 : 4) to give diethyl [3-[1-(tert-butoxycarbonyl)piperidin-2-yl]propyl]malonate (27.54 g). To the obtained diethyl [3-[1-(tert-butoxycarbonyl)piperidin-2-yl]propyl]malonate was added 6 N hydrochloric acid (150 ml), and the mixture was heated under reflux for 20 hours. After concentration under reduced pressure, 2-propanol was added thereto and the resulting mixture was further concentrated. To the precipitated crystals were added 2-propanol and diisopropyl ether, and the crystals were collected by filtration. The crystals were washed with diisopropyl ether to give 4-(piperidin-2-yl)butyric acid hydrochloride (7.83 g) as colorless crystals.

¹H-NMR (200MHz, DMSO-d₆) δ 1.18-1.91 (10H, m), 2.19-2.30 (2H, m), 2.65-3.07 (2H, m), 3.12-3.25 (1H, m), 8.41-8.89 (2H, m).

IR (neat) 1686, 1609, 1518, 1480, 1456, 1250, 1181, 1128, 1057, 1013, 828 cm⁻¹

### Reference Example 22

A mixture of 5-bromo-2-fluorobenzaldehyde (4.8 g), 4-(piperidin-2-yl)butyric acid hydrochloride (7.0 g) and sodium carbonate (8.6 g) in DMSO (100 ml) and water (50 ml) was stirred at 115°C for 36 hours. After cooling to 0°C, the mixture was acidified with 6 N hydrochloric acid (pH 3 to 4) and extracted with ethyl acetate. The organic layer was washed with saturated brine, and dried over magnesium sulfate. After concentration under reduced pressure, the residue was separated and purified by column chromatography (ethyl acetate : hexane 1 : 3 → 1 : 2) to give 4-[1-(4-bromo-2-formylphenyl)piperidin-2-yl]butyric acid (3.19 g). To a mixture of 4-[1-(4-bromo-2-formylphenyl)piperidin-2-yl]butyric acid (3.19 g) and potassium carbonate (2.5 g) in DMF (50 ml) was added iodomethane (0.56 ml) at room temperature. After stirring the resulting mixture at room temperature for 2 hours, water was added thereto and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9) to give methyl 4-[1-(4-bromo-2-formylphenyl)piperidin-2-yl]butyrate (1.51 g) To a solution of methyl 4-[1-(4-bromo-2-formylphenyl)piperidin-2-yl]butyrate (1.51 g) in dimethyl carbonate (20 ml) was added sodium methoxide (28% solution in methanol, 1.2 m) at room temperature. After stirring at 60°C for 13 hours, the mixture was cooled to 0°C, 1 N hydrochloric acid was added thereto until the pH reached 3 to 4, and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 19) to give methyl 10-bromo-2,3,4,4a-5,6-hexahydro-1H-pyrido[1,2-a]benzazocine-7-carboxylate (837 mg). Under an argon atmosphere, a mixture of methyl 10-bromo-2,3,4,4a-5,6-hexahydro-1H-pyrido[1,2-a]benzazocine-7-carboxylate (837 mg), 4-(2-butoxyethoxy)phenylboric acid (0.77 g) and potassium carbonate (0.66 g) in toluene (30 ml), ethanol (3 ml) and water (3 ml) was stirred at room temperature for 1 hour. Tetrakis(triphenylphosphine)palladium (0.15 g) was added to the reaction system, and the mixture was heated under reflux for 5 hours. After cooling to room temperature, water was added thereto and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 15 → 1 : 9) to give methyl 10-[4-(2-butoxyethoxy)phenyl]-2,3,4,4a-5,6-hexahydro-1H-pyrido[1,2-a]benzazocine-7-carboxylate. To a solution of methyl 10-[4-(2-butoxyethoxy)phenyl]-2,3,4,4a-5,6-hexahydro-1H-pyrido[1,2-a]benzazocine-7-carboxylate (931 mg) in THF (5 ml) and ethanol (10 ml) was added a 1 N aqueous sodium hydroxide solution (5.0 ml, 5.0 millimole) at room temperature, and the mixture was stirred at 60°C for 20 hours. 1 N Hydrochloric acid (5.0 ml) was added thereto at room temperature and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 4 → 1 : 1 → 2 : 1) and further recrystallized (ethyl acetate-hexane) to give 10-[4-(2-butoxyethoxy)phenyl]-2,3,4,4a-5,6-hexahydro-1H-pyrido[1,2-a]benzazocine-7-carboxylic acid (558 mg) as yellow crystals.

m.p. 168-169°C
¹H-NMR (300MHz, CDCl₃) δ 0.93 (3H, t, J=7.2 Hz), 1.00-1.17 (1H, m), 1.31-1.79 (11H, m), 1.96-2.18 (2H, m), 2.71-2.82 (1H, m), 2.87-3.01 (1H, m), 3.55 (2H, t, J=6.6 Hz), 3.70-3.85 (3H, m), 4.15 (2H, t, J=5.0 Hz), 6.96 (2H, d, J=8.7 Hz), 7.02 (1H, d, J=8.4 Hz), 7.36 (1H, dd, J=2.4 Hz), 7.40-7.46 (3H, m), 7.90 (1H, s).

IR (KBr) 1672, 1607, 1495, 1240, 1181, 1159, 1125, 820 cm⁻¹

Elementary analysis C₂₈H₃₅NO₄, Calcd. C, 74.80 ; H, 7.85 ; N, 3.12 : Found. C, 74.74 ; H, 7.81 ; N, 3.02.

### Reference Example 23

A mixture of 1,2,3,4-tetrahydroquinoline (7.5 g), ethyl 4-bromobutyrate (9.7 ml) and potassium carbonate (15.56 g) in DMF (200 ml) was stirred at 90°C for 2 days. To the reaction system were further added ethyl 4-bromobutyrate (4.0 ml) and potassium carbonate (7.78 g), and the mixture was stirred at 100°C for 5 days. To the reaction system was added water and the resulting mixture was extracted with ethyl acetate. The organic layer was washed with saturated brine, and dried over magnesium sulfate. After concentration under reduced pressure, the residue was separated and purified by column chromatography (ethyl acetate : hexane 1 : 19) to give ethyl 4-(3,4-dihydroquinolin-1(2H)-yl)butyrate (11.17 g) as a yellow oily material.

¹H-NMR (200MHz, CDCl₃) δ 1.26 (3H, t, J=7.1 Hz), 1.84-2.00 (4H, m), 2.36 (2H, t, J=7.4 Hz), 2.74 (2H, t, J=6.4 Hz), 3.24-3.32 (4H, m), 4.14 (2H, q, J=7.1 Hz), 6.51-6.61 (2H, m), 6.92-6.95 (1H, m), 7.00-7.07 (1H, m).

### Reference Example 24

To a solution of ethyl 4-(3,4-dihydroquinolin-1(2H)-yl)butyrate (11.17 g) in dichloromethane (500 ml) was added tetrabutylammonium tribromide (21.78 g) at 0°C. After stirring the resulting mixture at room temperature for 18 hours, water was added thereto and the mixture was extracted with dichloromethane. The organic layer was washed with saturated brine, and dried over magnesium sulfate. After concentration under reduced pressure, the residue was separated and purified by column chromatography (ethyl acetate : hexane 1 : 19 → 1 : 9) to give ethyl 4-(6-bromo-3,4-dihydroquinolin-1(2H)-yl)butyrate (14.59 g) as a pale yellow oily material.

¹H-NMR (200MHz, CDCl₃) δ 1.26 (3H, t, J=7.2 Hz), 1. 81-1.97 (4H, m), 2.35 (2H, t, J=7.2 Hz), 2.71 (2H, t, J=6.4 Hz), 3.22-3.30 (4H, m), 4.14 (2H, q, J=7.2 Hz), 6.46 (1H, d, J=8.8 Hz), 7.03 (1H, d, J=2.6 Hz), 7.10 (1H, dd, J=8.8, 2.6 Hz).

### Reference Example 25

To a solution of ethyl 4-(6-bromo-3,4-dihydroquinolin-1(2H)-yl)butyrate (14.59 g) in DMF (75 ml) was added chloromethylenedimethyliminium chloride (8.64 g) at room temperature. After stirring at room temperature for 13 hours, the reaction mixture was added to ice. The resulting mixture was neutralized with a 1 N aqueous sodium hydroxide solution, and then extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4) to give ethyl 4-(6-bromo-8-formyl-3,4-dihydroquinolin-1(2H)-yl)butyrate (5.28 g) as a yellow oily material.

¹H-NMR (200MHz, CDCl₃) δ 1.25 (3H, t, J=7.2 Hz), 1.82-1.96 (4H, m), 2.27 (2H, t, J=7.2 Hz), 2.76 (2H, t, J=6.3 Hz), 3.12-3.26 (4H, m), 4.12 (2H, q, J=7.2 Hz), 7.25 (1H, d, J=2.6 Hz), 7.67 (1H, d, J=2.6 Hz), 9.97 (1H, s).

### Reference Example 26

To a solution of ethyl 4-(6-bromo-8-formyl-3,4-dihydroquinolin-1(2H)-yl)butyrate (5.66 g) in diethyl carbonate (100 ml) was added sodium ethoxide (20% solution in ethanol, 8.15 g) at room temperature, and the mixture was stirred at 50°C for 16 hours. After cooling to 0°C, the mixture was neutralized with 1 N hydrochloric acid and extracted with ethyl acetate. The organic layer was washed with an aqueous sodium hydrogen carbonate solution and saturated brine, and dried over magnesium sulfate. After concentration under reduced pressure, the residue was separated and purified by column chromatography (ethyl acetate : hexane 1 : 9 → 1 : 4) to give ethyl 10-bromo-2,3,5,6-tetrahydro-1H-azepino[3,2,1-ij]quinoline-7-carboxylate (4.82 g) as yellow crystals.

¹H-NMR (200MHz, CDCl₃) δ 1.33 (3H, t, J=7.1 Hz), 1.85-1.97 (2H, m), 2.68 (2H, t, J=6.0 Hz), 2.79-2.84 (2H, m), 3.21-3.32 (4H, m), 4.24 (2H, q, J=7.1 Hz), 7.00 (1H, d, J=2.6 Hz), 7.27 (1H, d, J=2.6 Hz), 7.55 (1H, s).

### Reference Example 27

Under an argon atmosphere, a mixture of ethyl 10-bromo-2,3,5,6-tetrahydro-1H-azepino[3,2,1-ij]quinoline-7-carboxylate (2.50 g), 4-(2-butoxyethoxy)phenylboric acid (2.3 g) and potassium carbonate (2.06 g) in toluene (70 ml), ethanol (7 ml) and water (7 ml) was stirred at room temperature for 1 hour. Tetrakis(triphenylphosphine)palladium (0.26 g) was added to the reaction system, and the mixture was heated under reflux for 6 hours. After cooling to room temperature, the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 15 → 1 : 9) to give ethyl 10-[4-(2-butoxyethoxy)phenyl]-2,3,5,6-tetrahydro-1H-azepino[3,2,1-ij]quinoline-7-carboxylate (2.74 g) as a yellow oily material.

¹H-NMR (200MHz, CDCl₃) δ 0.93 (3H, t, J=7.4 Hz), 1.31-1.50 (5H, m), 1.53-1.68 (2H, m), 1.89-2.04 (2H, m), 2.71-2.89 (4H, m), 3.27-3.37 (4H, m), 3.55 (2H, t, J=6.6 Hz), 3.80 (2H, t, J=4.9 Hz), 4.15 (2H, t, J=4.9 Hz), 4.26 (2H, q, J=7.0 Hz), 6.96 (2H, d, J=8.8 Hz), 7.15 (1H, d, J=2.0 Hz), 7.36 (1H, d, J=2.0 Hz), 7.46 (2H, d, J=8.8 Hz), 7.74 (1H, s).

### Reference Example 28

To a solution of ethyl 10-[4-(2-butoxyethoxy)phenyl]-2,3,5,6-tetrahydro-1H-azepino[3,2,1-ij]quinoline-7-carboxylate (2.74 g) in ethanol (30 ml) and THF (15 ml) was added a 1 N aqueous sodium hydroxide solution (15 ml) at room temperature. After stirring the resulting mixture at 60°C for 20 hours, 1 N hydrochloric acid (15 ml) was added thereto at 0°C. The mixture was extracted with ethyl acetate, and then the organic layer was washed with water and saturated brine, and dried over magnesium sulfate. After concentration under reduced pressure, the precipitated crystals were collected by filtration. The crystals were washed with hexane and diisopropyl ether to give 10-[4-(2-butoxyethoxy)phenyl]-2,3,5,6-tetrahydro-1H-azepino[3,2,1-ij]quinoline-7-carboxylic acid (2.1 g) as yellow crystals.

m.p. 200-202°C
¹H-NMR (200MHz, CDCl₃) δ 0.93 (3H, t, J=7.2 Hz), 1.28-1.48 (2H, m), 1.51-1.69 (2H, m), 1.92-2.07 (2H, m), 2.72-2.91 (4H, m), 3.26-3.41 (4H, m), 3.55 (2H, t, J=6.6 Hz), 3.80 (2H, t, J=5.0 Hz), 4.16 (2H, t, J=5.0 Hz), 6.96 (2H, d, J=8.5 Hz), 7.18 (1H, d, J=2.2 Hz), 7.37 (1H, d, J=2.2 Hz), 7.45 (2H, d, J=8.5 Hz), 7.87 (1H, s).

### Reference Example 29

A mixture of indoline (10.0 g), ethyl 5-bromovalerate (19.9 ml), potassium carbonate (23.2 g) and sodium iodide (18.86 g) in DMF (200 ml) was stirred at 120°C for 2 days. To the reaction system was added water and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 9) to give ethyl 5-(2,3-dihydro-1H-indol-1-yl)valerate (19.24 g) as a pale yellow oily material.

¹H-NMR (200MHz, CDCl₃) δ 1.26 (3H, t, J=7.1 Hz), 1.57-1.82 (4H, m), 2.36 (2H, t, J=7.1 Hz), 2.95 (2H, t, J=8.3 Hz), 3.06 (2H, t, J=6.8 Hz), 3.33 (2H, t, J=8.1 Hz), 4.13 (2H, q, J=7.1 Hz), 6.44-6.47 (1H, m), 6.59-6.67 (1H, m), 7.02-7.09 (2H, m).

### Reference Example 30

To ethyl 5-(2,3-dihydro-1H-indol-1-yl)valerate (19.24 g) in dichloromethane (250 ml) was added tetrabutylammonium tribromide (37.5 g) at 0°C. After stirring the resulting mixture at 0°C for 30 minutes and then at room temperature for 20 hours, water was added thereto and the mixture was extracted with ethyl acetate. The organic layer was washed with an aqueous sodium thiosulfate solution and saturated brine, and dried over magnesium sulfate. After concentration under reduced pressure, the residue was separated and purified by column chromatography (ethyl acetate : hexane 1 : 19 → 1 : 9 → 1 : 4) to give ethyl 5-(5-bromo-2,3-dihydro-1H-indol-1-yl)valerate (24.23 g) as a pale yellow oily material.

¹H-NMR (200MHz, CDCl₃) δ 1.26 (3H, t, J=7.1 Hz), 1.51-1.80 (4H, m), 2.35 (2H, t, J=7.1 Hz), 2.93 (2H, t, J=8.3 Hz), 3.03 (2H, t, J=6.8 Hz), 3.34 (2H, t, J=8.3 Hz), 4.13 (2H, q, J=7.1 Hz), 6.29 (1H, d, J=8.8 Hz), 7.06-7.16 (2H, m).

IR (neat) 1732, 1599, 1489, 1470, 1258, 1182 cm⁻¹

### Reference Example 31

To a solution of chloromethylenedimethyliminium chloride (14.3 g) in DMF (250 ml) was added a solution of ethyl 5-(5-bromo-2,3-dihydro-1H-indol-1-yl)valerate (24.23 g) in DMF (50 ml) at room temperature. After stirring at room temperature for 20 hours, the reaction mixture was added to ice. The resulting mixture was neutralized with potassium carbonate and extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 8 → 1 : 7) to give ethyl 5-(5-bromo-7-formyl-2,3-dihydro-1H-indol-1-yl)valerate (14.26 g) as a yellow oily material.

¹H-NMR (200MHz, CDCl₃) δ 1.26 (3H, t, J=7.2 Hz), 1.53-1.76 (4H, m), 2.31-2.38 (2H, m), 3.04 (2H, t, J=8.8 Hz), 3.43-3.50 (2H, m), 3.66 (2H, t, J=8.8 Hz), 4.13 (2H, q, J=7.2 Hz), 7.16-7.18 (1H, m), 7.54-7.55 (1H, m), 9.84 (1H, s).

IR (neat) 1732, 1682, 1661, 1497, 1456, 1375, 1277, 1198 cm⁻¹

### Reference Example 32

To a solution of ethyl 5-(5-bromo-7-formyl-2,3-dihydro-1H-indol-1-yl)valerate (14.26 g) in diethyl carbonate (300 ml) was added sodium ethoxide (20% solution in ethanol, 20.5 g) at room temperature, and the mixture was stirred at 65°C for 14 hours. The resulting mixture was neutralized with 1 N hydrochloric acid at 0°C and extracted with ethyl acetate. The organic layer was washed with saturated brine, and dried over magnesium sulfate. After concentration under reduced pressure, the residue was separated and purified by column chromatography (ethyl acetate : hexane 1 : 19) to give ethyl 10-bromo-1,2,5,6-tetrahydro-4H-azocino[3,2,1-hi]indole-7-carboxylate (11.63 g) as a yellow oily material.

¹H-NMR (200MHz, CDCl₃) δ 1.33 (3H, t, J=7.0 Hz), 1.37-1.58 (2H, m), 2.66 (2H, t, J=6.3 Hz), 2.95 (2H, t, J=8.8 Hz), 3.31-3.53 (4H, m), 4.22 (2H, q, J=7.0 Hz), 6.92-7.02 (2H, m), 7.58 (1H, s).

IR (neat) 1699, 1624, 1574, 1464, 1269, 1194, 1182, 1096, 1030, 912, 737 cm⁻¹

### Reference Example 33

Under an argon atmosphere, a mixture of ethyl 10-bromo-1,2,5,6-tetrahydro-4H-azocino[3,2,1-hi]indole-7-carboxylate (10.0 g), 4-(2-butoxyethoxy)phenylboric acid (8.50 g) and potassium carbonate (8.21 g) in toluene (300 ml), ethanol (30 ml) and water (30 ml) was stirred at room temperature for 0.5 hour. Tetrakis(triphenylphosphine)palladium (1.03 g) was added to the reaction system, and the mixture was heated under reflux for 6 hours. After cooling to room temperature, the mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 9) to give ethyl 10-[4-(2-butoxyethoxy)phenyl]-1,2,5,6-tetrahydro-4H-azocino[3,2,1-hi]indole-7-carboxylate as a yellow oily material. To a solution of ethyl 10-[4-(2-butoxyethoxy)phenyl]-1,2,5,6-tetrahydro-4H-azocino[3,2,1-hi]indole-7-carboxylate (6.19 g) in ethanol (40 ml) and THF (20 ml) was added a 1 N aqueous sodium hydroxide solution (2.6 ml) at room temperature, and the mixture was stirred at 65°C for 6 hours. After cooling to 0°C, 1 N hydrochloric acid (2.6 ml) was added thereto and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 2 → 1 : 1 → ethyl acetate) to give 10-[4-(2-butoxyethoxy)phenyl]-1,2,5,6-tetrahydro-4H-azocino[3,2,1-hi]indole-7-carboxylic acid (3.77 g). To a mixture of 10-[4-(2-butoxyethoxy)phenyl]-1,2,5,6-tetrahydro-4H-azocino[3,2,1-hi]indole-7-carboxylic acid (3.77 g) and potassium carbonate (2.49 g) in DMF (200, 1) was added iodomethane (0.56 ml) at room temperature. After stirring the resulting mixture at room temperature for 3 hours, water was added thereto and the mixture was extracted with ethyl acetate. The organic layer was washed with saturated brine, and dried over magnesium sulfate. After concentration under reduced pressure, the residue was separated and purified by column chromatography (basic silica gel, ethyl acetate : hexane 1 : 19) to give methyl 10-[4-(2-butoxyethoxy)phenyl]-1,2,5,6-tetrahydro-4H-azocino[3,2,1-hi]indole-7-carboxylate (2.72 g) as yellow crystals.

¹H-NMR (200MHz, CDCl₃) δ 0.93 (3H, t, J=7.2 Hz), 1.29-1.68 (6H, m), 2.72 (2H, t, J=6.4 Hz), 3.01 (2H, t, J=8.4 Hz), 3.35-3.58 (6H, m), 3.77-3.82 (5H, m), 4.14 (2H, t, J=4.9 Hz), 6.94 (2H, d, J=8.8 Hz), 7.04 (1H, d, J=1.8 Hz), 7.20 (1H, d, J=1.8 Hz), 7.40 (2H, d, J=8.8 Hz), 7.76 (1H, s).

### Reference Example 34

To a solution of methyl 10-[4-(2-butoxyethoxy)phenyl]-1,2,5,6-tetrahydro-4H-azocino[3,2,1-hi]indole-7-carboxylate (1.14 g) in ethanol(20 ml) and THF (10 ml) was added a 1 N aqueous sodium hydroxide solution (5.0 ml) at room temperature, and the mixture was stirred at 65°C for 4 hours. After cooling to 0°C, 1 N hydrochloric acid (5.0 ml) was added thereto, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give 10-[4-(2-butoxyethoxy)phenyl]-1,2,5,6-tetrahydro-4H-azocino[3,2,1-hi]indole-7-carboxylic acid (1.02 g) as yellow crystals.

m.p. 217-218°C
¹H-NMR (200MHz, CDCl₃) δ 0.93 (3H, t, J=7.3 Hz), 1.30-1.67 (6H, m), 2.66-2.80 (2H, m), 2.94-3.08 (2H, m), 3.32-3.58 (6H, m), 3.80 (2H, t, J=4.8 Hz), 4.15 (2H, t, J=4.8 Hz), 6.95 (2H, d, J=8.6 Hz), 7.06 (1H, br s), 7.21 (1H, br s), 7.41 (2H, d, J=8.6 Hz), 7.89 (1H, s).

### Reference Example 35

A mixture of 1,2,3,4-tetrahydroquinoline (10.0 g), ethyl 5-bromovalerate (17.8 ml), sodium iodide (16.9 g) and potassium carbonate (20.7 g) in DMF (100 ml) was stirred at 100°C for 5 days. To the reaction system were added ethyl 5-bromovalerate (3.6 ml) and potassium carbonate (5.18 g), and the mixture was further stirred at 120°C for 2 days. After cooling to room temperature, water was added thereto and the mixture was extracted with ethyl acetate. The organic layer was washed with water and saturated brine, and dried over magnesium sulfate. After concentration under reduced pressure, the residue was separated and purified by column chromatography (ethyl acetate : hexane 1 : 19) to give ethyl 5-(3,4-dihydroquinolin-1(2H)-yl)valerate (17.92 g) as a pale yellow oily material.

¹H-NMR (200MHz, CDCl₃) δ 1.26 (3H, t, J=7.2 Hz), 1.49-1.78 (4H, m), 1.88-2.00 (2H, m), 2.34 (2H, t, J=7.1 Hz), 2.74 (2H, t, J=6.4 Hz), 3.22-3,29 (4H, m), 4.13 (2H, q, J=7.2 Hz), 6.52-6.58 (2H, m), 6.91-7.07 (2H, m).

IR (neat) 1734, 1601, 1505, 1456, 1373, 1346, 1182, 745 cm⁻¹

### Reference Example 36

To a solution of ethyl 5-(3,4-dihydroquinolin-1(2H)-yl)valerate (17.96 g) in dichloromethane (500 ml) was added tetrabutylammonium tribromide (33.10 g) at 0°C. After stirring the resulting mixture at room temperature for 18 hours, water was added thereto and the mixture was extracted with dichloromethane. The organic layer was washed with saturated brine, and dried over magnesium sulfate. After concentration under reduced pressure, the residue was separated and purified by column chromatography (ethyl acetate : hexane 1 : 19 → 1 : 9) to give ethyl 5-(6-bromo-3,4-dihydroquinolin-1(2H)-yl)valerate (22.83 g) as a pale yellow oily material.

¹H-NMR (200MHz, CDCl₃) δ 1.26 (3H, t, J=7.2 Hz), 1.47-1. 75 (4H, m), 1.85-1.97 (2H, m), 2.34 (2H, t, J=7.2 Hz), 2.71 (2H, t, J=6.4 Hz), 3.19-3.28 (4H, m), 4.13 (2H, q, J=7.2 Hz), 6.39 (1H, d, J=8.8 Hz), 7.02 (1H, d, J=2.6 Hz), 7.09 (1H, dd, J=8.8, 2.6 Hz).

IR (neat) 1732, 1593, 1499, 1337, 1192 cm⁻¹

### Reference Example 37

To a solution of ethyl 5-(6-bromo-3,4-dihydroquinolin-1(2H)-yl)valerate (22.83 g) in DMF (250 ml) was added chloromethylenedimethyliminium chloride (14.6 g) at room temperature. After stirring the resulting mixture at 50°C for 40 minutes, the mixture was returned to room temperature and further stirred for 20 hours. The reaction mixture was added to ice, and the mixture was neutralized with potassium carbonate and extracted with ethyl acetate. The organic layer was washed with water and saturated brine, and dried over magnesium sulfate. After concentration under reduced pressure, the residue was separated and purified by column chromatography (ethyl acetate : hexane 1 : 19 → 1 : 9 → 1 : 4) to give ethyl 5-(6-bromo-8-formyl-3,4-dihydroquinolin-1(2H)-yl)valerate (10.96 g) as a yellow oily material.

¹H-NMR (200MHz, CDCl₃) δ 1.26 (3H, t, J=7.1 Hz), 1.46-1.95 (6H, m), 2.31 (2H, t, J=7.2 Hz), 2.75 (2H, t, J=6.4 Hz), 3.11-3.23 (4H, m), 4.13 (2H, q, J=7.1 Hz), 7.24 (1H, d, J=2.5 HZ), 7.67 (1H, d, J=2.5 Hz), 9.95 (1H, s).

IR (neat) 1732, 1676, 1453, 1375, 1233, 1188, 1163, 1030, 912, 741 cm⁻¹

### Reference Example 38

To a solution of ethyl 5-(6-bromo-8-formyl-3,4-dihydroquinolin-1(2H)-yl)valerate (10.96 g) in diethyl carbonate (200 ml) was added sodium ethoxide (20% solution in ethanol, 15.2 g) at room temperature. After stirring the resulting mixture at 60°C for 2 hours, the mixture was cooled to room temperature and further stirred for 12 hours. The resulting mixture was acidified with 1 N hydrochloric acid (pH 3 to 4) and extracted with ethyl acetate. The organic layer was washed with saturated brine, and dried over magnesium sulfate. After concentration under reduced pressure, the residue was separated and purified by column chromatography (ethyl acetate : hexane = 1 : 9) to give ethyl 11-bromo-2,3,6,7-tetrahydro-1H,5H-azocino[3,2,1-ij]quinoline-8-carboxylate (7.81 g) as a yellow oily material.

¹H-NMR (200MHz, CDCl₃) δ 1.23-1.48 (5H, m), 1.84-1.99 (2H, m), 2.47 (2H, t, J=6.0 Hz), 2.69 (2H, t, J=6.1 Hz), 3.14 (2H, t, J=6.0 Hz), 3.34-3.46 (2H, m), 4.23 (2H, q, J=7.2 Hz), 6.99 (1H, d, J=2.6 Hz), 7.09 (1H, d, J=2.6 Hz), 7.68 (1H, s).

IR (neat) 1698, 1497, 1449, 1264, 1219, 1181, 1098, 1046, 912, 741 cm⁻¹

### Reference Example 39

Under an argon atmosphere, a mixture of ethyl 11-bromo-2,3,6,7-tetrahydro-1H,5H-azocino[3,2,1-ij]quinoline-8-carboxylate (2.0 g), 4-(2-butoxyethoxy)phenylboric acid (1.63 g) and potassium carbonate (1.58 g) in toluene (60 ml), ethanol (6 ml) and water (6 ml) was stirred at room temperature for 1 hour. Tetrakis(triphenylphosphine)palladium (0.20 g) was added to the reaction system, and the mixture was heated under reflux for 7 hours. After cooling to room temperature, the mixture was extracted with ethyl acetate. The organic layer was washed with saturated brine, and dried over magnesium sulfate. After concentration under reduced pressure, the residue was separated and purified by column chromatography (ethyl acetate : hexane 1 : 19 → 1 : 9) to give ethyl 11-[4-(2-butoxyethoxy)phenyl]-2,3,6,7-tetrahydro-1H,5H-azocino[3,2,1-ij]quinoline-8-carboxylate (1.4 g) as yellow crystals.

¹H-NMR (200MHz, CDCl₃) δ 0.93 (3H, t, J=7.2 Hz), 1.27-1.49 (5H, m), 1.52-1.67 (2H, m), 1.90-2.05 (2H, m), 2.52 (2H, t, J=6.2 Hz), 2.79 (2H, t, J=6.1 Hz), 3.19 (2H, t, J=6.2 Hz), 3.40-3.52 (2H, m), 3.55 (2H, t, J=6.6 Hz), 3.80 (2H, t, J=4.9 Hz), 4.14 (2H, t, J=4.9 Hz), 4.25 (2H, q, J=7.1 Hz), 6.94 (2H, d, J=8.6 Hz), 7.15 (1H, d, J=2.2 Hz), 7.19 (1H, d, J=2.2 Hz), 7.43 (2H, d, J=8.6 Hz), 7.87 (1H, s).

### Reference Example 40

To a solution of ethyl 11-[4-(2-butoxyethoxy)phenyl]-2,3,6,7-tetrahydro-1H,5H-azocino[3,2,1-ij]quinoline-8-carboxylate (1.4 g) in ethanol (20 ml) and THF (10 ml) was added a 1 N aqueous sodium hydroxide solution (10 ml) at room temperature. After stirring the resulting mixture at 65°C for 3 hours, 1 N hydrochloric acid (10 ml) was added thereto at 0°C and the mixture was extracted with ethyl acetate. The organic layer was washed with saturated brine, and dried over magnesium sulfate. After concentration under reduced pressure, the precipitated crystals were collected by filtration. The crystals were washed with diisopropyl ether-hexane to give 11-[4-(2-butoxyethoxy)phenyl]-2,3,6,7-tetrahydro-1H,5H-azocino[3,2,1-ij]quinoline-8-carboxylic acid (940 mg) as yellow crystals.

¹H-NMR (200MHz, CDCl₃) δ 0.93 (3H, t, J=7.3 Hz), 1.28-1.67 (6H, m), 1.92-2.05 (2H, m), 2.45-2.60 (2H, m), 2.74-2.85 (2H, m), 3.17-3.23 (2H, m), 3.41-3.58 (4H, m), 3.80 (2H, t, J=5.0 Hz), 4.15 (2H, t, J=5.0 Hz), 6.94 (2H, d, J=8.8 Hz), 7.18-7.20 (2H, m), 7.42 (2H, d, J=8.8 Hz), 8.00 (1H, s).

### Reference Example 41

A mixture of 3,4-dihydro-2H-1,4-benzoxazine (12.2 g), ethyl 5-bromovalerate (20 ml), sodium iodide (20.1 g) and potassium carbonate (23.42 g) in DMF (100 ml) was stirred at 125°C for 2 days. To the reaction system was added water and the mixture was extracted with ethyl acetate. The organic layer was washed with water and saturated brine, and dried over magnesium sulfate. The residue was separated and purified by column chromatography (ethyl acetate : hexane 1 : 9 → 1 : 4) to give ethyl 5-(2,3-dihydro-4H-1,4-benzoxazin-4-yl)pentanoate (12.23 g) as a yellow oily material.

¹H-NMR (200MHz, CDCl₃) δ 1.26 (3H, t, J=7.1 Hz), 1.49-1.78 (4H, m), 2.32-2.39 (2H, m), 3.22-3.29 (2H, m), 3.33 (2H, t, J=4.4 Hz), 4.13 (2H, q, J=7.1 Hz), 4.24 (2H, t, J=4.4 Hz), 6.56-6.66 (2H, m), 6.75-6.87 (2H, m).

IR (neat) 1732, 1505, 1372, 1348, 1310, 1236, 912, 743 cm⁻¹

### Reference Example 42

To ethyl 5-(2,3-dihydro-4H-1,4-benzoxazin-4-yl)pentanoate (1.0 g) in dichloromethane (10 ml) was added tetrabutylammonium tribromide (1.83 g) at -20°C. After stirring the resulting mixture at -20°C for 30 minutes and at 0°C for 1 hour, water was added thereto and the mixture was extracted with ethyl acetate. The organic layer was washed with an aqueous sodium thiosulfate solution and saturated brine, and dried over magnesium sulfate. After concentration under reduced pressure, the residue was separated and purified by column chromatography (ethyl acetate : hexane 1 : 4) to give ethyl 5-(7-bromo-2,3-dihydro-4H-1,4-benzoxazin-4-yl)pentanoate (1.18 g) as a yellow oily material.

¹H-NMR (200MHz, CDCl₃) δ 1.25 (3H, t, J=7.0 Hz), 1.52-1.75 (4H, m), 2.31-2.38 (2H, m), 3.19-3.26 (2H, m), 3.30 (2H, t, J=4.4 Hz), 4.13 (2H, q, J=7.0 Hz), 4.21 (2H, t, J=4.4 Hz), 6.48 (1H, d, J=9.4 Hz), 6.87-6.93 (2H, m).

IR (neat) 1732, 1505, 1304, 1252, 1049 cm⁻¹

### Reference Example 43

To a solution of ethyl 5-(7-bromo-2,3-dihydro-4H-1,4-benzoxazin-4-yl)pentanoate (1.18 g) in DMF (10 ml) was added chloromethylenedimethylammonium chloride (0.53 g) at room temperature, and the mixture was stirred at room temperature for 2 hours. To the reaction system was added chloromethylenedimethylammonium chloride (0.6 g), and the mixture was further stirred at 50°C for 16 hours. Then, the reaction mixture was added to ice, the resulting mixture was neutralized with an aqueous sodium hydrogen carbonate solution and extracted with ethyl acetate. The organic layer was washed with water and saturated brine, and dried over magnesium sulfate. After concentration under reduced pressure, the residue was separated and purified by column chromatography (ethyl acetate : hexane 1 : 9 → 1 : 4) to give ethyl 5-(7-bromo-5-formyl-2,3-dihydro-4H-1,4-benzoxazin-4-yl)pentanoate (414.2 mg) as yellow crystals.

¹H-NMR (300MHz, CDCl₃) δ 1.26 (3H, t, J=7.1 Hz), 1.56-1.69 (2H, m), 1.70-1.83 (2H, m), 2.34 (2H, t, J=7.2 Hz), 3.04 (2H, t, J=7.8 Hz), 3.22 (2H, t, J=4.4 Hz), 4.10-4.17 (4H, m), 7.15 (1H, d, J=2.4 Hz), 7.47 (1H, d, J=2.4 Hz), 10.08 (1H, s).

### Reference Example 44

To a solution of ethyl 5-(7-bromo-5-formyl-2,3-dihydro-4H-1,4-benzoxazin-4-yl)pentanoate (3.39 g) in diethyl carbonate (40 ml) was added sodium ethoxide (20% solution in ethanol, 4.67 g) at room temperature, and the mixture was stirred at 55°C for 2 hours. To the reaction system was added water and the mixture was extracted with ethyl acetate. The organic layer was washed with saturated brine, and dried over magnesium sulfate. After concentration under reduced pressure, the residue was separated and purified by column chromatography (ethyl acetate : hexane 1 : 9) to give ethyl 11-bromo-2,3,6,7-tetrahydro-5H-[1,4]oxazino[2,3,4-kl][1]benzazocine-8-carboxylate (1.92 g) as yellow crystals.

¹H-NMR (200MHz, CDCl₃) δ 1.29-1.46 (5H, m), 2.57 (2H, t, J=6.2 Hz), 3.32 (2H, t, J=4.7 Hz), 3.45 (2H, t, J=5.7 Hz), 4.16 (2H, t, J=4.7 Hz), 4.23 (2H, q, J=7.2 Hz), 6.87 (2H, s), 7.69 (1H, s).

### Reference Example 45

Under an argon atmosphere, a mixture of ethyl 11-bromo-2,3,6,7-tetrahydro-5H-[1,4]oxazino[2,3,4-kl][1]benzazocine-8-carboxylate (1.92 g), 4-(2-butoxyethoxy)phenylboric acid (1.56 g) and potassium carbonate (1.51 g) in toluene (60 ml), ethanol (6 ml) and water (6 ml) was stirred at room temperature for 1 hour. Tetrakis(triphenylphosphine)palladium (0.19 g) was added to the reaction system, and the mixture was heated under reflux for 6 hours. After cooling to room temperature, the mixture was extracted with ethyl acetate. The organic layer was washed with saturated brine, and dried over magnesium sulfate. After concentration under reduced pressure, the residue was separated and purified by column chromatography (basic silica gel, ethyl acetate : hexane 1 : 19 → 1 : 9) to give ethyl 11-[4-(2-butoxyethoxy)phenyl]-2,3,6,7-tetrahydro-5H-[1,4]oxazino[2,3,4-kl][1]benzazocine-8-carboxylate (1.62 g) as a yellow solid.

¹H-NMR (200MHz, CDCl₃) δ 0.93 (3H, t, J=7.3 Hz), 1.22-1.64 (9H, m), 2.55-2.68 (2H, m), 3.27-3.58 (6H, m), 3.80 (2H, t, J=4.9 Hz), 4.07-4.30 (6H, m), 6.87-7.05 (4H, m), 7.34-7.51 (2H, m), 7.87 (1H, br s).

### Reference Example 46

To a solution of ethyl 11-[4-(2-butoxyethoxy)phenyl]-2,3,6,7-tetrahydro-5H-[1,4]oxazino[2,3,4-kl][1]benzazocine-8-carboxylate (1.62 g) in ethanol (20 ml) and THF (10 ml) was added a 1 N aqueous sodium hydroxide solution (8.0 ml) at room temperature, and the mixture was stirred at 60°C for 20 hours. After cooling to 0°C, 1 N hydrochloric acid (8.0 ml) was added thereto, and the mixture was extracted with ethyl acetate. The organic layer was washed with water and saturated brine, and dried over magnesium sulfate. After concentration under reduced pressure, the precipitated crystals were collected by filtration. The crystals were washed with diisopropyl ether and hexane to give 11-[4-(2-butoxyethoxy)phenyl]-2,3,6,7-tetrahydro-5H-[1,4]oxazino[2,3,4-kl][1]benzazocine-8-carboxylic acid (1.19 g) as yellow crystals.

m.p. 205-209°C
¹H-NMR (200MHz, CDCl₃) δ 0.93 (3H, t, J=7.1 Hz), 1.28-1.68 (6H, m), 2.58-2.69 (2H, m), 3.39 (2H, t, J=4.7 Hz), 3.47-3.58 (4H, m), 3.80 (2H, t, J=4.9 Hz), 4.15 (2H, t, J=4.9 Hz), 4.22 (2H, t, J=4.7 Hz), 6.95 (2H, d, J=8.8 Hz), 6.98 (1H, d, J=2.2 Hz), 7.04 (1H, d, J=2.2 Hz), 7.42 (2H, d, J=8.8 Hz), 8.01 (1H, s).

### Reference Example 47

4-(Pyrrolidin-2-yl)butanoic acid (1.39 g), 5-bromo-2-chloronicotinaldehyde (1.5 g) and sodium carbonate (1.88 g) were added to DMSO (30 ml) and water (15 ml), and then the mixture was stirred at 90°C for 2 hours. The resulting mixture was cooled to 0°C, water was added thereto, 1 N hydrochloric acid (35 ml) was then added thereto, and the mixture was extracted with ethyl acetate. The organic layer was washed with saturated brine, dried over magnesium sulfate, and the solvent was distilled off under reduced pressure. The resulting residue as such was dissolved in DMF (50 ml), potassium carbonate (2.82 g) and iodomethane (1.27 ml) were added thereto, and then the mixture was stirred at room temperature for 2 hours under a nitrogen atmosphere. Water was added thereto, and the mixture was then extracted with ethyl acetate. The organic layer was washed with saturated brine, and dried over magnesium sulfate. The solvent was distilled off under reduced pressure, and the residue was purified by silica gel column chromatography (hexane : ethyl acetate = 10 : 1 → hexane : ethyl acetate = 4 : 1) to give methyl 4-[1-(5-bromo-3-formylpyridin-2-yl)pyrrolidin-2-yl]butanoate (2.26 g) as a yellow oily material.

¹H-NMR (300MHz, CDCl₃) δ 1.40-2.40 (10H, m), 3.04-3.44 (1H, m), 3.66 (3H, s), 3.70-3.79 (1H, m), 4.37-4.50 (1H, m), 8.01 (1H, d, J=2.7 Hz), 8.30 (1H, d, J=2.7 Hz), 9.91 (1H, s).

### Reference Example 48

To a solution of methyl 4-[1-(5-bromo-3-formylpyridin-2-yl)pyrrolidin-2-yl]butanoate (2.2 g) in dimethyl carbonate (50 ml) was added sodium methoxide (28% solution in methanol, 2.39 g), and the mixture was heated at 65°C for 2 hours under a nitrogen atmosphere. The resulting mixture was returned to room temperature, water was added thereto, and then the mixture was extracted with ethyl acetate. The organic layer was washed with saturated brine, and dried over magnesium sulfate. The solvent was distilled off under reduced pressure, and the residue was purified by silica gel column chromatography (hexane : ethyl acetate = 9 : 1). The resulting residue was washed with hexane-ethyl acetate to give methyl 3-bromo-7,8,8a,9,10,11-hexahydropyrido[3,2-g]pyrrolo[1,2-a]azocine-6-carboxylate (1.77 g) as yellow crystals.

m.p. 98.0-99.0°C
¹H-NMR (300MHz, CDCl₃) δ 1.17-1.28 (1H, m), 1.41-1.50 (1H, m), 1.69-1.73 (1H, m), 1.90-2.00 (3H, m), 2.40-2.51 (1H, m), 2.77-2.84 (1H, m), 3.47-3.63 (2H, m), 3.79 (3H, s), 4.11-4.29 (1H, m), 7.38 (1H, d, J=2.4 Hz), 7.57 (1H, s), 8.10 (1H, d, J=2.4 Hz).

Elementary analysis C₁₅H₁₇N₂O₂Br, Calcd. C, 53.43 ; H, 5.08 ; N, 8.31 ; Found. C, 53.51 ; H, 5.12 ; N, 8.14.

### Reference Example 49

A suspension of methyl 3-bromo-7,8,8a,9,10,11-hexahydropyrido[3,2-g]pyrrolo[1,2-a]azocine-6-carboxylate (1.7 g), 4-(2-butoxyethoxy)phenylboric acid (1.56 g) and potassium carbonate (1.81 g) in toluene (30 ml), ethanol (3 ml) and water (3 ml) was stirred for 30 minutes under an argon atmosphere. Tetrakis(triphenylphosphine)palladium (291 mg) was then added thereto, and the mixture was heated at 115°C for 6 hours under an argon atmosphere, which was allowed to cool. Water was added thereto and the mixture was extracted with ethyl acetate. The organic layer was washed with saturated brine, and dried over magnesium sulfate. The solvent was distilled off under reduced pressure, and the resulting residue was purified by silica gel column chromatography (hexane : ethyl acetate = 19 : 1 → hexane : ethyl acetate = 4 : 1) to give methyl 3-[4-(2-butoxyethoxy)phenyl]-7,8,8a,9,10,11-hexahydropyrido[3,2-g]pyrrolo[1,2-a]azocine-6-carboxylate (2.06 g) as a yellow oily material.

¹H-NMR (300MHz, CDCl₃) δ 0.93 (3H, t, J=7.5 Hz), 1.21-1.75 (7H, m), 1.95-2.00 (3H, m), 2.49-2.59 (1H, m), 2.80-2.86 (1H, m), 3.53-3.70 (4H, m), 3.78-3.82 (5H, m), 4.15 (2H, t, J=4.5 Hz), 4.25-4.40 (1H, m), 6.97 (2H, d, J=8.7 Hz), 7.40 (2H, d, J=8.7 Hz), 7.50 (1H, d, J=2.4 Hz), 7.75 (1H, s), 8.34 (1H, d, J=2.4 Hz).

### Reference Example 50

To a solution of methyl 3-[4-(2-butoxyethoxy)phenyl]-7,8,8a,9,10,11-hexahydropyrido[3,2-g]pyrrolo[1,2-a]azocine-6-carboxylate (2.0 g) in THF (71 ml) and methanol (71 ml) was added a 1 N aqueous sodium hydroxide solution (17.8 ml), and the mixture was heated at 90°C for 3 hours. The resulting mixture was allowed to cool, and the solvent was distilled off under reduced pressure. After cooling to 0°C, water was added thereto. The resulting mixture was neutralized with 1 N hydrochloric acid and extracted with ethyl acetate. The organic layer was washed with saturated brine, and then dried over magnesium sulfate. The solvent was distilled off under reduced pressure, and the resulting residue was washed with hexane to give 3-[4-(2-butoxyethoxy)phenyl]-7,8,8a,9,10,11-hexahydropyrido[3,2-g]pyrrolo[1,2-a]azocine-6-carboxylic acid (1.66 g) as yellow crystals.

m.p. 184.5-186.0°C
¹H-NMR (300MHz, CDCl₃) δ 0.93 (3H, t, J=7.2 Hz), 1.28-1.75 (7H, m), 1.80-2.10 (3H, m), 2.51-2.60 (1H, m), 2.80-2.88 (1H, m), 3.53-3.75 (4H, m), 3.80 (2H, t, J=4.5 Hz), 4.15 (2H, t, J=4.5 Hz), 4.30-4.35 (1H, m), 6.98 (2H, d, J=9.0 Hz), 7.41 (2H, d, J=9.0 Hz), 7.52 (1H, d, J=1.8 Hz), 7.87 (1H, s), 8.36 (1H, d, J=1.8 Hz).

Elementary analysis C₂₆H₃₂N₂O₄, Calcd. C, 71.53 ; H, 7.39 ; N, 6.42 ; Found. C, 71.39 ; H, 7.44 ; N, 6.12.

### Reference Example 51

A mixture of potassium thiocyanate (119.2 g), dihydroxyacetone dimer (73.9 g) and propylamine hydrochloride (100 g) was added portionwise to a mixed solution of acetic acid (89 ml) and 1-butanol(590 ml). The mixture was stirred at room temperature for 1 day, and then water (118 ml) was added thereto, followed by stirring for 30 minutes. The precipitated solid was collected by filtration, and further washed with water (180 ml) twice and hexane once. The resulting solid was dried under reduced pressure to give 5-hydroxymethyl-2-mercapto-1-propylimidazole (71.2 g) as colorless crystals.

¹H-NMR (200MHz, CDCl₃) δ 0.87 (3H, t, J=7.4 Hz), 1.61-1.79 (2H, m), 3.91 (2H, t, J=7.4 Hz), 4.32 (2H, s), 5.26 (1H, br), 6.79 (1H, s), 11.95 (1H, s).

Elementary analysis C₇H₁₂N₂OS·0.25H₂O, Calcd. C, 47.57 ; H, 7.13 ; N, 15.85 ; Found. C, 47.22 ; H, 6.94 ; N, 15.99.

### Reference Example 52

To 5.0 M nitric acid (370 ml) was added sodium nitrate (1.14 g), and then 5-hydroxymethyl-2-mercapto-1-propylimidazole (71.0 g) was added portionwise at 0°C. The mixture was returned to room temperature and stirred for 2 hours, followed by adding water (200 ml) thereto. The resulting mixture was neutralized with potassium carbonate at 0°C, and then the solvent was distilled off under reduced pressure. Ethanol was added thereto, the insolubles were removed by filtration, and the solvent was then distilled off under reduced pressure. To the resulting residue was added methanol-ethyl acetate, and then basic silica gel was added thereto. The resulting mixture was purified by basic silica gel column chromatography (methanol-ethyl acetate = 1 : 8). The resulting solid was recrystallized from diisopropyl ether-ethyl acetate to give 5-hydroxymethyl-1-propylimidazole (33.6 g) as brown crystals.

¹H-NMR (200MHz, CDCl₃) δ 0.96 (3H, t, J=7.4 Hz), 1.76-1.94 (2H, m), 3.97 (2H, t, J=7.2 Hz), 4.63 (2H, s), 6.97 (1H, s), 7.48 (1H, s).

### Reference Example 53

To 5-hydroxymethyl-1-propylimidazole (33.0 g) was added portionwise thionyl chloride (80 ml) at 0°C, and the mixture was heated at 90°C for 30 minutes under a nitrogen atmosphere. The mixture was returned to room temperature, and then the solvent was distilled off under reduced pressure. The resulting residue was dissolved in methanol, and the solvent was again distilled off under reduced pressure. The resulting solid was recrystallized from ethyl acetate to give 5-chloromethyl-1-propylimidazole hydrochloride (43.8 g) as colorless crystals.

¹H-NMR (200MHz, DMSO-d₆) δ 0.92 (3H, t, J=7.4 Hz), 1.84-1.95 (2H, m), 4.18 (2H, t, J=7.2 Hz), 5.04 (2H, s), 7.82 (1H, s), 9.24 (1H, s).

### Reference Example 54

4-Aminothiophenol (2.5 g) was dissolved in water (2.5 ml) and isopropanol (10 ml). Triethylamine (5.5 ml) was added thereto, and then the mixture was cooled to -15 to -10°C. A solution of 5-(chloromethyl)-1-propyl-1H-imidazole hydrochloride (3.9 g) in water (2.5 ml) was added dropwise at -15 to -10°C, and the mixture was stirred at the same temperature for 1 hour. After isopropanol was distilled off under reduced pressure, methyl isobutyl ketone (25 ml) was added thereto, and the organic layer was washed with water. To the organic layer was added activated carbon (0.1 g), and the mixture was stirred at room temperature for 10 minutes. The organic layer was concentrated and dissolved in methyl isobutyl ketone (30 ml). Separately, di-p-toluoyl-(D)-tartaric acid (7.7 g) was dissolved in a mixed solution of toluene (90 ml) and methyl isobutyl ketone (60 ml), and to the solution was added water (3.6 ml). Then, the above methyl isobutyl ketone solution was slowly added dropwise over 2 hours. After stirring the resulting mixture for 1 hour, aqueous 30% hydrogen peroxide (6.8 g) was added thereto, and the mixture was stirred at room temperature for 24 hours. Methanol (30 ml) was added thereto and the mixture was stirred at 50°C for 8 hours. Water (30 ml) was added thereto, and the mixture was stirred at room temperature for 5 hours. The precipitated crystals were collected by filtration and washed with water (30 ml) to give (-)-4-{[(1-propyl-1H-imidazol-5-yl)methyl]sulfinyl}phenylamine di-p-toluoyl-D-tartrate monohydrate (7.1 g).

m.p. 134-136°C

### Reference Example 55

L(-)-proline (17.0 g) was dissolved in tetrahydrofuran (350 ml) and a 1 N aqueous sodium hydroxide solution (296 ml), and then benzyl chlorocarbonate (23.2 ml) was added dropwise thereto at 0°C under a nitrogen atmosphere. The resulting mixture was returned to room temperature and stirred overnight. 1 N Hydrochloric acid (296 ml) was added thereto at 0°C, and the mixture was extracted with ethyl acetate. The organic layer was washed with saturated brine, and dried over magnesium sulfate. The solvent was distilled off under reduced pressure to give 1-[(benzyloxy)carbonyl]-L-proline, which was as such dissolved in tetrahydrofuran (250 ml), and then triethylamine (26.8 ml) was added thereto. To the resulting solution was added dropwise ethyl chlorocarbonate (16.9 ml) at 0°C under a nitrogen atmosphere. The mixture was returned to room temperature and stirred for 1 hour, and the insolubles were removed by filtration. To the filtrate was added dropwise an aqueous sodium borohydride (11.2 g) solution (100 ml) at 0°C, and the mixture was returned to room temperature and stirred for 3 hours under a nitrogen atmosphere. Next, water was added thereto, and the mixture was extracted with ethyl acetate twice. The organic layer was washed with saturated brine. The solvent was distilled off under reduced pressure, and the resulting residue was purified by silica gel column chromatography (hexane : ethyl acetate 2 : 1 → 1 : 4) to give benzyl (2S)-2-(hydroxymethyl)pyrrolidine-1-carboxylate (25.0 g) as a colorless oily material. To a solution of oxalyl chloride (6.23 ml) in dichloromethane (70 ml) was added dropwise a solution of dimethyl sulfoxide (10.9 ml) in dichloromethane (100 ml) at -78°C under an argon atmosphere. The mixture was stirred as such for 15 minutes, and then a solution of benzyl (2S)-2-(hydroxymethyl)pyrrolidine-1-carboxylate (12.0 g) in dichloromethane (80 ml) was added dropwise thereto. The mixture was stirred as such for 15 minutes, and then triethylamine (42.7 ml) was added dropwise thereto, followed by stirring as such for 20 minutes. The resulting mixture was returned to room temperature and then water was added thereto, followed by separation. The organic layer was washed with 1 N hydrochloric acid twice, an aqueous 4% sodium carbonate solution and saturated brine, respectively, and then dried over magnesium sulfate. The solvent was distilled off under reduced pressure, and the resulting residue was purified by silica gel column chromatography (hexane : ethyl acetate = 2 : 1 → 1 : 1) to give benzyl (2S)-2-formylpyrrolidine-1-carboxylate. To [2-(1,3-dioxolan-2-yl)ethyl]triphenylphosphonium bromide (90.4 g) was added tetrahydrofuran (400 ml), further added t-butoxypotassium, and the mixture was stirred at room temperature for 1.5 hours under a nitrogen atmosphere. To the resulting mixture was added dropwise a solution of benzyl (2S)-2-formylpyrrolidine-1-carboxylate in tetrahydrofuran (200 ml), and the mixture was stirred at room temperature for 1 hour and at 50°C for 3 hours under a nitrogen atmosphere. After returning to room temperature, water was added thereto and the mixture was extracted with ethyl acetate. The organic layer was washed with saturated brine, and dried over magnesium sulfate. The solvent was distilled off under reduced pressure, and the resulting residue was purified by silica gel column chromatography (hexane : ethyl acetate = 3 : 1 → 2 : 1) to give benzyl (2S)-2-[3-(1,3-dioxolan-2-yl)-1-propenyl]pyrrolidine-1-carboxylate (15.1 g). Benzyl (2S)-2-[3-(1,3-dioxolan-2-yl)-1-propenyl]pyrrolidine-1-carboxylate (15.0 g) was dissolved in ethanol (200 ml), and then 10% palladium carbon (4.0 g, water content: 50%) was added thereto, followed by overnight stirring at room temperature under a hydrogen atmosphere. The insolubles were removed by filtration, and then the solvent was distilled off under reduced pressure. The resulting residue was dissolved in tetrahydrofuran (200 ml) and a 1 N aqueous sodium hydroxide solution (150 ml), and then benzyl chlorocarbonate (10.1 ml) was added dropwise thereto at 0°C under a nitrogen atmosphere. The mixture was returned to room temperature, stirred for 3 hours and extracted with ethyl acetate. The organic layer was washed with saturated brine, and dried over magnesium sulfate. The solvent was distilled off under reduced pressure, and the resulting residue was purified by silica gel column chromatography (hexane : ethyl acetate = 7 : 1) to give benzyl (2R)-2-[3-(1,3-dioxolan-2-yl)propyl]pyrrolidine-1-carboxylate (13.6 g). Benzyl (2R)-2-[3-(1,3-dioxolan-2-yl)propyl]pyrrolidine-1-carboxylate (13.0 g) was dissolved in tetrahydrofuran (130 ml), and then 2 N hydrochloric acid (305 ml) was added thereto at 0°C. After stirring the resulting mixture at room temperature for 3.5 hours, a 1 N aqueous sodium hydroxide solution (610 ml) was added thereto at 0°C. The mixture was extracted with ethyl acetate, the organic layer was then washed with saturated brine, and dried over magnesium sulfate. The solvent was distilled off under reduced pressure, and the resulting residue was purified by silica gel column chromatography (hexane : ethyl acetate = 2 : 1) to give colorless benzyl (2R)-2-(4-oxobutyl)pyrrolidine-1-carboxylate (8.4 g). To a solution of benzyl (2R)-2-(4-oxobutyl)pyrrolidine-1-carboxylate (8.0 g) and 1-methyl-1-cyclohexene (45.9 ml) in tert-butanol (250 ml) was added dropwise an aqueous solution (200 ml) of sodium chlorite (36.8 g) and sodium dihydrogen phosphate dihydrate (40.9 g) at room temperature. The mixture was stirred as such for 3 hours, and then 1 N hydrochloric acid (40 ml) was added thereto at 0°C. The mixture was extracted with ethyl acetate, washed with an aqueous sodium thiosulfate solution twice and saturated brine once, and then dried over magnesium sulfate. The solvent was distilled off under reduced pressure, and the resulting residue was purified by silica gel column chromatography (hexane : ethyl acetate = 3 : 1 → methanol : ethyl acetate = 1 : 19). The resulting residue was dissolved in ethanol (200 ml), 10% palladium carbon (3.0 g, water content: 50%) was added thereto, and the mixture was stirred for 6 hours under a hydrogen atmosphere. The insolubles were removed by filtration, and then the solvent was distilled off under reduced pressure. The resulting residue was washed with hexane-ethyl acetate to give 4-[(2R)-pyrrolidin-2-yl]butanoic acid (2.63 g) as colorless crystals. 4-[(2R)-pyrrolidin-2-yl]butanoic acid (1.21 g), 5-bromo-2-chloronicotinaldehyde (1.3 g) and sodium carbonate (1.63 g) were added to dimethyl sulfoxide (30 ml) and water (15 ml), and then the mixture was stirred at 90°C for 4.5 hours. After cooling to 0°C, water was added thereto, 1 N hydrochloric acid (30 ml) was then added thereto, and the mixture was extracted with ethyl acetate. The organic layer was washed with water and saturated brine, and dried over magnesium sulfate. The solvent was distilled off under reduced pressure, and the resulting residue was as such dissolved in N,N-dimethylformamide (20 ml), and then potassium carbonate (2.45 g) and iodomethane (1.1 ml) were added thereto, followed by stirring at room temperature for 1 hour under a nitrogen atmosphere. Water was added thereto and the mixture was then extracted with ethyl acetate. The organic layer was washed with water and saturated brine, and dried over magnesium sulfate. The solvent was distilled off under reduced pressure, and the resulting residue was purified by silica gel column chromatography (hexane : ethyl acetate = 16 : 1 → hexane : ethyl acetate = 4 : 1) to give methyl 4-[(2R)-1-(5-bromo-3-formylpyridin-2-yl)pyrrolidin-2-yl]butanoate (1.94 g) as a yellow oily material. To a solution of methyl 4-[(2R)-1-(5-bromo-3-formylpyridin-2-yl)pyrrolidin-2-yl]butanoate (1.9 g) in dimethyl carbonate (50 ml) was added sodium methoxide (28% solution in methanol, 2.06 g), and the mixture was heated at 70°C for 3 hours under a nitrogen atmosphere. After ice-cooling the resulting mixture, water was added thereto and the mixture was extracted with ethyl acetate. The organic layer was washed with saturated brine, and dried over magnesium sulfate. The solvent was distilled off under reduced pressure, and the resulting residue was purified by silica gel column chromatography (hexane : ethyl acetate = 9 : 1 → 4 : 1). The resulting residue was washed with hexane-ethyl acetate to give methyl (8aR)-3-bromo-7,8,8a,9,10,11-hexahydropyrido[3,2-g]pyrrolo[1,2-a]azocine-6-carboxylate (1.33 g) as yellow crystals. A suspension of methyl (8aR)-3-bromo-7,8,8a,9,10,11-hexahydropyrido[3,2-g]pyrrolo[1,2-a]azocine-6-carboxylate (1.2 g), 4-(2-butoxyethoxy)phenylboric acid (1.10 g) and potassium carbonate (1.28 g) in toluene (20 ml), ethanol (2 ml) and water (2 ml) was stirred for 30 minutes under an argon atmosphere. Tetrakis(triphenylphosphine) palladium (206 mg) was added thereto, and the mixture was heated at 115°C for 6 hours under an argon atmosphere and was allowed to cool. Water was added thereto and the mixture was extracted with ethyl acetate. The organic layer was washed with saturated brine, and dried over magnesium sulfate. The solvent was distilled off under reduced pressure, and the resulting residue was purified by silica gel column chromatography (hexane : ethyl acetate = 19 : 1 → hexane : ethyl acetate = 4 : 1) to give methyl (8aR)-3-[4-(2-butoxyethoxy)phenyl]-7,8,8a,9,10,11-hexahydropyrido[3,2-g]pyrrolo[1,2-a]azocine-6-carboxylate (700 mg) as a yellow oily material. To a solution of methyl (8aR)-3-[4-(2-butoxyethoxy)phenyl]-7,8,8a,9,10,11-hexahydropyrido[3,2-g]pyrrolo[1,2-a]azocine-6-carboxylate (690 mg) in THF (25 ml) and methanol (25 ml) was added a 1 N aqueous sodium hydroxide solution (6.1 ml), and the mixture was heated at 90°C for 3 hours. After cooling the resulting mixture to 0°C, water was added thereto and the mixture was neutralized with 1 N hydrochloric acid, and then extracted with ethyl acetate. The organic layer was washed with saturated brine, and dried over magnesium sulfate. The solvent was distilled off under reduced pressure, and the resulting residue was recrystallized from ethyl acetate to give (8aR)-3-[4-(2-butoxyethoxy)phenyl]-7,8,8a,9,10,11-hexahydropyrido[3,2-g]pyrrolo[1,2-a]azocine-6-carboxylic acid (403 mg) as yellow crystals.

m.p. 215.5-216.5°C
Elementary analysis C₂₆H₃₂N₂O₄, Calcd. C, 71.53 ; H, 7.39 ; N, 6.42 ; Found. C, 71.43 ; H, 7.48 ; N, 6.19.

[α]_{D} = +196.3° (C = 0.404%, in chloroform)

### Experimental Example

(1) Cloning of human CCR5 chemokine receptor
   Cloning of a CCR5 gene was conducted from human spleen cDNA by a PCR method. Using 0.5 ng of spleen cDNA (Toyobo Co., Ltd., QUICK-Clone cDNA) as a template, the PCR reaction was carried out in a DNA Thermal Cycler 480 (Perkin Elmer) using a TaKaRa EX Taq (Takara Shuzo Co., Ltd.) (reaction conditions: 30 cycles of treatments at 95°C for 1 minute, at 60°C for 1 minute, and at 75°C for 5 minutes) by adding 25 pmol of primers, SEQ ID NO. 1 (sequence length: 34; sequence type: nucleic acid; number of chains: a single chain; topology: linear; sequence kind: other nucleic acid, synthetic DNA) described in Experimental Example (1) of WO 99/32100, and SEQ ID NO. 2 (sequence length: 34; sequence type: nucleic acid; number of chains: a single chain; topology: linear; sequence kind: other nucleic acid, synthetic DNA) described in Experimental Example (1) of WO 99/32100, respectively, which were prepared by referring to the base sequence of the CCR5 gene described by Samson et al. (Biochemistry 35 (11), 3362-3367 (1996)). The PCR products were subjected to agarose gel electrophoresis to collect DNA fragments of about 1.0 kb. Then, the CCR5 gene was cloned using an Original TA Cloning Kit (Funakoshi Co., Ltd.).
(2) Preparation of Plasmid for Expression of human CCR5
   The plasmids obtained above were digested with restriction enzymes XbaI (Takara Shuzo Co., Ltd.) and BamHI (Takara Shuzo Co., Ltd.), and subjected to agarose gel electrophoresis to collect DNA fragments of about 1.0 kb. The DNA fragments and a plasmid pcDNA3.1 (Funakoshi Co., Ltd.) for expression in animal cells, which was previously digested with XbaI and BamHI, were mixed and ligated by DNA Ligation Kit Ver.2 (Takara Shuzo Co., Ltd.). Transformation of E. coli JM109 competent cells (Takara Shuzo Co., Ltd.) gave plasmid pCKR5.
(3) Introduction of the Plasmid for Expression of human CCR5 into CHO-K1 cells and Expression thereof
   CHO-K1 cells grown in a 750 ml tissue culture flask (Becton Dickinson) using Ham's F12 medium (Nihon Pharmaceutical Co,. Ltd.) containing 10% fetal bovine serum (Lifetech Oriental) were collected from the flask by using 0.5 g/L trypsin-0.2 g/L EDTA (Lifetech Oriental). The cells were then washed with PBS (Lifetech Oriental), centrifuged (1000 rpm, 5 minutes), and suspended in PBS. Next, DNA was introduced into the cells using Gene Pulser (Bio-Rad Laboratories Inc.) under the following conditions. Namely, 8 × 10⁶ cells and 10 µg of plasmid pCKR5 for expression of human CCR5 were added into a cuvette of a 0.4 cm-gap, and electroporation was carried out at an electric voltage of 0.25 kV and a capacitance of 960 µF. Subsequently, the cells were transferred into Ham's F12 medium containing 10% fetal bovine serum, and incubated for 24 hours. The cells were again collected, centrifuged, and then suspended in Ham's F12 medium containing 10% fetal bovine serum and Geneticin (Lifetech Oriental) at a concentration of 500 µg/ml. The suspension of cells was diluted to a concentration of 10⁴ cells/ml, and inoculated on a 96-well plate (Becton Dickinson) to give Geneticin-resistant strains.
   Subsequently, the Geneticin-resistant strains were cultured in the 96-well plate (Becton Dickinson), and then CCR5-expressing cells were selected from the resistant strains. Namely, an assay buffer (Ham's F12 medium containing 0.5% BSA, and 20 mM HEPES (Wako Pure Chemical Industries, Ltd., pH 7.2)) containing 200 pM [¹²⁵I]-RANTES (Amersham) as a ligand was added to each well and the binding reaction was carried out at room temperature for 40 minutes. Each well plate containing the cells was washed with ice-cooled PBS, and then to each well was added 1 M NaOH in an amount of 50 µl/well, which was stirred. The cells to which the ligand bound specifically, i.e., CCR5/CHO strains, were selected by measurement of radioactivity by γ-counter.
(4) Evaluation of Compound based on CCR5 antagonist activity

The CCR5/CHO strains were inoculated on a 96-well microplate at a concentration of 5 × 10⁴ cells/well, respectively and were cultured for 24 hours. After the medium was removed by suction, to each well was added an assay buffer containing a test compound (1 µM), and [¹²⁵I]-RANTES (Amersham) used as a ligand at a concentration of 100 pM. The reaction was carried out at room temperature for 40 minutes. After the assay buffer was removed by suction, each well plate containing the cells were washed with ice-cooled PBS twice. Then, to each well was added 200 µl of MicroScint-20 (Packard Industry Company, Inc.), and the radioactivity was measured with TopCount (Packard Industry Company, Inc.)

According to the method above, inhibitory ratios to CCR5 binding of the test compounds were determined. The results are shown in Table 1.

**[Table 1]**

| Compound No. | Binding Inhibitory Ratio (%) |
|---|---|
| 5 | 96 |

| | |
|---|---|
| 6 | 98 |
| 8 | 97 |
| 9 | 95 |
| 15 | 100 |
| 16 | 94 |
| 17 | 98 |

| Formulation Example 1 (capsules) | |
|---|---|
| (1) (Ss)-9-[4-(2butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]bensazocine-6-carboxamide | |
| (2) lactose | 61 mg |
| (3) microcrystalline cellulose | 18 mg |
| (4) magnesium stearate | 1 mg |
| contents of 1 capsule | 120 mg |

After mixing (1), (2), (3) and (4), the mixture is filled in gelatin capsules.

| Formulation Example 2 (capsules) | |
|---|---|
| (1) (Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide | 40 mg |
| (2) lactose | 61 mg |
| (3) microcrystalline cellulose | 18 mg |
| (4) magnesium stearate | 1 mg |
| contents of 1 capsule | 120 mg |

After mixing (1), (2), (3) and (4), the mixture is filled in gelatin capsules.

| Formulation Example 3 (tablets) | |
|---|---|
| (1) (Ss)-9-[4-(2butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]bensazocine-6-carboxamide | |
| (2) lactose | 61 mg |
| (3) microcrystalline cellulose | 18 mg |
| (4) magnesium stearate | 1 mg |
| contents of 1 capsule | 120 mg |

After mixing (1), (2), (3) and (4), the mixture is filled in gelatin capsules.

| Formulation Example 4 (tablets) | |
|---|---|
| (1) (Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide | 40 mg |
| (2) mannitol | 51.2 mg |
| (3) microcrystalline cellulose | 18 mg |
| (4) hydroxypropyl cellulose | 3.6 mg |
| (5) croscarmellose sodium | 6 mg |
| (6) magnesium stearate | 1.2 mg |
| 1 tablet | 120 mg |

(1), (2), (3) and (4) are mixed and granulated. To the granules are added (5) and (6), and the mixture is compressed into tablets.

### Industrial Applicability

The compound represented by the formula (I) of the present invention or a salt thereof has strong CCR5 antagonistic activity, and thus can be used advantageously in prevention and treatment of various HIV infections, for example, AIDS, in humans.

## Claims

1. A compound of the formula: 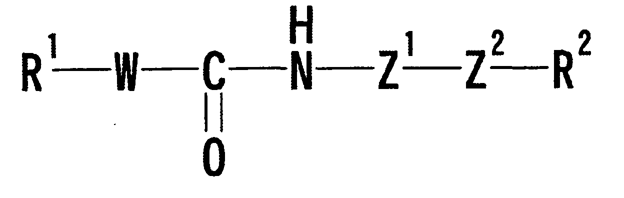 wherein R¹ is a 5- or 6-membered ring which may be substituted;
Z¹ is a 5- or 6-membered aromatic ring which may be further substituted;
Z² is a group of -Z^{2a}-W¹-Z^{2b}-, wherein Z^{2a} and Z^{2b} are each O, S(O)_{q} (wherein q is 0, 1 or 2), an imino group which may be substituted, or a bond; and W¹ is an alkylene chain which may be substituted, an alkenylene chain which may be substituted, or a bond;
W is a group represented by 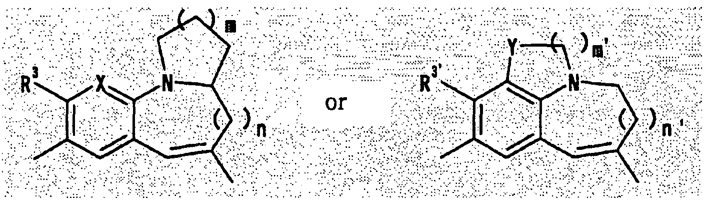 wherein R³ and R^{3'} are each a hydrogen atom, a lower alkyl group which may be substituted, or a lower alkoxy group which may be substituted; X is CH or N; n and n' are each an integer of 0 or 1 to 4; m and m' are each 1 or 2; Y is O, S(O)ₚ (wherein p is 0, 1 or 2), CH₂ or NR⁴ (wherein R⁴ is a hydrogen atom, a lower alkyl group which may be substituted, or a lower acyl group which may be substituted); and
R² is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula: 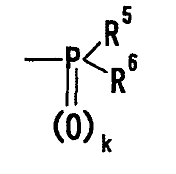 wherein k is 0 or 1, and when k is 0, the phosphorus atom may form a phosphonium salt; R⁵ and R⁶ are each a hydrocarbon group which may be substituted, a hydroxy group which may be substituted, or an amino group which may be substituted; or R⁵ and R⁶ may be bonded to each other to form a cyclic group with the adjacent phosphorus atom, (4) an amidino group which may be substituted, or (5) a guanidino group which may be substituted; or a salt thereof.

2. A prodrug of the compound according to claim 1.

3. The compound according to claim 1, wherein R¹ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted.

4. The compound according to claim 1, wherein R¹ is a benzene which may be substituted.

5. The compound according to claim 1, wherein n is 2.

6. The compound according to claim 1, wherein Z¹ is a benzene which may be substituted with a substituent selected from (1) a halogen atom, (2) a C₁₋₄ alkyl group which may be substituted with a halogen atom, and (3) a C₁₋₄ alkoxy group which may be substituted with a halogen atom.

7. The compound according to claim 1, wherein Z¹ is a benzene which may be substituted with a methyl group or a trifluoromethyl group.

8. The compound according to claim 1, wherein Z² is a group represented by -Z^{2a}-W²-Z^{2b}-, wherein Z^{2a} and Z^{2b} are each O, S(O)_{q} (wherein q is 0, 1 or 2), an imino group which may be substituted, or a bond, and W² is an alkylene chain which may be substituted.

9. The compound according to claim 1, wherein Z² is - CH₂-, -CH(OH)- or -S(O)_{q}-CH₂- (wherein q is 0, 1 or 2).

10. The compound according to claim 1, wherein Z² is a group represented by -S(O)_{q}-CH₂- (wherein q is 0, 1 or 2).

11. The compound according to claim 1, wherein R² is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) an amidino group which may be substituted, or (4) a guanidino group which may be substituted.

12. The compound according to claim 1, wherein R² is an amino group which may be substituted, or a nitrogen-containing heterocyclic group which may be substituted and may contain a sulfur atom or an oxygen atom as the ring-constituting atom.

13. The compound according to claim 1, wherein R² is -NRR', wherein R and R' are each an aliphatic hydrocarbon group which may be substituted or an alicyclic heterocyclic group which may be substituted.

14. The compound according to claim 1, wherein R² is a nitrogen-containing heterocyclic aromatic group which may be substituted.

15. The compound according to claim 1, wherein R² is an imidazolyl group which may be substituted or a triazolyl group which may be substituted.

16. The compound according to claim 1, wherein W is a group represented by 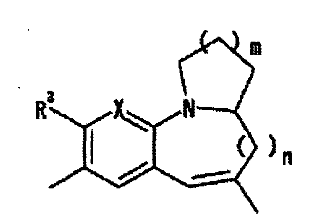 wherein each symbol has the same meaning as defined in claim 1.

17. The compound according to claim 1, wherein R¹ is a benzene, a furan, a thiophene, a pyridine, a cyclopentane, a cyclohexane, a pyrrolidine, a piperidine, a piperazine, a morpholine, a thiomorpholine or a tetrahydropyran, each of which may be substituted with a halogen, a nitro, a cyano, a C₁₋₆ alkyl, a C₁₋₆ alkoxy, a C₁₋₆ alkoxy-C₁₋₆ alkyl or a C₁₋₆ alkoxy-C₁₋₆ alkoxy,
Z¹ is a benzene which may be substituted with a substituent selected from (1) a halogen atom, (2) a C₁₋₄ alkyl group which may be substituted with a halogen atom and (3) a C₁₋₄ alkoxy group which may be substituted with a halogen atom,
Z² is -Z^{2a}-W¹-Z^{2b}-, wherein Z^{2a} and Z^{2b} are each O, S(O)_{q} (wherein q is 0, 1 or 2), an imino group which may be substituted with a C₁₋₄ alkyl group, or a bond, and W¹ is a bond, or a C₁₋₄ alkylene chain or a C₂₋₄ alkenylene chain, each of which may be substituted with C₁₋₆ alkyl, a hydroxy group, a hydroxyimino or a C₁₋₆ alkoxyimino, and
R² is an amino group which may be substituted with a C₁₋₄ alkyl group, or a nitrogen-containing heterocyclic group which may contain a sulfur atom or an oxygen atom as the ring-constituting atom and may be substituted with a C₁₋₄ alkyl group.

18. A compound represented by the formula: 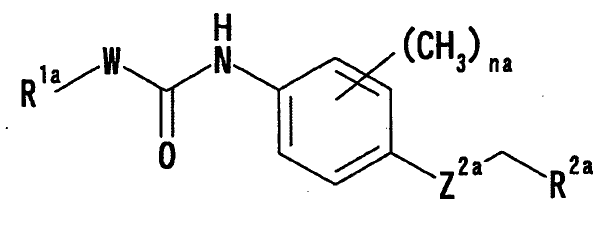 wherein R^{1a} is a (C₁₋₆ alkoxy-C₁₋₆ alkoxy) phenyl,
R^{2a} is (1) an N-C₁₋₆ alkyl-N-tetrahydropyranylamino, (2) an imidazolyl which may be substituted with a C₁₋₆ alkyl which may be substituted, or (3) a triazolyl which may be substituted with a C₁₋₆ alkyl which may be substituted,
R³ is a hydrogen atom, a lower alkyl group which may be substituted, or a lower alkoxy group which may be substituted,
na is 0 or 1,
Z^{2a} is a bond, S, SO or SO₂, and
W is represented by 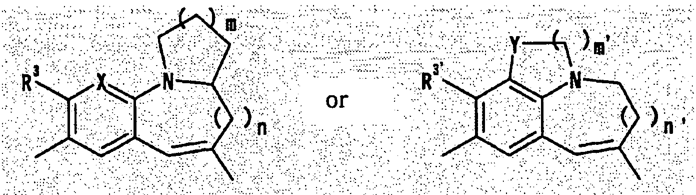 wherein R³ and R^{3'} are each a hydrogen atom, a lower alkyl group which may be substituted, or a lower alkoxy group which may be substituted; X is CH or N; n and n' are each an integer of 0 or 1 to 4; m and m' are each 1 or 2; Y is O, S(O)ₚ (wherein p is 0, 1 or 2), CH₂ or NR⁴ (wherein R⁴ is a hydrogen atom, a lower alkyl group which may be substituted, or a lower acyl group which may be substituted), or a salt thereof.

19. The compound according to claim 18, wherein Z^{2a} is SO.

20. The compound according to claim 19, wherein Z^{2a} is SO having a configuration of (S).

21. The compound according to claim 18, wherein n and n' are each 2.

22. (Ss)-9-[4-(2-butoxyethoxy)phenyl]-N-[4-[[(1-propyl-1H-imidazol-5-yl)methyl]sulfinyl]phenyl]-1,2,3,3a,4,5-hexahydropyrrolo[1,2-a][1]benzazocine-6-carboxamide and a diastereomer thereof.

23. (Ss)-3-[4-(2-butoxyethoxy)phenyl]-N-[4-[[(1-propyl-1H-imidazol-5-yl)methyl]sulfinyl]phenyl]-7,8,8a,9,10,11-hexahydropyrido[3,2-g]pyrrolo[1,2-a]azocine-6-carboxamide and a diastereomer thereof.

24. A process for producing a compound represented by the formula: 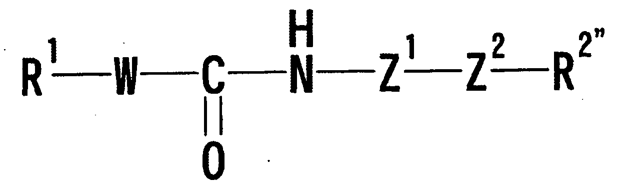 wherein Z¹ and Z² have the same meanings as defined in claim 1, R^{2"} is (1) an amino group which may be substituted, in which the nitrogen group may be converted to a quaternary ammonium, (2) a nitrogen-containing heterocyclic group which may be substituted and may contain a sulfur atom or an oxygen atom as the ring-constituting atom, in which the nitrogen atom may be converted to a quaternary ammonium, or (3) a group represented by the formula: 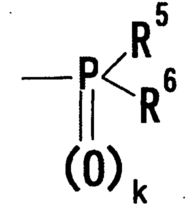 wherein k is 0 or 1, and when k is 0, the phosphorus atom may form a phosphonium salt; R⁵ and R⁶ are each a hydrocarbon group which may be substituted, a hydroxy group which may be substituted, or an amino group which may be substituted; or R⁵ and R⁶ may be bonded to each other to form a cyclic group with the adjacent phosphorus atom; and the other symbols have the same meanings as defined in claim 1, or a salt thereof, which comprises subjecting a compound represented by the formula: 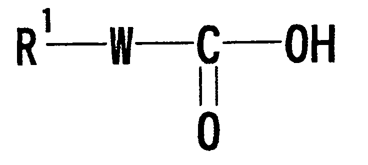 wherein each symbol has the same meaning as defined above, a salt thereof or a reactive derivative thereof, and a compound represented by the formula: 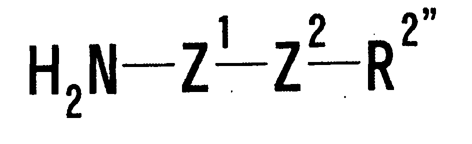 wherein R^{2"} has the same meaning as defined above and the other symbols have the same meanings as defined in claim 1, a salt thereof to a condensation reaction, and then optionally to deprotection, oxidation-reduction and/or quaternization reaction.

25. A pharmaceutical composition comprising the compound represented by the formula: 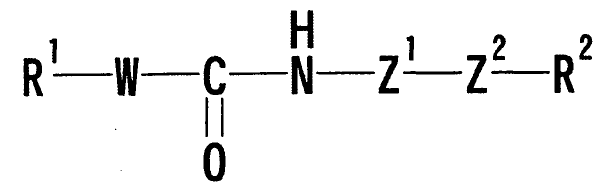 wherein R¹ is a 5- or 6-membered ring which may be substituted;
Z¹ is a 5- or 6-membered aromatic ring which may be further substituted;
Z² is Z^{2a}-W¹-Z^{2b}-, wherein Z^{2a} and Z^{2b} are each O, S(O)_{q} (wherein q is 0, 1 or 2), an imino group which may be substituted, or a bond; and W¹ is an alkylene chain which may be substituted, an alkenylene chain which may be substituted, or a bond;
W is a group represented by 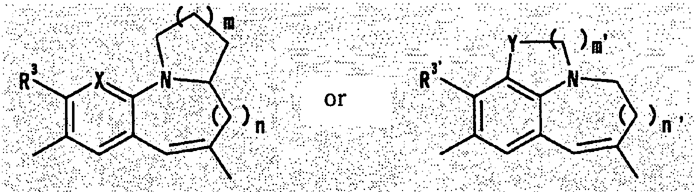 wherein R³ and R^{3'} are each a hydrogen atom, a lower alkyl group which may be substituted, or a lower alkoxy group which may be substituted; X is CH or N; n and n' are each an integer of 0 or 1 to 4; m and m' are each 1 or 2; Y is O, S(O)ₚ (wherein p is 0, 1 or 2), CH₂ or NR⁴ (wherein R⁴ is a hydrogen atom, a lower alkyl group which may be substituted, or a lower acyl group which may be substituted); and
R² is (1) an amino group which may be substituted, in which the nitrogen atom may be converted to a quaternary ammonium or an oxide, (2) a nitrogen-containing heterocyclic group which may be substituted and may contain a sulfur atom or an oxygen atom as the ring-constituting atom, in which the nitrogen atom may be converted to a quaternary ammonium or an oxide, (3) a group represented by the formula: 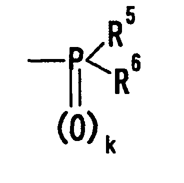 wherein k is 0 or 1, and when k is 0, the phosphorus atom may form a phosphonium salt; R⁵ and R⁶ are each a hydrocarbon group which may be substituted, a hydroxy group which may be substituted, or an amino group which may be substituted; or R⁵ and R⁶ may be bonded to each other to form a cyclic group with the adjacent phosphorus atom, (4) an amidino group which may be substituted, or (5) a guanidino group which may be substituted; a salt thereof or a prodrug thereof.

26. The pharmaceutical composition according to claim 25, which is a CCR antagonist.

27. The pharmaceutical composition according to claim 26, wherein CCR is CCR5 and/or CCR2.

28. The pharmaceutical composition according to claim 26, wherein CCR is CCR5.

29. The pharmaceutical composition according to claim 25, which is a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.

30. The pharmaceutical composition according to claim 25, which is a prophylactic and/or therapeutic agent for HIV infection.

31. The pharmaceutical composition according to claim 25, which is a prophylactic and/or therapeutic agent for AIDS.

32. The pharmaceutical composition according to claim 25, which is a suppressive agent for disease progression of AIDS.

33. A method for preventing or treating HIV infection, chronic rheumatoid arthritis, autoimmune diseases, allergic diseases, ischemic brain cell disorder, cardiac infarction, nephritis/nephropathy, arteriosclerosis or graft-versus-host diseases, which comprises administering an effective amount of the compound according to claim 1, a salt thereof or a prodrug thereof to a subject in need thereof.

34. Use of the compound according to claim 1, a salt thereof or a prodrug thereof, for the manufacture of a prophylactic and/or therapeutic agent for HIV infection, chronic rheumatoid arthritis, autoimmune diseases, allergic diseases, ischemic brain cell disorder, cardiac infarction, nephritis/nephropathy, arteriosclerosis or graft-versus-host diseases.
